(19) Europäisches Patentamt
European Patent Office
Office européen des brevets

(11) EP 2 850 094 B1

(12) EUROPEAN PATENT SPECIFICATION

(45) Date of publication and mention of the grant of the patent:
**17.04.2024 Bulletin 2024/16**

(21) Application number: **13791383.6**

(22) Date of filing: **14.05.2013**

(51) International Patent Classification (IPC):
***A61K 47/68*** (2017.01) ***A61P 35/00*** (2006.01)

(52) Cooperative Patent Classification (CPC):
**C07K 5/0205; A61K 47/6803; A61K 47/6851; A61K 47/6889; A61P 35/00**

(86) International application number:
**PCT/US2013/040951**

(87) International publication number:
**WO 2013/173337 (21.11.2013 Gazette 2013/47)**

(54) **SELF-STABILIZING LINKER CONJUGATES**

SELBSTSTABILISIERENDE LINKERKONJUGATE

CONJUGUÉS DE LIEURS AUTO-STABILISANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:
**15.05.2012 US 201261647373 P**
**28.02.2013 US 201361770983 P**
**05.03.2013 US 201361773067 P**
**13.03.2013 US 201313799244**

(43) Date of publication of application:
**25.03.2015 Bulletin 2015/13**

(73) Proprietor: **Seagen Inc.**
**Bothell, WA 98021 (US)**

(72) Inventors:
- **LYON, Robert**
  **Bothell, WA 98021 (US)**
- **DORONINA, Svetlana**
  **Bothell, WA 98021 (US)**
- **BOVEE, Timothy**
  **Bothell, WA 98021 (US)**

(74) Representative: **Mewburn Ellis LLP**
**Aurora Building**
**Counterslip**
**Bristol BS1 6BX (GB)**

(56) References cited:
**EP-A2- 0 446 071**
**WO-A1-2014/151030**
**WO-A2-2012/054748**
**US-A- 5 523 360**
**US-A1- 2006 193 865**
**US-A1- 2009 012 241**
**US-A1- 2010 104 589**

- **ABU AJAJ KHALID ET AL: "Development of Protein-Binding Bifunctional Linkers for a New Generation of Dual-Acting Prodrugs", BIOCONJUGATE CHEMISTRY, vol. 20, no. 2, February 2009 (2009-02), pages 390-396, XP002758088, ISSN: 1043-1802**
- **STEPHEN C. ALLEY ET AL: "Contribution of Linker Stability to the Activities of Anticancer Immunoconjugates", BIOCONJUGATE CHEMISTRY, vol. 19, no. 3, 1 March 2008 (2008-03-01), pages 759-765, XP055037546, ISSN: 1043-1802, DOI: 10.1021/bc7004329**
- **LYON ROBERT P ET AL: "Self-stabilizing ADCs: antibody-drug conjugates prepared with maleimido drug-linkers that catalyze their own thiosuccinimide ring hydrolysis.", CANCER RESEARCH, vol. 73, no. 8, Suppl. 1, April 2013 (2013-04), page 4333, XP002758089, & 104TH ANNUAL MEETING OF THE AMERICAN-ASSOCIATION-FOR-CANCER-RESEARCH (AACR); WASHINGTON, DC, USA; APRIL 06 -10, 2013**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BALDWIN, AD ET AL.: 'Tunable degradation of maleimide-thiol adducts in reducing environment.' BIOCONJUG CHEM vol. 22, no. ISSUE, 19 October 2011, pages 1946 - 1953, XP055246194**
- **SHEN, BQ ET AL.: 'Conjugation site modulates the in vivo stability and therapeutic activity of antibody-drug conjugates.' NATURE BIOTECHNOLOGY vol. 30, no. 2, February 2012, pages 184 - 189, XP055123129**
- **KALIA, J ET AL.: 'Advances in Bioconjugation.' CURRENT ORGANIC CHEMISTRY vol. 14, no. 2, January 2010, XP008178861**

## Description

### CONTINUITY

This application claims the benefit of US Provisional Patent Application No. 61/647,373, filed May 15, 2012, and US Provisional Patent Application No. 61/773,067, filed March 5, 2013, and also claims priority to US Application Ser. No. 13/799,244, filed March 13, 2013.

### BACKGROUND OF THE INVENTION

The antibody-drug conjugate (ADC) field has made significant advances with the FDA approval of Brentuximab Vedotin for the treatment of a select group of patients and with the advancement of many other ADCs in the clinic. The linker component of ADCs is one important feature in developing optimized therapeutic agents that are highly active at well tolerated doses. The electrophilic maleimide functional group has proven very useful in the preparation of ADCs due to its high degree of specificity for reacting with thiol groups and the very fast thiol addition kinetics under gentle conditions.

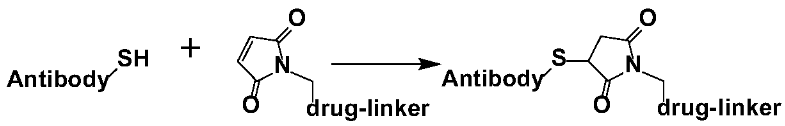

As has been noted by multiple investigators in the bioconjugate field, the thio-substituted product of the reaction between the electrophilic maleimide functional group and free thiol of antibody is subject to slow elimination, thus reversing the above reaction:

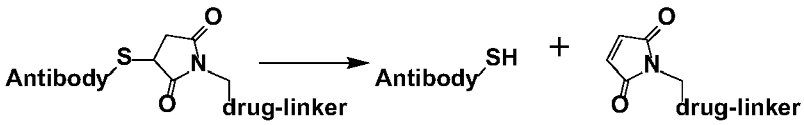

When this reversible reaction occurs in a purified preparation of the ADC, the reaction is largely undetectable because the maleimide and thiol which are regenerated through the elimination process simply react again, thus reforming the intact conjugate. However, when other thiols are present, the net effect can be the transfer of the maleimide from the antibody of the ADC onto any other available thiol. This process has been documented to occur in plasma, in which the maleimide of an ADC transfers to cysteine 34 of serum albumin (Alley et al., Bioconjugate Chem. 2008, 19, 759-765). This process has also been reported when an ADC is incubated in the presence of excess cysteine or glutathione (Shen et al., Nature Biotech, 30(2): 184-9, 2012). The present invention provides, *inter alia,* bioconjugates that do not undergo this transfer reaction. EP0446071 A1 relates to tris-maleimide compounds as intermediates in trifunctional antibody synthesis. Stephen C. Alley et al, Bioconjugate chemistry, vol. 19, no.3, 1 March 2008, 759-765 relates to contribution of linker stability to the activity of anticancer immunoconjugates.

### BRIEF SUMMARY OF THE INVENTION

The invention provides *inter alia,* Linkers, Drug-Linkers, Ligand-Drug Conjugates, Ligand-Linker Conjugates, Ligand-Functional Agent Conjugates, and Functional Agent-Linkers, and methods of preparing and using them. The Ligand-Drug Conjugates are stable in circulation, yet capable of inflicting cell death once released in the vicinity or within tumor cells.

### BRIEF DESCRIPTION OF THE DRAWINGS

**Figure 1** provides a reaction scheme illustrating the reduction of the interchain disulfides of a human IgG1, followed by conjugation of the resulting thiols with a self-stabilizing linker and subsequent hydrolysis of the succinimide ring (Top); and the use of mass spectrometry to monitor the change in the molecular weight of the antibody conjugates due to hydrolysis (Bottom).

**Figure 2** illustrates the timecourse of succinimide ring hydrolysis of a self-stabilizing antibody conjugate by electrospray mass spectrometry. Conjugation of fully reduced cAC10 with maleimido-DPR-val-cit-PAB-MMAE was performed at pH 7.2 and 22°C, then samples were subjected to analysis by LC-MS at the indicated times (Top). The resulting data of % hydrolysis was plotted versus time and fit to an exponential equation to determine kinetic parameters (Bottom).

**Figure 3** provides the hydrolysis kinetic profiles for bioconjugates prepared with an IgG1 antibody and self-stabilizing linkers with varying spacing between the maleimide and the basic group (a primary amine). Conjugation was performed at pH 8 and 37°C, then hydrolysis of the IgG1 light chain conjugate was immediately monitored by mass spectrometry, plotted as a function of time, and fit to an exponential equation.

**Figure 4** provides kinetic profiles of the hydrolysis of bioconjugates prepared with an IgG1 antibody and self-stabilizing maleimide linkers with varying spacing between the maleimide and the basic group (a primary amine). Conjugation was performed at pH 8 and 37°C, then hydrolysis of the IgG1 light chain conjugate was immediately monitored by mass spectrometry, plotted as a function of time, and fit to an exponential equation.

**Figure 5** provides hydrolysis kinetic profiles for bioconjugates prepared with an IgG1 antibody and various N-substituted maleimides. Conjugation was performed at pH 7.4 and 22°C, then hydrolysis of the IgG1 light chain conjugate was immediately monitored by mass spectrometry, plotted as a function of time, and fit to an exponential equation. Hydrolysis of the maleimido-caproyl conjugate (bottom structure) is too slow to produce any detectable hydrolysis in 24 hours under these conditions. The presence of the carboxamide electron withdrawing group (EWG) or the primary amine (BASE) accelerate the hydrolysis, and the combination of the two (top structure) results in a conjugate which hydrolyzes with a half-life of less than 20 minutes under these mild conditions.

**Figure 6** provides hydrolysis kinetic profiles for self-stabilizing maleimido drug-linkers prepared with $\alpha$-diaminopropionic acid ($\alpha$-DPR, open circles) and with $\beta$-diaminopropionic acid ($\beta$-DPR, filled circles). Although isomers of each other, the positioning of the basic amino group and the electron withdrawing carboxamide relative to the succinimide results in a 17-fold difference in the rate of succinimide hydrolysis.

**Figure 7** illustrates the change in drug loading over time for an ADC prepared with a self-stabilizing maleimido-DPR drug-linker versus one prepared with a maleimido-caproyl drug linker when incubated in a buffer containing excess thiol. The reversed-phase chromatograms of the two ADCs at time zero and time 14 days after incubation is shown in the top panel. Chromatographic peak assignments L0, L1, H0, H1, H2, and H3 correspond to unconjugated light chain, light chain with one drug, unconjugated heavy chain, and heavy chain with 1, 2, or 3 drugs, respectively. The self-stabilizing maleimido-DPR drug-linker is represented with open circles versus one prepared with a maleimido-caproyl drug linker (open squares). Drug loading remains constant at 8 per antibody for the self-stabilizing drug-linker (open circles), but falls to 4 drugs per antibody over 14 days for the maleimido-caproyl drug linker (open squares), reflecting loss of drug by maleimide elimination.

**Figure 8** illustrates the change in drug loading over time for ADCs prepared with a self-stabilizing maleimido-DPR drug-linker and a maleimido-caproyl drug linker, when incubated in rat plasma at 37 °C (R=val-cit-PAB-MMAE). ADC samples at each timepoint were purified by Ig Select affinity resin and their drug loading evaluated by reversed-phase HPLC analysis of the ADCs.

**Figure 9** provides the stability profile of drugs conjugated to antibodies via a maleimido-caproyl drug- linker (squares) or a self-stabilizing maleimide linker(circles) during incubation in rat (open symbols) or human (filled symbols) plasma (R=val-cit-PAB-MMAE). ADCs were captured on Protein A affinity resin at each timepoint and the drug released enzymatically via its protease-cleavable linker. The released drug was then quantified by LC-MS/MS and normalized to the initial value. Each timepoint reflects the percent of the conjugated drug that was observed at t0.

**Figure 10** illustrates the decrease in drug loading *in vivo* (rats) for ADCs prepared with a self-stabilizing maleimido-DPR drug-linker and a maleimido-caproyl drug linker (R=val-cit-PAB-MMAE). ADCs were dosed i.v. and plasma samples from each timepoint were purified by Ig Select affinity resin and their drug loading evaluated by reversed-phase HPLC analysis of the ADCs.

**Figure 11** illustrates the antitumor activity of ADCs in a murine xenograft model of ALCL (Karpas-299 cell line). ADCs were prepared with the anti-CD30 antibody cAC10 and drug linkers containing the val-cit-PAB-MMAE cytotoxic payload linked to the antibody via either a maleimido-caproyl group (closed circles) or a self-stabilizing maleimido-DPR group (open circles). Tumors were allowed to reach a volume of approximately 250 $mm^3$ before dosing at 1 mg/kg weekly for three doses (six mice per dose group). The self-stabilizing ADC dose group experienced complete responses (no detectable tumor) in all six animals, with five animals experiencing durable regressions, while the maleimido-caproyl ADC experienced no complete responses.

## DETAILED DESCRIPTION

### Abbreviations and Definitions

Unless stated otherwise, the following terms and phrases as used herein are intended to have the following meanings. When trade names are used herein, the trade name includes the product formulation, the generic drug, and the active pharmaceutical ingredient(s) of the trade name product, unless otherwise indicated by context.

The term "electron-withdrawing group" refers to a functional group that draws electrons away from a reaction center. Exemplary electron withdrawing groups include, but are not limited to, -C(=O), -CN, $-NO_2$, $-CX_3$, -X, -COOR, $-CONR_2$, -COR, -COX, $-SO_2R$, $-SO_2OR$, $-SO_2NHR$, $-SO_2NR_2$, $-PO_3R_2$, $-P(O)(CH_3)NHR$, NO, $-NR_3^+$, $-CR=CR_2$, and -C≡CR wherein X is F, Br, Cl, or I, and R is, at each occurrence, independently selected from the group consisting of hydrogen and $C_{1-6}$ alkyl. Exemplary electron withdrawing groups can also include aryl groups (e.g., phenyl) and certain heteroaryl groups (e.g., pyridine). The term "electron withdrawing groups" includes aryls or heteroaryls further substitued with electron withdrawing groups. Preferred electron withdrawing groups are -C(=O), -CN, $-NO_2$, $-CX_3$, and -X.

The term "base" refers to a functional group that deprotonates water to produce a hydroxide ion. Exemplary bases are amines and nitrogen containing heterocycles. Representative bases include $-N(R^3)(R^4)$ wherein $R^3$ and $R^4$ are independently selected from H or $C_{1-6}$ alkyl, preferably H or methyl,

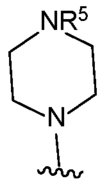
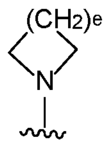
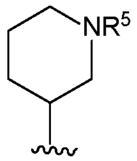
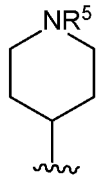
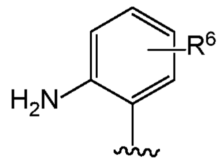

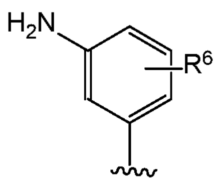
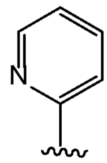
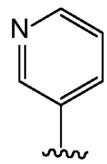
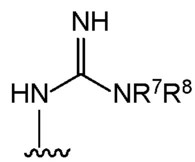

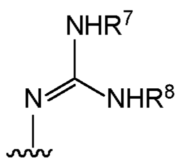
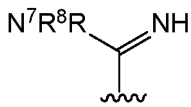
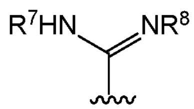

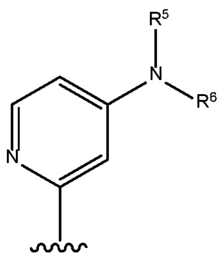
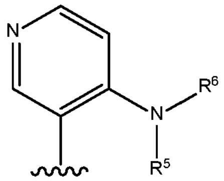

wherein $R^5$, $R^6$, $R^7$ and $R^8$ are, at each occurrence, independently selected from hydrogen or $C_{1-6}$ alkyl, preferably H or methyl, and e is 0-4. In some aspects, the base is a nitrogenous base.

The term "antibody" herein is used in the broadest sense and specifically covers intact monoclonal antibodies, polyclonal antibodies, monospecific antibodies, multispecific antibodies (*e.g.*, bispecific antibodies), and antibody fragments that exhibit the desired biological activity. An intact antibody has primarily two regions: a variable region and a constant region. The variable region binds to and interacts with a target antigen. The variable region includes a complementary determining region (CDR) that recognizes and binds to a specific binding site on a particular antigen. The constant region may be recognized by and interact with the immune system (*see, e.g.,* Janeway et al., 2001, Immuno. Biology, 5th Ed., Garland Publishing, New York). An antibody can be of any type (*e.g.,* IgG, IgE, IgM, IgD, and IgA), class (*e.g.,* IgG1, IgG2, IgG3, IgG4, IgA1 and IgA2) or subclass. The antibody can be derived from any suitable species. In some embodiments, the antibody is of human or murine origin. An antibody can be, for example, human, humanized or chimeric.

The term "monoclonal antibody" as used herein refers to an antibody obtained from a population of substantially homogeneous antibodies, *i.e.,* the individual antibodies comprising the population are identical except for possible naturally-occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

An "intact antibody" is one which comprises an antigen-binding variable region as well as a light chain constant domain ($C_L$) and heavy chain constant domains, $C_H1$, $C_H2$, $C_H3$ and $C_H4$, as appropriate for the antibody class. The constant domains may be native sequence constant domains (*e.g.*, human native sequence constant domains) or amino acid sequence variant thereof.

An "antibody fragment" comprises a portion of an intact antibody, comprising the antigen-binding or variable region thereof. Examples of antibody fragments include Fab, Fab', $F(ab')_2$, and Fv fragments, diabodies, triabodies, tetrabodies, linear antibodies, single-chain antibody molecules, scFv, scFv-Fc, multispecific antibody fragments formed from antibody fragment(s), a fragment(s) produced by a Fab expression library, or an epitope-binding fragments of any of the above which immunospecifically bind to a target antigen (*e.g.,* a cancer cell antigen, a viral antigen or a microbial antigen).

An "antigen" is an entity to which an antibody specifically binds.

The terms "specific binding" and "specifically binds" mean that the antibody or antibody derivative will bind, in a highly selective manner, with its corresponding target antigen and not with the multitude of other antigens. Typically, the antibody or antibody derivative binds with an affinity of at least about $1\times10^{-7}$ M, and preferably $10^{-8}$ M to $10^{-9}$ M, $10^{-10}$ M, $10^{-11}$ M, or $10^{-12}$ M and binds to the predetermined antigen with an affinity that is at least two-fold greater than its affinity for binding to a non-specific antigen (*e.g.*, BSA, casein) other than the predetermined antigen or a closely-related antigen.

The term "inhibit" or "inhibition of" means to a reduce by a measurable amount, or to prevent entirely.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.*, slow to some extent and preferably stop) cancer cell infiltration into peripheral organs; inhibit (*i.e.*, slow to some extent and preferably stop) tumor metastasis; inhibit, to some extent, tumor growth; and/or relieve to some extent one or more of the symptoms associated with the cancer. To the extent the drug may inhibit growth and/or kill existing cancer cells, it may be cytostatic and/or cytotoxic. For cancer therapy, efficacy can, for example, be measured by assessing the time to disease progression (TTP) and/or determining the response rate (RR).

The term "substantial" or "substantially" refers to a majority, *i.e.* >50% of a population, of a mixture or a sample, preferably more than 50%, 55%, 60%, 65%, 70%, 75%, 80%, 85%, 90%, 91%, 92%, 93%, 94%, 95%, 96%, 97 %, 98%, or 99% of a population.

The terms "intracellularly cleaved" and "intracellular cleavage" refer to a metabolic process or reaction inside a cell on a Ligand Drug conjugate (*e.g.*, an Antibody Drug Conjugate (ADC) or the like), whereby the covalent attachment, *e.g.*, the linker, between the Drug moiety (D) and the Ligand unit (*e.g.,* an antibody (Ab)) is broken, resulting in the free Drug, or other metabolite of the conjugate dissociated from the antibody inside the cell. The cleaved moieties of the Drug-Linker-Ligand conjugate are thus intracellular metabolites.

The term "cytotoxic activity" refers to a cell-killing, a cytostatic or an anti-proliferative effect of a Drug-Linker-Ligand conjugate compound or an intracellular metabolite of a Drug-Linker-Ligand conjugate. Cytotoxic activity may be expressed as the $IC_{50}$ value, which is the concentration (molar or mass) per unit volume at which half the cells survive.

The term "cytotoxic agent" as used herein refers to a substance that inhibits or inhibits the function of cells and/or causes destruction of cells. The term is intended to include radioactive isotopes (*e.g.*, $^{211}At$, $^{131}I$, $^{125}I$, $^{90}Y$, $^{186}Re$, $^{188}Re$, $^{153}Sm$, $^{212}Bi$, $^{32}P$, $^{60}C$, and radioactive isotopes of Lu), chemotherapeutic agents, and toxins such as small molecule toxins or enzymatically active toxins of bacterial, fungal, plant or animal origin, including synthetic analogs and derivatives thereof.

The terms "cancer" and "cancerous" refer to or describe the physiological condition or disorder in mammals

that is typically characterized by unregulated cell growth. A "tumor" comprises one or more cancerous cells.

An "autoimmune disease" herein is a disease or disorder arising from and directed against an individual's own tissues or proteins.

Examples of a "patient" include, but are not limited to, a human, rat, mouse, guinea pig, monkey, pig, goat, cow, horse, dog, cat, bird and fowl. In an exemplary embodiment, the patient is a human.

The terms "treat" or "treatment," unless otherwise indicated by context, refer to therapeutic treatment and prophylactic measures to prevent relapse, wherein the object is to inhibit or slow down (lessen) an undesired physiological change or disorder, such as the development or spread of cancer. For purposes of this invention, beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder.

In the context of cancer, the term "treating" includes any or all of: inhibiting growth of tumor cells, cancer cells, or of a tumor; inhibiting replication of tumor cells or cancer cells, lessening of overall tumor burden or decreasing the number of cancerous cells, and ameliorating one or more symptoms associated with the disease.

In the context of an autoimmune disease, the term "treating" includes any or all of: inhibiting replication of cells associated with an autoimmune disease state including, but not limited to, cells that produce an autoimmune antibody, lessening the autoimmune-antibody burden and ameliorating one or more symptoms of an autoimmune disease.

As used herein, the term "Detection unit" refers to refers to any molecule which produces, or can be induced to produce, a detectable signal. Detection units having reporter molecules that can be detected by imaging equipment include, but are not limited to, radioactive, paramagnetic, fluorescent or radioopaque chemical entities. In some embodiments, the Detection unit will be a radioactive compound, a chemiluminescent agent, a fluorescent agent, or a chromogen. In some embodiments, the Detection unit will be a fluorescent molecule such as a fluorophore.

As used herein, the term "Stability unit" refers to a compound that promotes the stability of the conjugate, e.g., by increasing systemic retention of the Ligand when administered to a patient. A Stability unit can also increase the water solubility of the conjugate. An exemplary Stability unit is polyethylene glycol.

The phrase "pharmaceutically acceptable salt," as used herein, refers to pharmaceutically acceptable organic or inorganic salts of a compound *(e.g.,* a Drug, Drug-Linker, or a Ligand-Drug Conjugate). The compound can contain at least one amino group, and accordingly acid addition salts can be formed with the amino group. Exemplary salts include, but are not limited to, sulfate, trifluoroacetate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p*-toluenesulfonate, and pamoate (*i.e.,* 1,1'-methylene-bis - (2-hydroxy-3- naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

Unless otherwise indicated, the term "alkyl" by itself or as part of another term refers to a substituted or unsubstituted straight chain or branched, saturated or unsaturated hydrocarbon having the indicated number of carbon atoms (*e.g.*, "$-C_1-C_8$ alkyl" or "$-C_1-C_{10}$" alkyl refer to an alkyl group having from 1 to 8 or 1 to 10 carbon atoms, respectively). When the number of carbon atoms is not indicated, the alkyl group has from 1 to 8 carbon atoms. Representative straight chain "$-C_1-C_8$ alkyl" groups include, but are not limited to, -methyl, -ethyl, -n-propyl, - n-butyl, -n-pentyl, -n-hexyl, -n-heptyl and -n-octyl; while branched $-C_1-C_8$ alkyls include, but are not limited to, -isopropyl, -*sec*-butyl, -isobutyl, -*tert*-butyl, -isopentyl, and -2-methylbutyl; unsaturated $-C_2-C_8$ alkyls include, but are not limited to, -vinyl, -allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexyl, 2-hexyl, -3-hexyl, -acetylenyl, -propynyl, -1-butynyl, -2-butynyl, -1-pentynyl, -2-pentynyl and -3-methyl-1 butynyl. In some embodiments, an alkyl group is unsubstituted. An alkyl group can be substituted with one or more groups. In some aspects, an alkyl group will be saturated.

Unless otherwise indicated, "alkylene," by itself of as part of another term, refers to a substituted or unsubstituted saturated, branched or straight chain or cyclic hydrocarbon radical of the stated number of carbon atoms, typically 1-10 carbon atoms, and having two monovalent radical centers derived by the removal of two hydrogen atoms from the same or two different carbon atoms of a parent alkane. Typical alkylene radicals include, but are not limited to: methylene ($-CH_2-$), 1,2-ethyl ($-CH_2CH_2-$), 1,3-propyl ($-CH_2CH_2CH_2-$), 1,4-butyl ($-CH_2CH_2CH_2CH_2-$), and the like. In preferred aspects, an alkylene is a branched or straight chain hydrocarbon (i.e., it is not a cyclic hydrocarbon).

Unless otherwise indicated, "aryl," by itself or as part of another term, means a substituted or unsubstituted

monovalent carbocyclic aromatic hydrocarbon radical of 6-20 carbon atoms derived by the removal of one hydrogen atom from a single carbon atom of a parent aromatic ring system. Some aryl groups are represented in the exemplary structures as "Ar". Typical aryl groups include, but are not limited to, radicals derived from benzene, substituted benzene, naphthalene, anthracene, biphenyl, and the like. An exemplary aryl group is a phenyl group as follows:

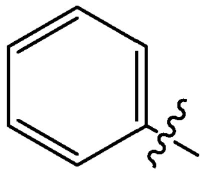

.

Unless otherwise indicated, an "arylene," by itself or as part of another term, is an aryl group as defined above which has two covalent bonds (i.e., it is divalent) and can be in the ortho, meta, or para configurations as shown in the following structures, with phenyl as the exemplary group:

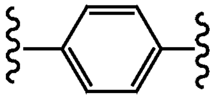 , 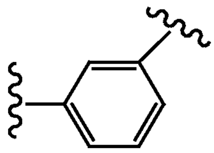 , 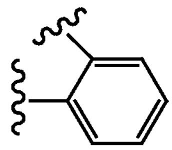 ,

Unless otherwise indicated, a "$C_3$-$C_8$ heterocycle," by itself or as part of another term, refers to a monovalent substituted or unsubstituted aromatic or non-aromatic monocyclic or bicyclic ring system having from 3 to 8 carbon atoms (also referred to as ring members) and one to four heteroatom ring members independently selected from N, O, P or S, and derived by removal of one hydrogen atom from a ring atom of a parent ring system. One or more N, C or S atoms in the heterocycle can be oxidized. The ring that includes the heteroatom can be aromatic or nonaromatic. Unless otherwise noted, the heterocycle is attached to its pendant group at any heteroatom or carbon atom that results in a stable structure. Representative examples of a $C_3$-$C_8$ heterocycle include, but are not limited to, pyrrolidinyl, azetidinyl, piperidinyl, morpholinyl, tetrahydrofuranyl, tetrahydropyranyl, benzofuranyl, benzothiophene, indolyl, benzopyrazolyl, pyrrolyl, thiophenyl (thiophene), furanyl, thiazolyl, imidazolyl, pyrazolyl, pyrimidinyl, pyridinyl, pyrazinyl, pyridazinyl, isothiazolyl, and isoxazolyl. A"$C_3$-$C_8$ heteroaryl," is an aromatic $C_3$-$C_8$ heterocycle.

Unless otherwise indicated, "$C_3$-$C_8$ heterocyclo," by itself or as part of another term, refers to a $C_3$-$C_8$ heterocycle group defined above wherein one of the heterocycle group's hydrogen atoms is replaced with a bond (i.e., it is divalent). A "$C_3$-$C_8$ heteroarylene," by itself or as part of another term, refers to a $C_3$-$C_8$ heteroaryl group defined above wherein one of the heteroaryl group's hydrogen atoms is replaced with a bond (i.e., it is divalent).

Unless otherwise indicated, a "$C_3$-$C_8$ carbocycle," by itself or as part of another term, is a 3-, 4-, 5-, 6-, 7- or 8-membered monovalent, substituted or unsubstituted, saturated or unsaturated non-aromatic monocyclic or bicyclic carbocyclic ring derived by the removal of one hydrogen atom from a ring atom of a parent ring system. Representative -$C_3$-$C_8$ carbocycles include, but are not limited to, cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and cyclooctadienyl.

Unless otherwise indicated, a "$C_3$-$C_8$ carbocyclo," by itself or as part of another term, refers to a $C_3$-$C_8$ carbocycle group defined above wherein another of the carbocycle groups' hydrogen atoms is replaced with a bond (i.e., it is divalent).

Unless otherwise indicated, the term "heteroalkyl," by itself or in combination with another term, means, unless otherwise stated, a stable straight or branched chain hydrocarbon, or combinations thereof, fully saturated or containing from 1 to 3 degrees of unsaturation, consisting of the stated number of carbon atoms and from one to ten, preferably one to three, heteroatoms selected from the group consisting of O, N, Si and S, and wherein the nitrogen and sulfur atoms may optionally be oxidized and the nitrogen heteroatom may optionally be quaternized. The heteroatom(s) O, N and S may be placed at any interior position of the heteroalkyl group or at the position at which the alkyl group is attached to the remainder of the molecule. The heteroatom Si may be placed at any position of the heteroalkyl group, including the position at which the alkyl group is attached to the remainder of the molecule. Examples include $-CH_2-CH_2-O-CH_3$, $-CH_2-CH_2-NH-CH_3$, $-CH_2-CH_2-N(CH_3)-CH_3$, $-CH_2-S-CH_2-CH_3$, - $CH_2-CH_2-S(O)-CH_3$, $-NH-CH_2-CH_2-NH-C(O)-CH_2-CH_3$, $-CH_2-CH_2-S(O)_2-CH_3$, $-CH=CH-O-CH_3$, $-Si(CH_3)_3$, $-CH_2-CH=N-O-CH_3$, and $-CH=CH-N(CH_3)-CH_3$. Up to two heteroatoms may be consecutive, such as, for example, $-CH_2-NH-OCH_3$ and $-CH_2-O-Si(CH_3)_3$. In preferred embodiments, a $C_1$ to $C_4$ heteroalkyl or heteroalkylene has 1 to 4 carbon atoms and 1 or 2 heteroatoms and a $C_1$ to $C_3$ heteroalkyl or heteroalkylene has 1 to 3 carbon atoms and 1 or 2 heteroatoms. In some aspects, a heteroalkyl or

heteroalkylene is saturated.

Unless otherwise indicated, the term "heteroalkylene" by itself or as part of another substituent means a divalent group derived from heteroalkyl (as discussed above), as exemplified by $-CH_2-CH_2-S-CH_2-CH_2-$ and $-CH_2-S-CH_2-CH_2-NH-CH_2-$. For heteroalkylene groups, heteroatoms can also occupy either or both of the chain termini. Still further, for alkylene and heteroalkylene linking groups, no orientation of the linking group is implied.

"Substituted alkyl" and "substituted aryl" mean alkyl and aryl, respectively, in which one or more hydrogen atoms are each independently replaced with a substituent. Typical substituents include, but are not limited to, -X, -R, $-O^-$, -OR, -SR, $-S^-$, $-NR_2$, $-NR_3$, =NR, $-CX_3$, -CN, -OCN, -SCN, -N=C=O, -NCS, -NO, $-NO_2$, $=N_2$, $-N_3$, -NRC(=O)R, -C(=O)R, $-C(=O)NR_2$, $-SO_3^-$, $-SO_3H$, $-S(=O)_2R$, $-OS(=O)_2OR$, $-S(=O)_2NR$, -S(=O)R, $-OP(=O)(OR)_2$, $-P(=O)(OR)_2$, $-PO_3^-$, $-PO_3H_2$, $-AsO_2H_2$, -C(=O)R, -C(=O)X, -C(=S)R, $-CO_2R$, $-CO_2^-$, -C(=S)OR, -C(=O)SR, -C(=S)SR, $-C(=O)NR_2$, $-C(=S)NR_2$, or $-C(=NR)NR_2$, where each X is independently a halogen: - F, -Cl, -Br, or -I; and each R is independently -H, $-C_1-C_{20}$ alkyl, $-C_6-C_{20}$ aryl, $-C_3-C_{14}$ heterocycle, a protecting group or a prodrug moiety. Alkylene, carbocycle, carbocyclo, arylene, heteroalkyl, heteroalkylene, heterocycle, heterocyclo, heteroaryl, and heteroarylene groups as described above may also be similarly substituted.

RG is a reactive group that contains a reactive site (RS) that is capable of forming a bond with either the components of the Linker unit (i.e., A, W, Y) or the Drug unit D. RS is the reactive site within a Reactive Group (RG). Reactive groups include sulfhydryl groups to form disulfide bonds or thioether bonds, aldehyde, ketone, or hydrazine groups to form hydrazone bonds, carboxylic or amino groups to form peptide bonds, carboxylic or hydroxy groups to form ester bonds, sulfonic acids to form sulfonamide bonds, alcohols to form carbamate bonds, and amines to form sulfonamide bonds or carbamate bonds. The following table is illustrative of Reactive Groups, Reactive Sites, and exemplary functional groups that can form after reaction of the reactive site. The table is not limiting. One of skill in the art will appreciate that the noted R' and R" portions in the table are effectively any organic moiety (e.g., an alkyl group, aryl group, heteroaryl group, or substituted alkyl, aryl, or heteroaryl, group) which is compatible with the bond formation provided in converting RG to one of the Exemplary Functional Groups. It will also be appreciated that, as applied to the embodiments of the present invention, R' may represent one or more components of the self-stabilizing linker or optional secondary linker, as the case may be, and R" may represent one or more components of the optional secondary linker, Drug unit, Stability unit, or Detection unit, as the case may be.

| RG | RS | Exemplary Functional Groups |
|---|---|---|
| 1) R'-SH | -S- | R'-S-R"<br>R'-S-S-R" |
| 2) R'-C(=O)OH | -C(=O)- | R'-C(=O)NH-R" |
| 3) R'-C(=O)ONHS | -C(=O)- | R'-C(=O)NH-R" |
| 4) $R'S(=O)_2-OH$ | $-S(=O)_2-$ | $R'S(=O)_2NH-R"$ |
| 5) $R'-CH_2-X$ (X is Br, I, Cl) | $-CH_2-$ | $R'-CH_2-S-R"$ |
| 6) $R'-NH_2$ | -N- | R'-NHC(=O)R" |

It will be understood that, once reacted, the reactive site RS can form a new bond with components of the Linker unit or the Drug unit, as the case may be. The reactive site, RS, once linked to the remainder of the Linker unit has typically lost its reactivity.

The term "dilactam" as used herein refers to a cyclic amide that forms from a macro-cyclicization reaction with a thio-substituted succinimide and base present on the self-stabilizing linker assembly.

**General**

Hydrolysis of a maleimide (or thio-substituted succinimide) represents a nucleophilic addition reaction in which water, acting as the nucleophile, attacks one of the electrophilic carbonyl carbon atoms of the maleimide ring (or succinimide ring). The rate of this reaction is influenced by electrophilicity of the carbonyls, which can vary with the substitution of electron-donating or electron-withdrawing groups present on the nitrogen of the imide group. The rate of the hydrolysis reaction is also influenced by the pH of an aqueous solvent, which effectively increases the nucleophilicity of water with increasing pH. It has been discovered by the present inventors that the placement of a basic group on an N-substituted maleimide also increases the rate of the hydrolysis. By careful engineering of an N-substituent group on the maleimide, the combination of its electron withdrawing influence on the maleimide ring (thus increasing its electrophilicity) and localized basicity (increasing the effective nucleophilicity of nearby water) can be used to tune the rate of hydrolysis of

either the parent maleimide or its thio-substituted succinimide derivative. The present invention provides, *inter alia,* N-substituted maleimides with hydrolysis rates that fall within a useful range wherein their reaction with thiols occurs more quickly than their hydrolysis to the maleic acid derivative, but which yield thio-substituted succinimides with hydrolysis rates that are sufficiently rapid to achieve complete hydrolysis under gentle conditions that are very suitable for the manufacture of protein-based bioconjugates.

The present invention is based, in part, on the discovery that a basic functional group proximal to a maleimide will catalyze the hydrolysis of a thio-substituted succinimide which is formed upon conjugation of the maleimide and a protein thiol leading to a stable bioconjugate. By further combining a proximal basic group with an electron withdrawing group, the rate of thio-substituted succinimide ring hydrolysis can be tuned to a desirable level. Design parameters that affect the rate of hydrolysis include the pKa of the basic group, the strength of the electron withdrawing group when present, and the proximity of both groups to the maleimide carbonyl carbons. Design parameters that affect the percentage hydrolysis include the nature and proximity of the base to the maleimide carbonyl carbons.

Conceptually, without limiting the invention, a Linker unit comprising a self-stabilizing linker assembly is referred to herein as a Self-Stabilizing Linker or Self-Stabilizing Linker unit. The Self-Stabilizing Linker prior to conjugation with the Ligand unit comprises a maleimide group. The Self-Stabilizing Linker is self-stabilizing by virtue of the proximity of the maleimide group to a base within the Linker unit which catalyzes the hydrolysis of its own thio-substituted succinimide after conjugation to the Ligand unit. This is represented schematically below:

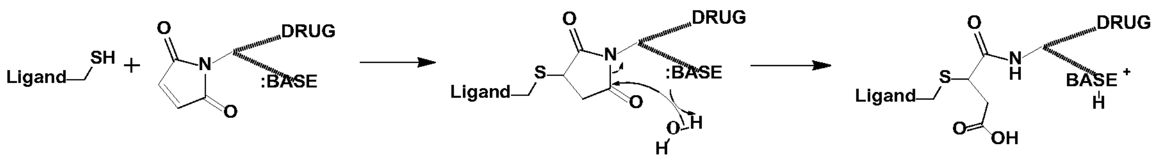

It will be understood that the term Self-Stabilizing Linker refers to the Linker unit both prior to and post stabilization.

In view of the above, the present invention as defined by the subject-matter of the claims provides in one group of embodiments, a Ligand-Functional Agent Conjugate comprising a Ligand unit and at least one Functional Agent selected from a Drug unit, a Detection Unit, or a Stabilizing Unit, wherein the Ligand unit and each of the Functional Agent(s) are joined by a self-stabilizing linker assembly comprising a succinimide ring or a hydrolyzed succinimide ring directly conjugated to the Ligand unit via a thioether linkage; and a base and an electron withdrawing group (conjugated to the Ligand unit via the succinimide) operably linked to stabilize the conjugate in plasma relative to a ligand drug conjugate lacking the self-stabilizing linker assembly (i.e. by increasing the rate of succinimide ring hydrolysis). In some aspects, the electron withdrawing group is positioned to increase the electrophilicity of the succinimide rendering it more reactive with water and the base is positioned to assist the hydrolysis of the succinimide ring (e.g., by an intramolecular base catalysis mechanism). In some aspects, in place of the succinimide ring is a dilactam formed when the base reacts with the succinimide ring. In another group of embodiments, Functional Agent-Linker units are provided wherein the Linker portion comprises a self-stabilizing linker assembly. In another group of embodiments, Ligand-Linker conjugates are provided, wherein the Linker portion comprises a self-stabilizing linker assembly. In some embodiments, the Linker portion further comprises an optional secondary linker assembly ($L^O$).

In some aspects, the Ligand-Functional Agent Conjugate is a Ligand-Drug Conjugate. Accordingly, the present invention as defined by the subject-matter of the claims provides in one group of embodiments, a Ligand-Drug Conjugate comprising a Ligand unit and at least one Drug unit, wherein the Ligand unit and each of the Drug unit(s) are joined by a self-stabilizing linker assembly comprising a succinimide ring or a hydrolyzed succinimide ring directly conjugated to the Ligand unit via a thioether linkage; and a base and an electron withdrawing group (conjugated to the Ligand unit via the succinimide ring) operably linked to stabilize the conjugate in plasma relative to a ligand drug conjugate lacking the self-stabilizing linker assembly (i.e. by increasing the rate of succinimide ring hydrolysis). In some aspects, the electron withdrawing group is positioned to increase the electrophilicity of the succinimide rendering it more reactive with water and the base is positioned to assist the hydrolysis of the succinimide ring (e.g., by an intramolecular base catalysis mechanism). In some aspects, in place of the succinimide ring is a dilactam formed when the base reacts with the succinimide ring. In another group of embodiments, Drug-Linker units are provided wherein the Linker portion comprises a self-stabilizing linker assembly. In another group of embodiments, Ligand-Linker conjugates are provided, wherein the Linker portion comprises a self-stabilizing linker assembly. In some embodiments, the Linker portion further comprises an optional secondary linker assembly ($L^O$). In some embodiments, the secondary linker assembly is a releasable linker assembly ($L^R$) which comprises a Cleavable unit and optionally one or more of a Stretcher and a Spacer unit. In some other embodiments, the secondary linker assembly is a non-releasable linker assembly ($L^N$) which comprises one or more of a Stretcher unit and a Spacer unit. In still other embodiments, the invention provides methods of treating cancer, immune disease, infectious diseases and other diseases and disorders using a Ligand-Drug Conjugate comprising a

self-stabilizing linker assembly.

The Linker unit of the Ligand-Functional Agent Conjugate (or Ligand-Drug Conjugate) can further comprise, in addition to a self-stabilizing linker assembly, an optional secondary linker assembly ($L^O$) which joins each Functional Agent (or Drug unit) to the self-stabilizing linker assembly. The secondary linker assembly can be a releasable linker assembly or a non-releasable linker assembly.

The term Linker unit can be used herein to refer to the linker portion of the Ligand-Functional Agent Conjugate (or Ligand-Drug Conjugate) comprising the self-stabilizing linker assembly and optional secondary linker assembly.

**The Self-Stabilizing Linker Assembly**

The Self-Stabilizing Linkers are designed such that the rate of the post-conjugation hydrolysis of the succinimide ring will be controllable and fall within a desired range. The limits of this range are typically dictated by issues which arise in the manufacture of ligand-drug conjugates. On the one hand, hydrolysis which is too slow would require unacceptable delays in the manufacturing process or aggressive conditions of pH and temperature which may induce damage to the protein backbone. Conversely, a maleimide which is too reactive with water may be hydrolyzed to the corresponding maleic acid derivative before it can react with available protein thiols (see undesired pathway):

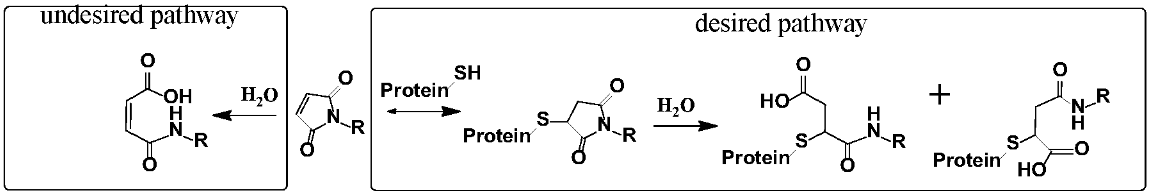


hydrolyzed maleimide (maleic acid derivative) maleimide thio-substituted succinimide hydrolyzed succinimide (2 possible positional isomers)

Such maleic acid derivatives are not reactive with thiols, and thus this reaction pathway does not result in a bioconjugate. Therefore, maleimides which undergo hydrolysis faster than thiol addition under applicable conditions are not useful reagents. In general, structural features which increase the hydrolysis rate of a thio-substituted succinimide will also increase the hydrolysis rate of the parent maleimide.

In designing the Self-Stabilizing Linkers of the present invention, it will be understood that the pKa of the basic group, the strength of the electron withdrawing group(s), and the proximity of both groups to the maleimide are interrelated variables and will affect the hydrolysis rate of both the maleimide and corresponding thio-substituted succinimide product. Accordingly, positioning of the electron withdrawing group and base will be dependent upon the pKa of the base and the strength of the electron withdrawing group(s). The skilled artisan will understand that for particularly strong electron withdrawing groups such as fluoro, trifluoromethyl, and nitro, the group can be further from the maleimide. In some embodiments, the hydrolysis reaction may compete with a macro-cyclization reaction such that the resultant conjugates comprise a heterogenous mixture of hydrolyzed thio-substituted succinimide conjugates and cyclized thio-substituted dilactam conjugates. In preferred embodiments, a dilactam will not be formed.

**Selected Embodiments of the Invention**

In selected embodiments, Ligand-Drug Conjugates have the formula:

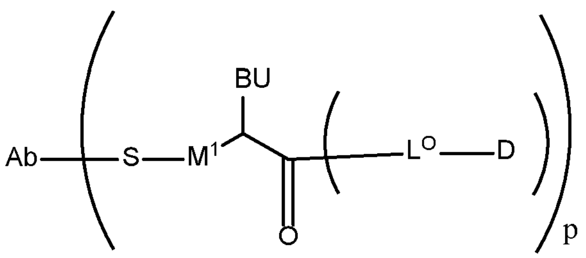

or a pharmaceutically acceptable salt thereof, wherein the Ligand portion is an antibody (Ab), the subscript p ranges from 1 to 20 (preferably 1 to 12), and $M^1$ is a succinimide ring or hydrolyzed succinimide or together with BU forms a dilactam resulting from the reaction of the base of BU with the succinimide ring and D is a Drug unit. Where S represents

a sulfur atom of the antibody. In some aspects, $L^o$ is a releasable linker assembly having the formula:

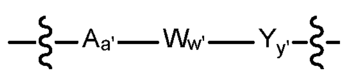

,

wherein -A- is an optional Stretcher unit, the subscript a' is 0 or 1; - W- is an optional Cleavable unit, the subscript w' is 0 or 1; and -Y- is an optional Spacer unit, and the subscript y' is 0 or 1; and the wavy lines indicate the points of attachment to the self-stabilizing linker assembly and the Drug unit. BU is $-(CH_2)_xNH_2$, $-(CH_2)_xNHR^a$, and $-(CH_2)_xNR^a{}_2$, wherein x is an integer of from 1-4 and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the succinimide (hydrolyzed or non-hydrolyzed) or dilactam. In yet other aspects, $L^O$ is a releasable linker assembly, and BU is $-CH_2NH_2$, $-CH_2CH_2NH_2$, $-CH_2CH_2CH_2NH_2$, or $-CH_2CH_2CH_2CH_2NH_2$. In some aspects, the Ab can be replaced by a non-antibody protein. $M^1$ is preferably a succinimide ring or a hydrolyzed succinimide ring.

In yet other selected embodiments, the Ligand-Drug Conjugates have the formula:

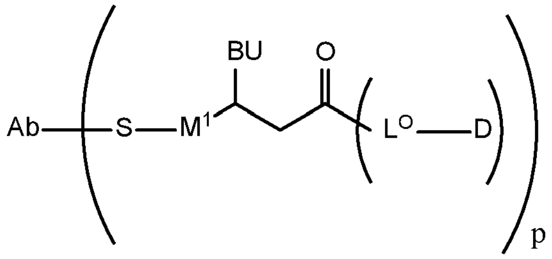

or a pharmaceutically acceptable salt thereof wherein the Ligand portion is an antibody (Ab) and the subscript p ranges from 1 to 20 (preferably 1 to 12) and $M^1$, BU, and $L^O$ are as described in any of the embodiments provided herein, and D is a Drug unit. Where S represents a sulfur atom of the antibody. $M^1$ is a succinimide ring or hydrolyzed succinimide or together with BU forms a dilactam resulting from the reaction of the base of BU with the succinimide ring. For example, in some aspects, $L^O$ is a releasable linker assembly having the formula:

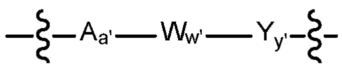

,

wherein -A- is an optional Stretcher unit, the subscript a' is 0 or 1; - W- is an optional Cleavable unit, the subscript w' is 0 or 1; and -Y- is an optional Spacer unit, and the subscript y' is 0 or 1; and the wavy lines indicate the points of attachment to the self-stabilizing linker assembly and the Drug unit. BU is $-(CH_2)_xNH_2$, $-(CH_2)_xNHR^a$, and $-(CH_2)_xNR^a{}_2$, wherein x is an integer of from 1-4 and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the succinimide (hydrolyzed or non-hydrolyzed) or dilactam. In yet other aspects, $L^O$ is a releasable linker assembly, and BU is $-CH_2NH_2$, $-CH_2CH_2NH_2$, $-CH_2CH_2CH_2NH_2$, or $-CH_2CH_2CH_2CH_2NH_2$. In some aspects, the Ab can be replaced by a non-antibody protein. $M^1$ is preferably a succinimide ring or a hydrolyzed succinimide ring.

In yet other selected embodiments, the Ligand-Drug Conjugates have the formula:

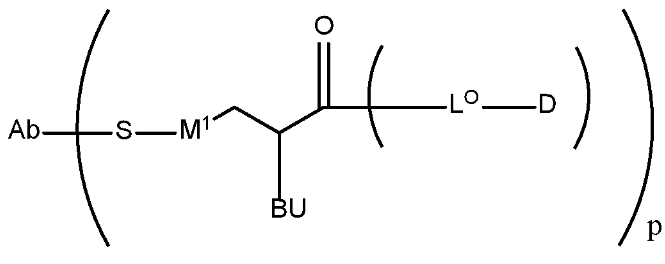

or a pharmaceutically acceptable salt thereof wherein the Ligand portion is an antibody (Ab), the subscript p ranges

from 1 to 20 (preferably 1 to 12), and $M^1$, BU, and $L^O$ are as described in any of the embodiments provided herein and D is a Drug unit. Where S represents a sulfur atom of the antibody. $M^1$ is a succinimide ring or hydrolyzed succinimide or together with BU forms a dilactam resulting from the reaction of the base of BU with the succinimide ring. For example, in some aspects, $L^O$ is a releasable linker assembly, having the formula:

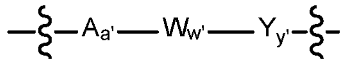
,

wherein -A- is an optional Stretcher unit, the subscript a' is 0 or 1; - W- is an optional Cleavable unit, the subscript w' is 0 or 1; and -Y- is an optional Spacer unit, and the subscript y' is 0 or 1; and the wavy lines indicate the points of attachment to the self-stabilizing linker assembly and the Drug unit. BU is $-(CH_2)_xNH_2$, $-(CH_2)_xNHR^a$, and $-(CH_2)_xNR^a{}_2$, wherein x is an integer of from 0-4 and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the succinimide (hydrolyzed or non-hydrolyzed) or dilactam. In yet other aspects, $L^O$ is a releasable linker assembly, and BU is $-NH_2$, $-CH_2NH_2$, $-CH_2CH_2NH_2$, $-CH_2CH_2CH_2NH_2$, or $-CH_2CH_2CH_2CH_2NH_2$. In some aspects, the Ab can be replaced by a non-antibody protein. $M^1$ is preferably a succinimide ring or a hydrolyzed succinimide ring.

Having described a variety of Ligand-Functional Agent Conjugates and Ligand-Drug Conjugates provided by the present disclosure, one of skill in the art will appreciate that component assemblies are also useful. Accordingly, the present invention provides Fuctional Agent-Linker Conjugates (e.g., Drug-Linker Conjugates), Linkers, and Ligand-Linker assemblies.

**Functional Agent-Linker Conjugates**

In certain selected embodiments, the Functional Agent-Linker Conjugate is represented by the formula:

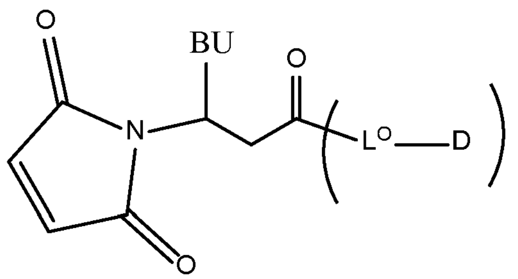
,

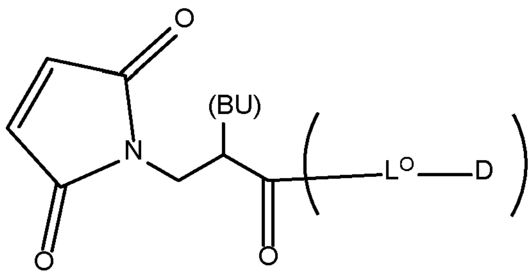

or

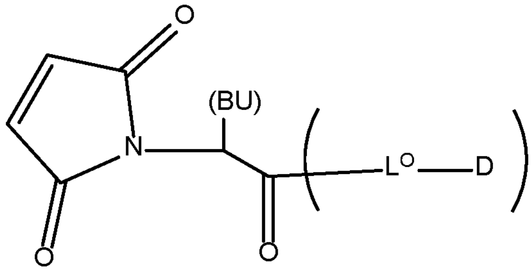

or a salt thereof, wherein the BU and $L^O$ have the meanings provided herein and D is a Drug unit. Additionally, each of the specifically recited selected embodiments for BU and $L^O$ (for any of the conjugates provided herein) are equally applicable to these Drug-Linker Conjugates. BU is a Basic Unit selected from the group consisting of $-(CH_2)_xNH_2$, $-(CH_2)_xNHR^a$, and $-(CH_2)_xNR^a{}_2$, wherein x is an integer of from 0-4 and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the maleimide. $L^O$ is an optional secondary linker assembly having the formula:

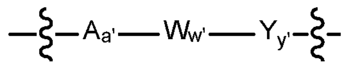

wherein -A- is an optional Stretcher unit, the subscript a' is 0 or 1; -W- is an optional Cleavable unit, the subscript w' is 0 or 1; and -Y- is an optional Spacer unit, and the subscript y' is 0 or 1; and the wavy lines indicate the points of attachment to the self-stabilizing linker assembly and the Drug unit.

**Linkers**

Also disclosed herein are Linkers having the formula:

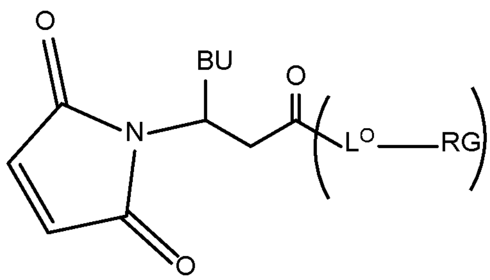

,

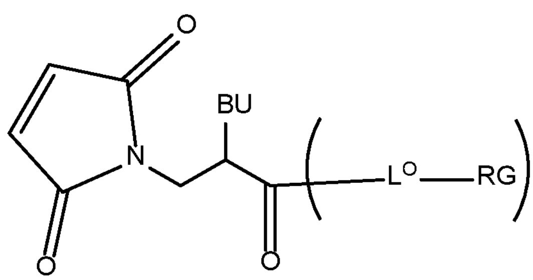

or

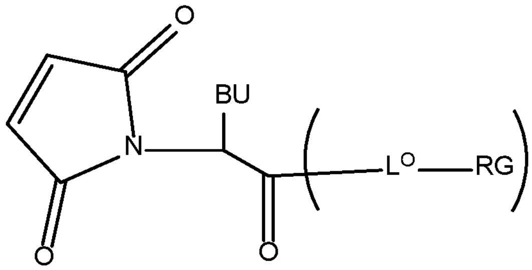

or a salt thereof (e.g., pharmaceutically acceptable salt) wherein BU and $L^O$ have the meanings provided above. RG is a reactive group (comprising a reactive site) at the terminus of $L^O$, suitable for attaching a Drug unit. Additionally, each of the specifically recited selected embodiments for BU, $L^O$ and RG (for any of the conjugates provided herein) are equally applicable to these Linkers.

**Ligand-Linker Conjugates**

Also disclosed herein are Ligand-Linker Conjugates, represented by the formula:

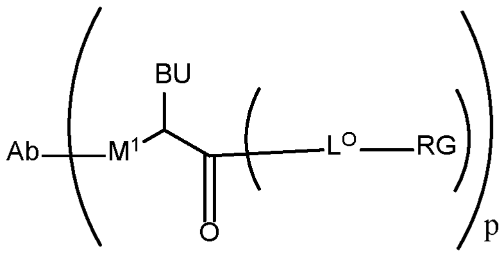

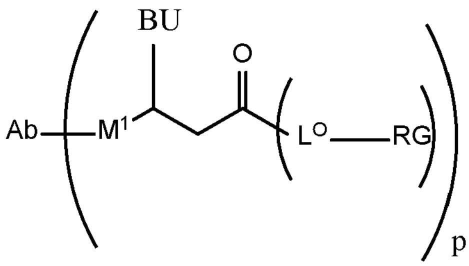

or

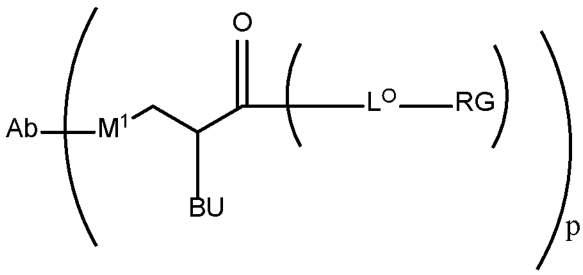

or a salt thereof (e.g., pharmaceutically acceptable salt), wherein L is an antibody (Ab), and $M^1$, BU, $L^O$ and RG have the meanings provided above. Additionally, each of the specifically recited selected embodiments for Ab, $M^1$, BU, $L^O$ and RG (for any of the conjugates provided herein) are equally applicable to these Ligand-Linker conjugates.

In each of the selected embodiments wherein the Ligand-Drug Conjugates, Drug-Linkers, Linkers, or Ligand-Linker or pharmaceutically acceptable salts thereof, the optional secondary linker assembly can be represented by the following formula:

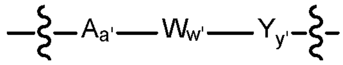

wherein -A- is an optional Stretcher unit, the subscript a' is 0 or 1 -W- is an optional Cleavable unit, the subscript w' is 0 or 1; and -Y- is an optional Spacer unit, and the subscript y' is 0 or 1.

**Further Embodiments of the Invention**

In some aspects of the present invention, a self-stabilizing linker assembly may undergo macro-cyclization to form a dilactam as follows wherein R represents the remainder of the conjugate:

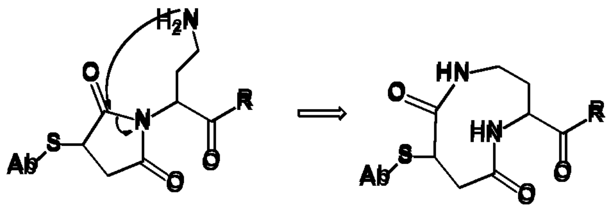

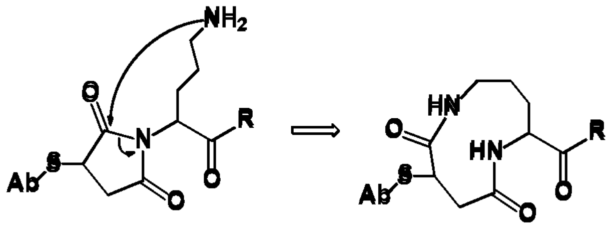

**Secondary Linker Assembly**

The optional secondary linker can comprise a variety of linking groups. In each of the embodiments provided herein, including the specifically recited embodiments, $L^O$ can be present and have the formula:

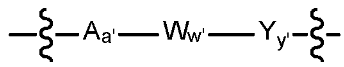

wherein

- -A- is an optional Stretcher unit, the subscript a' is 0 or 1;
- -W- is an optional Cleavable unit, the subscript w' is 0 or 1; and
- -Y- is an optional Spacer unit, and the subscript y' is 0 or 1;

The optional secondary linker assembly can be a releaseable linker assembly, $L^R$. In those embodiments, w is 1. In some other aspects, the optional secondary linker assembly is a non-releasable linker assembly. In those embodiments w is 0 and release of drug is via a total protein degradation pathway (*i.e.*, non-cleavable pathway).

**The Ligand Unit**

In some embodiments of the invention, a Ligand Unit is present. The Ligand unit (L-) is a targeting agent that specifically binds to a target moiety. The Ligand can specifically bind to a cell component (a Cell Binding Agent) or to other target molecules of interest. In some aspects, the Ligand unit acts to deliver the Drug unit to the particular target cell population with which the Ligand unit interacts. Ligands include, but are not limited to, proteins, polypeptides and peptides. Suitable Ligand units include, for example, antibodies, e.g., full-length antibodies and antigen binding fragments thereof, interferons, lymphokines, hormones, growth factors and colony-stimulating factors, vitamins, nutrient-transport molecules (such as, but not limited to, transferrin), or any other cell binding molecule or substance. In some aspects, the ligand is a non-antibody protein targeting agent. In some aspects, a Ligand-Functional Agent is provided wherein

D' is a Detection Unit or Stability unit and the Ligand unit is a protein (e.g., a non-antibody protein).

In some aspects, a Ligand unit forms a bond with the maleimide of the Self-Stabilizing Basic unit via a sulfhydryl group of the Ligand to form a thio-substituted succinimide. The sulfhydryl group can be present on the Ligand in the Ligand's natural state, for example a naturally-occurring residue, or can be introduced into the Ligand via chemical modification.

It has been observed for bioconjugates that the site of drug conjugation can affect a number of parameters including ease of conjugation, drug-linker stability, effects on biophysical properties of the resulting bioconjugates, and in-vitro cytotoxicity. With respect to drug-linker stability, the site of conjugation of a drug-linker to a ligand can affect the ability of the conjugated drug-linker to undergo an elimination reaction and for the drug linker to be transferred from the ligand of a bioconjugate to an alternative reactive thiol present in the milieu of the bioconjugate, such as, for example, a reactive thiol in albumin, free cysteine, or glutathione when in plasma. Use of the Self-Stabilizing Linkers of the present invention is particularly beneficial when conjugated to thiol residues at sites that are susceptible to the elimination reaction and subsequent transfer of drug-linker if non-self- stabilizing alkyl maleimides are used (e.g., maleimido-caproyl drug linker). Such sites include, for example, the interchain disulfides as well as select cysteine engineered sites. Use of the Self-Stabilizing Linkers of the present invention provides a stable linkage and ability to attach multiple drugs to each Ligand unit.

In one aspect, the Ligand unit has one or more lysine residues that can be chemically modified to introduce one or more sulfhydryl groups. The reagents that can be used to modify lysines include, but are not limited to, N-succinimidyl S-acetylthioacetate (SATA) and 2-Iminothiolane hydrochloride (Traut's Reagent).

In another embodiment, the Ligand unit can have one or more carbohydrate groups that can be chemically modified to have one or more sulfhydryl groups.

In another embodiment, the Ligand is an antibody and the sulfhydryl group is generated by reduction of an interchain disulfide. Accordingly, in some embodiments, the Linker unit is conjugated to a cysteine residue of the reduced interchain disulfides.

In another embodiment, the sulfhydryl group is chemically introduced into the antibody, for example by introduction of a cysteine residue. Accordingly, in some embodiments, the Linker unit is conjugated to an introduced cysteine residue.

Useful non-immunoreactive protein, polypeptide, or peptide Ligands include, but are not limited to, transferrin, epidermal growth factors ("EGF"), bombesin, gastrin, gastrin-releasing peptide, platelet-derived growth factor, IL-2, IL-6, transforming growth factors ("TGF"), such as TGF-$\alpha$ and TGF-$\beta$, vaccinia growth factor ("VGF"), insulin and insulin-like growth factors I and II, somatostatin, lectins and apoprotein from low density lipoprotein.

Particularly preferred ligands are antibodies. Useful polyclonal antibodies are heterogeneous populations of antibody molecules derived from the sera of immunized animals. Useful monoclonal antibodies are homogeneous populations of antibodies to a particular antigenic determinant (e.g., a cancer cell antigen, a viral antigen, a microbial antigen, a protein, a peptide, a carbohydrate, a chemical, nucleic acid, or fragments thereof). A monoclonal antibody (mAb) to an antigen-of-interest can be prepared by using any technique known in the art which provides for the production of antibody molecules by continuous cell lines in culture.

Useful monoclonal antibodies include, but are not limited to, human monoclonal antibodies, humanized monoclonal antibodies, or chimeric human-mouse (or other species) monoclonal antibodies. The antibodies include full-length antibodies and antigen binding fragments thereof. Human monoclonal antibodies may be made by any of numerous techniques known in the art (*e.g.,* Teng et al., 1983, Proc. Natl. Acad. Sci. USA. 80:7308-7312; Kozbor et al., 1983, Immunology Today 4:72-79; and Olsson et al., 1982, Meth. Enzymol. 92:3-16).

The antibody can be a functionally active fragment, derivative or analog of an antibody that immunospecifically binds to target cells (*e.g.,* cancer cell antigens, viral antigens, or microbial antigens) or other antibodies bound to tumor cells or matrix. In this regard, "functionally active" means that the fragment, derivative or analog is able to elicit anti-anti-idiotype antibodies that recognize the same antigen that the antibody from which the fragment, derivative or analog is derived. Specifically, in an exemplary embodiment the antigenicity of the idiotype of the immunoglobulin molecule can be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art *(e.g.,* the BIA core assay) *(See, e.g.,* Kabat et al., 1991, Sequences of Proteins of Immunological Interest, Fifth Edition, National Institute of Health, Bethesda, Md; Kabat E et al., 1980, J. Immunology 125(3):961-969).

Other useful antibodies include fragments of antibodies such as, but not limited to, $F(ab')_2$ fragments, Fab fragments, Fvs, single chain antibodies, diabodies, tribodies, tetrabodies, scFv, scFv-FV, or any other molecule with the same specificity as the antibody.

Additionally, recombinant antibodies, such as chimeric and humanized monoclonal antibodies, comprising both human and non-human portions, which can be made using standard recombinant DNA techniques, are useful antibodies. A chimeric antibody is a molecule in which different portions are derived from different animal species, such as for

example, those having a variable region derived from a murine monoclonal and human immunoglobulin constant regions. (*See, e.g.,* U.S. Patent No. 4,816,567; and U.S. Patent No. 4,816,397.) Humanized antibodies are antibody molecules from non-human species having one or more complementarity determining regions (CDRs) from the non-human species and a framework region from a human immunoglobulin molecule. (*See, e.g.,* U.S. Patent No. 5,585,089.) Such chimeric and humanized monoclonal antibodies can be produced by recombinant DNA techniques known in the art, for example using methods described in International Publication No. WO 87/02671; European Patent Publication No. 0 184 187; European Patent Publication No. 0 171 496; European Patent Publication No. 0 173 494; International Publication No. WO 86/01533; U.S. Patent No. 4,816,567; European Patent Publication No.012 023; Berter et al., 1988, Science 240: 1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Cancer. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, BioTechniques 4:214; U.S. Patent No. 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable and can be produced using transgenic mice that are incapable of expressing endogenous immunoglobulin heavy and light chains genes, but which can express human heavy and light chain genes.

Antibodies include analogs and derivatives that are either modified, *i.e.,* by the covalent attachment of any type of molecule as long as such covalent attachment permits the antibody to retain its antigen binding immunospecificity. For example, but not by way of limitation, derivatives and analogs of the antibodies include those that have been further modified, *e.g.*, by glycosylation, acetylation, pegylation, phosphorylation, amidation, derivatization by known protecting/blocking groups, proteolytic cleavage, linkage to a cellular antibody unit or other protein, etc. Any of numerous chemical modifications can be carried out by known techniques including, but not limited to, specific chemical cleavage, acetylation, formylation, metabolic synthesis in the presence of tunicamycin, etc. Additionally, the analog or derivative can contain one or more unnatural amino acids.

Antibodies can have modifications (*e.g.*, substitutions, deletions or additions) in amino acid residues that interact with Fc receptors. In particular, antibodies can have modifications in amino acid residues identified as involved in the interaction between the anti-Fc domain and the FcRn receptor (*see, e.g.,* International Publication No. WO 97/34631).

Antibodies immunospecific for a cancer cell antigen can be obtained commercially or produced by any method known to one of skill in the art such as, *e.g.*, chemical synthesis or recombinant expression techniques. The nucleotide sequence encoding antibodies immunospecific for a cancer cell antigen can be obtained, *e.g.*, from the GenBank database or a database like it, the literature publications, or by routine cloning and sequencing.

In a specific embodiment, known antibodies for the treatment of cancer can be used. Antibodies immunospecific for a cancer cell antigen can be obtained commercially or produced by any method known to one of skill in the art such as, *e.g.*, recombinant expression techniques. The nucleotide sequence encoding antibodies immunospecific for a cancer cell antigen can be obtained, *e.g.*, from the GenBank database or a database like it, the literature publications, or by routine cloning and sequencing.

Antibodies for the treatment of an autoimmune disease can be used. Antibodies immunospecific for an antigen of a cell that is responsible for producing autoimmune antibodies can be obtained from any organization (*e.g.*, a university scientist or a company) or produced by any method known to one of skill in the art such as, *e.g.*, chemical synthesis or recombinant expression techniques. In another embodiment, useful antibodies are immunospecific for the treatment of autoimmune diseases include, but are not limited to, anti-nuclear antibody; anti-ds DNA; Anti-ss DNA, anti-cardiolipin antibody IgM, IgG; anti-phospholipid antibody IgM, IgG; anti-SM antibody; anti-mitochondrial antibody; thyroid antibody; microsomal antibody; thyroglobulin antibody; anti-SCL-70 antibody; anti-Jo antibody; anti-$U_1$RNP antibody; anti-La/SSB antibody; anti-SSA; anti-SSB antibody; anti-perital cells antibody; anti-histones antibody; anti-RNP antibody; C-ANCA antibody; P-ANCA antibody; anti-centromere antibody; Anti-Fibrillarin antibody and anti-GBM antibody.

In certain embodiments, useful antibodies can bind to a receptor or a receptor complex expressed on an activated lymphocyte. The receptor or receptor complex can comprise an immunoglobulin gene superfamily member, a TNF receptor superfamily member, an integrin, a cytokine receptor, a chemokine receptor, a major histocompatibility protein, a lectin, or a complement control protein. Non-limiting examples of suitable immunoglobulin superfamily members are CD2, CD3, CD4, CD8, CD19, CD20, CD22, CD28, CD30, CD70, CD79, CD90, CD152/CTLA-4, PD-1, and ICOS. Non-limiting examples of suitable TNF receptor superfamily members are CD27, CD40, CD95/Fas, CD134/OX40, CD137/4-1BB, TNF-R1, TNFR-2, RANK, TACI, BCMA, osteoprotegerin, Apo2/TRAII,-R1, TRAIL-R2, TRAIL-R3, TRAIL-R4, and APO-3. Non-limiting examples of suitable integrins are CD1 1a, CD11b, CD11c, CD18, CD29, CD41, CD49a, CD49b, CD49c, CD49d, CD49e, CD49f, CD103, and CD104. Non-limiting examples of suitable lectins are C-type, S-type, and I-type lectin.

**The Drug Unit, D**

The drug unit (D) can be any cytotoxic, cytostatic or immunosuppressive drug also referred to herein as a cytotoxic, cytostatic or immunosuppressive agent. The Drug unit has an atom that can form a bond with the Linker Unit. In some embodiments, the Drug unit D has a nitrogen atom that can form a bond with the Linker unit. In other embodiments, the Drug unit D has a carboxylic acid that can form a bond with the Linker unit. In other embodiments, the Drug unit D has a sulfhydryl group that can form a bond with the Linker unit. In other embodiments, the Drug unit D has a hydroxyl group or ketone that can form a bond with the Linker unit.

Useful classes of cytotoxic or immunosuppressive agents include, for example, antitubulin agents, auristatins, DNA minor groove binders, DNA replication inhibitors, alkylating agents (*e.g.*, platinum complexes such as cis-platin, mono(platinum), bis(platinum) and tri-nuclear platinum complexes and carboplatin), anthracyclines, antibiotics, antifolates, antimetabolites, chemotherapy sensitizers, duocarmycins, etoposides, fluorinated pyrimidines, ionophores, lexitropsins, nitrosoureas, platinols, pre-forming compounds, purine antimetabolites, puromycins, radiation sensitizers, steroids, taxanes, topoisomerase inhibitors, vinca alkaloids, or the like. Particularly examples of useful classes of cytotoxic agents include, for example, DNA minor groove binders, DNA alkylating agents, and tubulin inhibitors. Exemplary cytotoxic agents include, for example, auristatins, camptothecins, duocarmycins, etoposides, maytansines and maytansinoids (e.g., DM1 and DM4), taxanes, benzodiazepines (*e.g.*, pyrrolo[1,4]benzodiazepines (PBDs), indolinobenzodiazepines, and oxazolidinobenzodiazepines) and vinca alkaloids. Select benzodiazepine containing drugs are described in WO 2010/091150, WO 2012/112708, WO 2007/085930, and WO 2011/023883.

Individual cytotoxic or immunosuppressive agents include, for example, an androgen, anthramycin (AMC), asparaginase, 5-azacytidine, azathioprine, bleomycin, busulfan, buthionine sulfoximine, calicheamicin, camptothecin, carboplatin, carmustine (BSNU), CC-1065, chlorambucil, cisplatin, colchicine, cyclophosphamide, cytarabine, cytidine arabinoside, cytochalasin B, dacarbazine, dactinomycin (formerly actinomycin), daunorubicin, decarbazine, docetaxel, doxorubicin, etoposide, an estrogen, 5-fluordeoxyuridine, 5-fluorouracil, gemcitabine, gramicidin D, hydroxyurea, idarubicin, ifosfamide, irinotecan, lomustine (CCNU), maytansine, mechlorethamine, melphalan, 6-mercaptopurine, methotrexate, mithramycin, mitomycin C, mitoxantrone, nitroimidazole, paclitaxel, palytoxin, plicamycin, procarbizine, rhizoxin, streptozotocin, tenoposide, 6-thioguanine, thioTEPA, topotecan, vinblastine, vincristine, vinorelbine, VP-16 and VM-26.

In some typical embodiments, suitable cytotoxic agents include, for example, DNA minor groove binders (*e.g.*, enediynes and lexitropsins, a CBI compound; see also U.S. Patent No. 6,130,237), duocarmycins (see U.S. Publication No. 20060024317), taxanes (*e.g.*, paclitaxel and docetaxel), puromycins, vinca alkaloids, CC-1065, SN-38, topotecan, morpholino-doxorubicin, rhizoxin, cyanomorpholino-doxorubicin, echinomycin, combretastatin, netropsin, epothilone A and B, estramustine, cryptophysins, cemadotin, maytansinoids, discodermolide, eleutherobin, and mitoxantrone.

In some embodiments, the Drug unit is an anti-tubulin agent. Examples of anti-tubulin agents include, but are not limited to, taxanes (*e.g.*, Taxol® (paclitaxel), Taxotere® (docetaxel)), T67 (Tularik) and vinca alkyloids (*e.g.*, vincristine, vinblastine, vindesine, and vinorelbine). Other antitubulin agents include, for example, baccatin derivatives, taxane analogs (*e.g.*, epothilone A and B), nocodazole, colchicine and colcimid, estramustine, cryptophysins, cemadotin, maytansinoids, combretastatins, discodermolide, and eleutherobin.

In certain embodiments, the cytotoxic agent is maytansine or a maytansinoid, another group of anti-tubulin agents. (ImmunoGen, Inc.; see also Chari et al., 1992, Cancer Res. 52:127-131 and U.S. Patent No. 8,163,888).

In some embodiments, the Drug unit is an auristatin. Auristatins include, but are not limited to, AE, AFP, AEB, AEVB, MMAF, and MMAE. The synthesis and structure of auristatins are described in U.S. Patent Application Publication Nos. 2003-0083263, 2005-0238649 2005-0009751, 2009-0111756, and 2011-0020343; International Patent Publication No. WO 04/010957, International Patent Publication No. WO 02/088172, and U.S. Patent Nos. 7,659,241 and 8,343,928. Exemplary auristatins of the present invention bind tubulin and exert a cytotoxic or cytostatic effect on the desired cell line.

Exemplary auristatin Drug units have the following formula or a pharmaceutically acceptable salt thereof wherein the wavy line indicates site of attachment to the Linker unit:

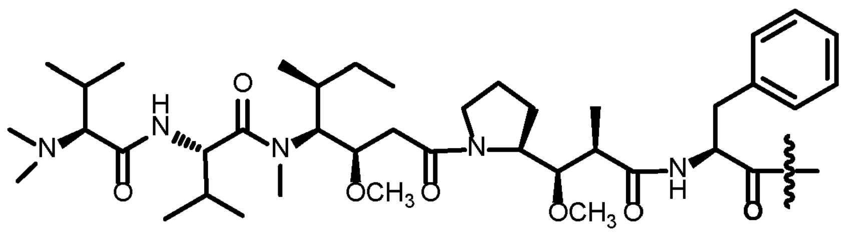

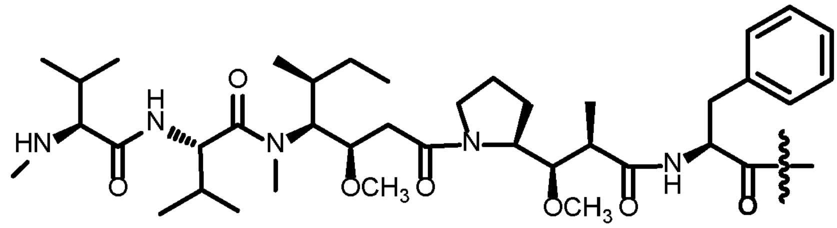

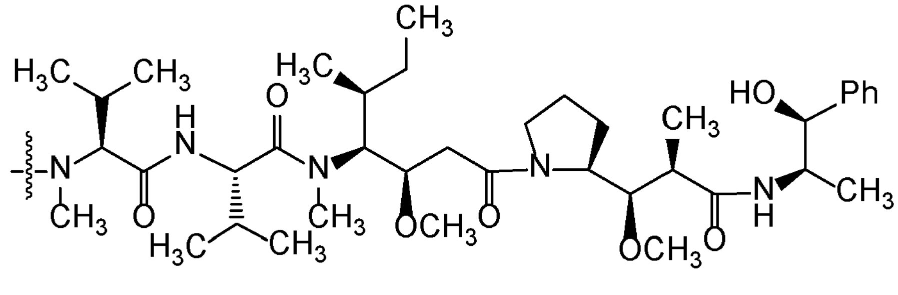

or

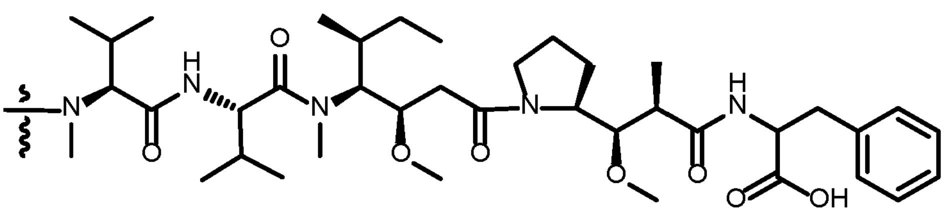

In some embodiments, the Drug is a benzodiazepine (including benzodiazepine containing drugs *e.g.*, pyrrolo[1,4]benzodiazepines (PBDs), indolinobenzodiazepines, and oxazolidinobenzodiazepines).

PBDs are of the general structure:

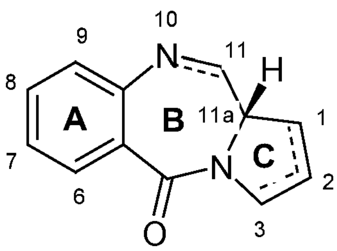

but can differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position, which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site. The ability of PBDs to form an adduct in the minor groove enables them to interfere with DNA processing, hence their use as antitumour agents. The biological activity of these molecules can be potentiated by, for example, joining two PBD units together through their C8/C'-hydroxyl functionalities via a flexible alkylene linker. The PBD dimers are thought to form sequence-selective DNA lesions such as the palindromic 5'-Pu-GATC-Py-3' interstrand cross-link which is thought to be mainly responsible for their biological activity.

There are a number of different assays that can be used for determining whether a Ligand-Drug Conjugate exerts a cytostatic or cytotoxic effect on a cell line. In one example for determining whether a Ligand-Drug Conjugate exerts a cytostatic or cytotoxic effect on a cell line, a thymidine incorporation assay is used. For example, cells at a density of 5,000 cells/well of a 96-well plated is cultured for a 72-hour period and exposed to 0.5 μCi of $^3$H-thymidine during the final 8 hours of the 72-hour period, and the incorporation of $^3$H-thymidine into cells of the culture is measured

in the presence and absence of Ligand-Drug Conjugate. The Ligand-Drug Conjugate has a cytostatic or cytotoxic effect on the cell line if the cells of the culture have reduced $^{3}H$-thymidine incorporation compared to cells of the same cell line cultured under the same conditions but not contacted with the Ligand-Drug Conjugate.

In another example, for determining whether a Ligand-Drug Conjugate exerts a cytostatic or cytotoxic effect on a cell line, cell viability is measured by determining in a cell the uptake of a dye such as neutral red, trypan blue, or ALAMAR™ blue (*see, e.g.,* Page et al., 1993, Intl. J. of Oncology 3:473-476). In such an assay, the cells are incubated in media containing the dye, the cells are washed, and the remaining dye, reflecting cellular uptake of the dye, is measured spectrophotometrically. The protein-binding dye sulforhodamine B (SRB) can also be used to measure cytoxicity (Skehan et al., 1990, J. Nat'l Cancer Inst. 82:1107-12). Preferred Ligand-Drug Conjugates include those with an $IC_{50}$ value (defined as the mAB concentration that gives 50% cell kill) of less than 1000 ng/ml, preferably less than 500 ng/ml, more preferably less than 100 ng/ml, even most preferably less than 50 or even less than 10 ng/ml on the cell line.

General procedures for linking a drug to linkers are known in the art. See, for example, U.S. Patent Nos. 8,163,888, 7,659,241, 7,498,298, U.S. Publication No. US20110256157 and International Application Nos. WO2011023883, and WO2005112919.

**$M^1$ - The succinimide**

A non-hydrolyzed succinimide (also referred to herein as a succinimide ring) conjugated to the Ligand unit via a thioether linkage can be represented as follows wherein R represents the remainder of the Linker unit optionally conjugated to a Drug unit, Detection unit or Stability unit:

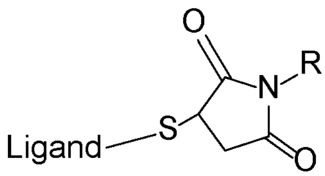

A hydrolyzed succinimide (also referred to herein as a hydrolyzed succinimide ring) conjugated to the Ligand unit via a thioether linkage can be represented as one of its two positional isomers as follows wherein R represents the remainder of the Linker unit optionally conjugated to a Drug unit, Detection unit or Stability unit:

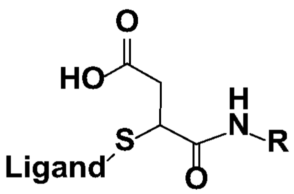 , 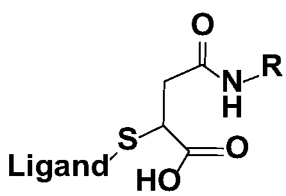

It will be understood for the non-hydrolyzed succinimides and hydrolyzed succinimide representations, there can be from 1 to 20, preferably 1 to 12, 1 to 10 or 1 to 8 self-stabilizing linkers conjugated to each Ligand. In some aspects, there are from 1 to 20, preferably 1 to 12, 1 to 10 or 1 to 8 drug-linkers conjugated to each Ligand. Additionally, for the conjugates described herein where a Ligand is not attached, the succinimide is in an unsaturated form as a maleimide (capable of reactive with a thiol or the Ligand).

**Basic units**

The Basic unit (BU) can be essentially a base capable of facilitating a hydroxide ion (or water) attack to hydrolyze a nearby succinimide group. Accordingly, the Basic unit of the present invention is selected from the group consisting of $-(CH_2)_xNH_2$, $-(CH_2)_xNHR^a$, and $-(CH_2)_xNR^a{}_2$, wherein x is an integer of from 0 to 4, 1 to 4, or from 1 to 3, or from 2 to 3, or from 2 to 4, but can also be 0, 1, 2, 3 or 4, and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the succinimide (hydrolyzed or non-hydrolyzed) or dilactam.

### The Optional Secondary Linker Assembly ($L^O$)

As noted above, the optional secondary linker assembly can be represented by the formula:

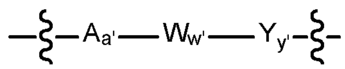

wherein -A- is an optional Stretcher unit, the subscript a' is 0 or 1; -W- is an optional Cleavable unit, the subscript w' is 0 or 1; and -Y- is an optional Spacer unit, and the subscript y' is 0 or 1. The wavy line adjacent to the optional Stretcher unit indicates the site of attachment to the self-stabilizing linker assembly and the wavy line adjacent to the optional Spacer unit indicates the site of attachment to the Drug unit.

General methods of linking a Drug unit, a Detection unit, or a Stability unit to a Ligand unit are known in the art and linkers known in the art can be adapted for use with a self-stabilizing linker assembly or modified to include a basic component and/or electron withdrawing group using the teachings described herein. For example, auristatin and maytansine ADCs are currently in clinical development for the treatment of cancer. Monomethyl auristatin E is conjugated through a protease cleavable peptide linker to an antibody, monomethyl auristatin F is conjugated directly to an antibody through maleimidocaproic acid, DM1 is conjugated through a disulfide or directly through the heterobifunctional SMCC linker, and DM4 is conjugated through a disulfide linker. These linker systems can be adapted for use with a self-stabilizing linker assembly or modified to include a basic component and/or electron withdrawing group using the teachings described herein and provide release of drug by a cleavable or non-cleavable system depending on the linker system used. Disulfide, thioether, peptide, hydrazine, ester, or carbamate bonds are all examples of bonds that can be used to connect a Drug Unit to a Linker Unit. Stretcher units, Cleavable units, and Spacer units are described in more detail below.

Stretcher units, Cleavable units, and Spacer units are described in more detail below.

### The Stretcher Unit

The Stretcher unit (-A-), when present, extends the framework of the Linker unit to provide more distance between the self-stabilizing linker assembly and the Drug unit. A Stretcher unit is capable of linking the self-stabilizing linker assembly to the Cleavable unit when the Cleavable unit is present, the self-stabilizing linker assembly to the Spacer unit when the Cleavable unit is absent but the Spacer unit is present and the self-stabilizing linker assembly to the Drug unit when both the Cleavable unit and the Spacer unit are absent. As described, a Stretcher unit is capable of attaching to more than one Cleavable unit, Spacer unit, and/or Drug unit.

The Stretcher unit can also act to alter the physiochemical properties of the Drug-Linker depending on components of the Stretcher unit. In some aspects, the Stretcher unit will be added in order to increase the solubility of the Drug-Linker and will comprise one or multiple solubility-enhancing groups such as ionic groups or water-soluble polymers. Water-soluble typically includes any segment or polymer that is soluble in water at room temperature and includes poly(ethylene)glycol groups as well as other polymers such as polyethyleneimines.

A Stretcher unit can comprise one or multiple stretcher groups. Exemplary stretcher groups include, for example, -NH-$C_1$-$C_{10}$ alkylene-, -NH-$C_1$-$C_{10}$ alkylene-NH-C(O)-$C_1$-$C_{10}$ alkylene-, -NH-$C_1$-$C_{10}$ alkylene-C(O)-NH-$C_1$-$C_{10}$ alkylene-, -NH-$(CH_2CH_2O)_s$-, -NH-$(CH_2CH_2O)_S$-$CH_2$-, -NH-$(CH_2CH_2NH)_s$-$(CH_2)_s$, -NH-$(CH_2CH_2NH)_s$-$(CH_2)_s$-NH-C(O)-$(CH_2)_s$, -NH-($C_3$-$C_8$ carbocyclo)-, -NH-(arylene-)-, and -NH-($C_3$-$C_8$ heterocyclo-)-, wherein each s is independently 1-10. A representative stretcher group having a carbonyl group for linkage to the remainder of the Linker unit or the Drug unit is as follow:

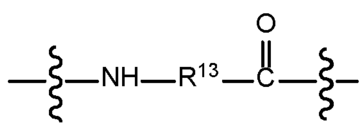

wherein $R^{13}$ is -$C_1$-$C_{10}$ alkylene-, -$C_3$-$C_8$carbocyclo-, -arylene-, -$C_1$-$C_{30}$heteroalkylene-, -$C_3$-Csheterocyclo-, -$C_1$-$C_{10}$alkylene-arylene-, -arylene-$C_1$-$C_{10}$alkylene-, -$C_1$-$C_{10}$alkylene-($C_3$-Cscarbocyclo)-, -($C_3$-$C_8$carbocyclo)-$C_1$-$C_{10}$alkylene-, -$C_1$-$C_{10}$alkylene-($C_3$-$C_8$ heterocyclo)-, -($C_3$-$C_8$ heterocyclo)-$C_1$-$C_{10}$ alkylene-, -$(CH_2CH_2O)_{1-10}(-CH_2)_{1-3}$-, or -$(CH_2CH_2NH)_{1-10}(-CH_2)_{1-3}$-. In some embodiments, $R^{13}$ is -$C_1$-$C_{10}$ alkylene- or -$C_1$-$C_{30}$heteroalkylene-. In some embodiments, $R^{13}$ is -$C_1$-$C_{10}$ alkylene-, -$(CH_2CH_2O)_{1-10}(-CH_2)_{1-3}$-, or -$(CH_2CH_2NH)_{1-10}(-CH_2)_{1-3}$-. In some embodiments, $R^{13}$ is -$C_1$-$C_{10}$ alkylene- polyethyleneglycol, or polyethyleneimine.

Non-cleavable drug release systems are known in the art and can be adapted for use with the self-stabilizing linker assemblies of the present invention as Stretcher units and/or Spacer units. A non-cleavable linker in capable of

linking a Drug unit to a Ligand in a generally stable and covalent manner and is substantially resistant to acid-induced cleavage, light-induced cleavage, peptidase- or esterase-induced cleavage, and disulfide bond cleavage. Drug is released from Ligand Drug Conjugates containing non-cleavable linkers via alternative mechanisms, such as proteolytic ligand degradation.

Cross-linking reagents that form non-cleavable linkers between maytansinoid drugs and ligands are well known in the art and can adapted for use herein. Exemplary cross-linking reagants that form non-cleavable linkers between the maytansinoid drugs and ligands comprise a maleimido or haloacetyl-based moiety. They include N-succinimidyl 4-(maleimidomethyl)cyclohexanecarboxylate (SMCC), N-succinimidyl-4-(N-maleimidomethyl)-cyclohexane-1-carboxy-(6-amidocaproate), which is a "long chain" analog of SMCC (LC-SMCC), κ-maleimidoundecanoic acid N-succinimidyl ester (KMUA), γ -maleimidobutyric acid N-succinimidyl ester (GMBS), ε-maleimidocaproic acid N-hydroxysuccinimide ester (EMCS), m-maleimidobenzoyl-N-hydroxysuccinimide ester(MBS), N-(.alpha.-maleimidoacetoxy)-succinimide ester [AMAS], succinimidyl-6-(β -maleimidopropionamido)hexanoate (SMPH), N-succinimidyl 4-(p-maleimidophenyl)-butyrate (SMPB), and N-(p-maleimidophenyl)isocyanate (PMPI), N-succinimidyl-4-(iodoacetyl)-aminobenzoate (SIAB), N-succinimidyl iodoacetate (SIA), N-succinimidyl bromoacetate (SBA) and N-succinimidyl 3-(bromoacetamido)propionate (SBAP). Additional Stretcher units for use in combination with the self-stabilizing linker assembly of the present invention can be found, for example, in U.S. Patent No. 8,142,784.

**The Cleavable Unit**

The Cleavable unit (-W-), when present, is capable of linking the self-stabilizing linker assembly to the Spacer unit when the Spacer unit is present or the self-stabilizing linker assembly to the Drug unit when the Spacer unit is absent. The linkage from the self-stabilizing linker assembly to the Spacer unit or to the Drug unit can be directly from the self-stabilizing linker assembly when the Stretcher unit is absent or via the Stretcher unit if the Stretcher unit is present.

In some embodiment, the Cleavable unit will be directly conjugated to the self-stabilizing linker assembly on one end and to the Drug unit on the other end. In other embodiments, the Cleavable unit will be directly conjugated to the Stretcher unit on one end and to the Drug unit on the other end. In yet other embodiments, the Cleavable unit will be directly conjugated to the Stretcher unit on one end and to the Spacer unit on the other end. In even yet other embodiments, the Cleavable unit will be directly conjugated to the self-stabilizing linker assembly on one end and to the Spacer unit on the other end. Any of specifically described self-stabilizing linker assemblies described herein can be used in these embodiments.

The Cleavable unit is capable of forming a cleavable bond with a Drug unit or a Spacer unit. Reactive groups for forming cleavable bonds can include, for example, sulfhydryl groups to form disulfide bonds, aldehyde, ketone, or hydrazine groups to form hydrazone bonds, carboxylic or amino groups to form peptide bonds, and carboxylic or hydroxy groups to form ester bonds.

The nature of the Cleavable unit can vary widely. For example, cleavable linkers include disulfide containing linkers that are cleavable through disulfide exchange, acid-labile linkers that are cleavable at acidic pH, and linkers that are cleavable by hydrolases, peptidases, esterases, and glucoronidases.

In some aspects, the structure and sequence of the Cleavable unit is such that the unit is cleaved by the action of enzymes present at the target site. In other aspects, the Cleavable unit can be cleavable by other mechanisms. The Cleavable unit can comprise one or multiple cleavage sites.

In some embodiments, the Cleavable unit will comprise one amino acid or one or more sequences of amino acids. The Cleavable unit can comprise, for example, a monopeptide, a dipeptide, tripeptide, tetrapeptide, pentapeptide, hexapeptide, heptapeptide, octapeptide, nonapeptide, decapeptide, undecapeptide or dodecapeptide unit.

Each amino acid can be natural or unnatural and/or a D- or L-isomer provided of course that there is a cleavable bond. In some embodiments, the Cleavable unit will comprise only natural amino acids. In some aspects, the Cleavable unit will comprise 1 to 12 amino acids in contiguous sequence.

In some embodiments, each amino acid is independently selected from the group consisting of alanine, arginine, aspartic acid, asparagine, histidine, glycine, glutamic acid, glutamine, phenylalanine, lysine, leucine, serine, tyrosine, threonine, isoleucine, proline, tryptophan, valine, cysteine, methionine, selenocysteine, ornithine, penicillamine, β-alanine, aminoalkanoic acid, aminoalkynoic acid, aminoalkanedioic acid, aminobenzoic acid, amino-heterocyclo-alkanoic acid, heterocyclo-carboxylic acid, citrulline, statine, diaminoalkanoic acid, and derivatives thereof. In some embodiments, each amino acid is independently selected from the group consisting of alanine, arginine, aspartic acid, asparagine, histidine, glycine, glutamic acid, glutamine, phenylalanine, lysine, leucine, serine, tyrosine, threonine, isoleucine, proline, tryptophan, valine, cysteine, methionine, and selenocysteine. In some embodiments, each amino acid is independently selected from the group consisting of alanine, arginine, aspartic acid, asparagine, histidine, glycine, glutamic acid, glutamine, phenylalanine, lysine, leucine, serine, tyrosine, threonine, isoleucine, proline, tryptophan, and valine. In some embodiments, each amino acid is selected from the proteinogenic or the non-proteinogenic amino acids.

In another embodiment, each amino acid is independently selected from the group consisting of the following

L-(natural) amino acids: alanine, arginine, aspartic acid, asparagine, histidine, glycine, glutamic acid, glutamine, phenylalanine, lysine, leucine, serine, tyrosine, threonine, isoleucine, tryptophan and valine.

In another embodiment, each amino acid is independently selected from the group consisting of the following D-isomers of these natural amino acids: alanine, arginine, aspartic acid, asparagine, histidine, glycine, glutamic acid, glutamine, phenylalanine, lysine, leucine, serine, tyrosine, threonine, isoleucine, tryptophan and valine.

In some embodiments, the bond between the Cleavable unit and the Drug unit can be enzymatically cleaved by one or more enzymes, including a tumor-associated protease, to liberate the Drug unit (-D), which in one embodiment is protonated *in vivo* upon release to provide a Drug (D).

Useful Cleavable units can be designed and optimized in their selectivity for enzymatic cleavage by a particular enzyme, for example, a tumor-associated protease. In one embodiment, a linkage (or bond) between the Cleavable unit and the Drug unit or Spacer unit is that which cleavage is catalyzed by cathepsin B, C and D, or a plasmin protease.

In certain embodiments, the Cleavable unit can comprise only natural amino acids. In other embodiments, the Cleavable unit can comprise only non-natural amino acids. In some embodiments, the Cleavable unit can comprise a natural amino acid linked to a non-natural amino acid. In some embodiments, the Cleavable unit can comprise a natural amino acid linked to a D-isomer of a natural amino acid.

An exemplary Cleavable unit is the dipeptide -Val-Cit-, -Phe-Lys- or -Val-Ala.

In some embodiments, the Cleavable unit will comprises a peptide and will comprise from 1 to 12 amino acids. In some such embodiments, the peptide will be conjugated directly to the Drug unit and the Spacer unit will be absent. In some such embodiments, the Stretcher unit and Spacer unit will be absent. In one aspect, the peptide will be a dipeptide.

In some embodiments, the Cleavable unit $-W_w-$ will be represented by $-(-AA-)_{1-12}-$, or $(-AA-AA-)_{1-6}$ wherein AA is at each occurrence independently selected from natural or non-natural amino acids. In one aspect, AA is at each occurrence independently selected from natural amino acids. One of skill in the art would appreciate that amino acids are typically linked to the Drug unit or Spacer unit through functional units present in the amino acid, e.g., its carboxylic acid or amino termini.

In other aspects, the Cleavable unit will comprise a glucoronide unit, preferably 1 or 2 glucoronide units. In some such embodiments, the Glucuronide unit comprises a sugar moiety (Su) linked via a glycoside bond (-O'-) to a self-immolative Spacer:

-[Su-O'-Y]-

The glycosidic bond (-O'-) is typically a β-glucuronidase-cleavage site, such as a bond cleavable by human, lysosomal β-glucuronidase.

In some aspects, -[Su-O'-Y]- is represented by the following formula:

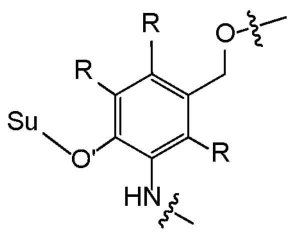

or

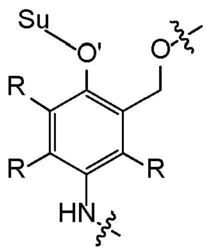

;

wherein Su is a Sugar moiety,_-O'- represents a glycosidic bond; each R is independently hydrogen, a halogen, -CN, or $-NO_2$; wherein the wavy bond adjacent to the nitrogen atom indicates covalent attachment to the Stretcher unit or to the Ligand and the wavy bond adjacent to the oxygen indicates covalent attachment to the Spacer unit or to the Drug unit. An exemplary Linker unit comprising a glucoronide prior to conjugation to an antibody and post conjugation is as follows wherein the wavy line indicates attachment to a Drug unit or Spacer unit and Ab represents an antibody and S is a sulfur atom of the antibody. It will be understood that more than one self-stabilizing assembly can be attached to each antibody:

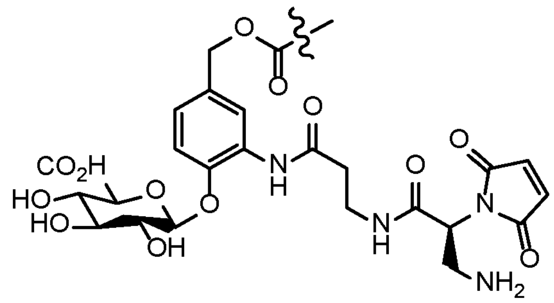

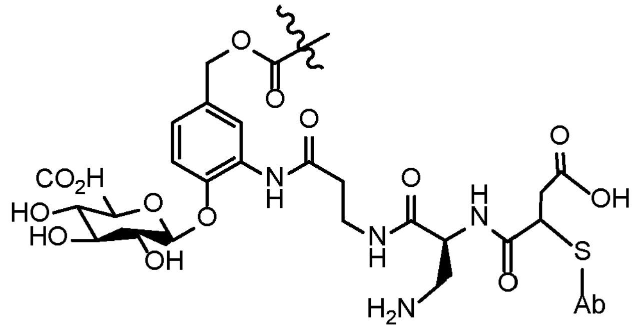

In some embodiments, the Cleavable unit itself will comprise a sulfur atom that is capable of forming a bond with a sulfur atom of a Spacer unit or Drug unit to form a disulfide or hindered disulfide. Cleavage occurs between the two sulfur atoms of the disulfide. In some such embodiments, one of the sulfur atoms is cleaved from the Drug unit and, provided there is no further release mechanism, the other sulfur atom remains attached to the Drug unit. A Linker unit comprising a Cleavable unit having a sulfur atom is capable of forming a bond with a sulfur atom of a Spacer unit or Drug unit to form a disulfide or hindered disulfide

Exemplary linkers include, for example, the following Drug-Linker wherein the wavy line indicates the site of attachment to the remainder of the Linker unit, D is a maytansinoid drug, and $R_a$ and $R_b$ are independently selected from H or methyl.

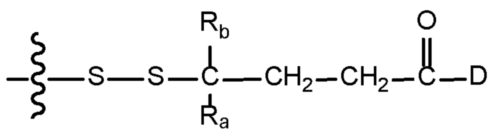

A variety of disulfide linkers are known in the art and can adapted for use in the present invention, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), SMPT (N-succinimidyl-oxycarbonyl-alpha-methyl-alpha-(2-pyridyl-dithio)toluene), and SPP (N-succinimidyl 4-(2-pyridyldithio)pentanoate). (*See, e.g.,* Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. *See also* U.S. Patent No. 4,880,935.)

In some embodiments, the cleavable linker is pH-sensitive and will comprise, for example, an acid-labile linker that is hydrolyzable in the lysosome (*e.g.*, a hydrazone, semicarbazone, thiosemicarbazone, cis-aconitic amide, orthoester, acetal, or ketal group) can be used. (*See, e.g.,* U.S. Patent Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome.

In some embodiments, the Cleavable unit will be conjugated directly to the Drug unit and the Spacer unit will be absent and the Cleavable unit will be linked to the Drug unit via a cleavable peptide, disulfide, or hydrazone bond.

**The Spacer Unit**

The Spacer unit (-Y-), when present, links a Cleavable unit to the Drug unit or a Stretcher unit to the Drug unit or a self-stabilizing linker assembly to a Drug unit. Like the Stretcher unit, the Spacer unit, when present can act to

extend the framework of the Linker unit. The Spacer unit can comprise multiple self-immolative or non-self immolative groups. In some embodiments, the Spacer unit comprises one or more self-immolative groups. In this context, the term "self-immolative group" refers to a bifunctional chemical moiety that is capable of covalently linking together two spaced chemical moieties into a normally stable tripartite molecule. It will spontaneously separate from the second chemical moiety if its bond to the first moiety is cleaved. In other embodiments, the Spacer unit is not self-immolative. In these embodiments, part or all of the Spacer unit remains attached to the Drug unit.

In some embodiments, -Y- is a self-immolative group and is linked to a Cleavable unit via the methylene carbon atom of the self-immolative group, and linked connected directly to the Drug unit via a carbonate, carbamate or ether group.

In some embodiments, -Yy- is a p-aminobenzyl alcohol (PAB) unit whose phenylene portion is optionally substituted with -$C_1$-$C_8$ alkyl, -O-($C_1$-$C_8$ alkyl), -halogen,- nitro or -cyano. In another embodiment, -Yy- can be a carbonate group. An unsubstituted PAB unit is as follows:

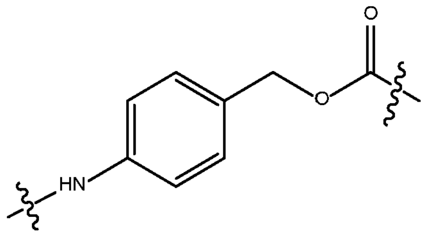

Other examples of self-immolative groups include, but are not limited to, aromatic compounds that are electronically similar to the PAB group such as 2-aminoimidazol-5-methanol derivatives (*see, e.g.,* Hay et al., 1999, Bioorg. Med. Chem. Lett. 9:2237) and ortho or para-aminobenzylacetals. Spacers can be used that undergo cyclization upon amide bond hydrolysis, such as substituted and unsubstituted 4-aminobutyric acid amides (*see, e.g.,* Rodrigues et al., 1995, Chemistry Biology 2:223), appropriately substituted bicyclo[2.2.1] and bicyclo[2.2.2] ring systems (*see, e.g.,* Storm et al., 1972, J. Amer. Chem. Soc. 94:5815) and 2-aminophenylpropionic acid amides (*see, e.g.,* Amsberry et al., 1990, J. Org. Chem. 55:5867). Elimination of amine-containing drugs that are substituted at the a-position of glycine (*see, e.g.,* Kingsbury et al., 1984, J. Med. Chem. 27:1447) are also examples of self-immolative groups.

Other suitable Spacer units are disclosed in Published U.S. Patent Application No. 2005-0238649.

Exemplary Stretcher units, Cleavable units, and Spacer units that can be used with the present compositions and methods are described in WO 2004010957, WO 2007/038658, WO 2005/112919, U.S. Patent No. 6,214,345, 7,659,241, 7,498,298, 7,968,687, 8,163,888, and U.S. Publication No. 2009-0111756, 2009-0018086, 2009-0274713.

In embodiments wherein the Conjugates are conjugated to a Stability unit or a Detection unit in lieu of a Drug unit, the optional Secondary Linker Assembly will typically be absent. In embodiments where the Secondary Linker Assembly is present, the Stretcher unit will generally be present but the Cleavable unit and the Spacer unit will be absent. The Stretcher unit will extend the framework of the Linker unit to provide more distance between the self-stabilizing assembly and the Detection unit or Stability unit. In such aspects, the Stretcher unit is capable of linking the self-stabilizing linker assembly to the Detection unit or the Stability unit.

## DRUG LOADING

The number of self- stabilizing linkers per Ligand is represented by p. In embodiments wherein the linkers are not branched, p represents the number of drug-linker molecules (or detection-linker or stability-linker molecules) per Ligand molecule (e.g., antibody). Depending on the context, p can represent the average number of self-stabilizing linkers per Ligand (or in embodiments where the linkers are not branched, the average number of drug-linker molecules (or detection-linker or stability-linker molecules) per Ligand (e.g., antibody)). The variable p ranges from 1 to 20, typically 1 to 12, 1 to 10 and is preferably from 1 to 8. In some preferred embodiments, when p represents the average number of self- stabilizing linkers per antibody, p ranges from about 2 to about 5. In some embodiments, p is about 2, about 4, or about 8. In some preferred embodiments, when p represents the average number of drug-linker molecules per antibody, p ranges from about 2 to about 5. In some embodiments, p is about 2, about 4, or about 8. The number of D' per self-stabilizing linkers is represented by u. u ranges from 1 to 10.

The average number of Drugs units per Ligand unit in a preparation from a conjugation reaction may be characterized by conventional means such as mass spectroscopy, ELISA assay, HIC and HPLC. The quantitative distribution of Drug-Linker-Ligand conjugates in terms of p may also be determined. In some instances, separation, purification, and characterization of homogeneous Ligand-Drug Conjugates, where p is a certain value from Ligand-Drug Conjugate with other drug loadings may be achieved by means such as reverse phase HPLC or electrophoresis.

**Self-Stabilizing Linker Assembly and Rates of Hydrolysis**

The Self-Stabilizing linker assembly links the Ligand unit to a Stretcher unit if the Stretcher unit is present, links the Ligand unit to a Cleavable unit if the Stretcher unit is absent and a Cleavable unit is present, links the Ligand unit to a Spacer unit if the Stretcher unit and the Cleavable unit are absent and the Spacer unit is present, or links the Ligand Unit to D' (e.g., a Drug Unit) if the Stretcher unit, Cleavable unit and Spacer unit are absent. In some embodiments, the Stretcher unit, Cleavable unit, and Spacer unit will be absent and the self-stabilizing linker assembly will be conjugated directly to D' (e.g., a Drug Unit). In other embodiments, one or more of the Stretcher unit, Cleavable unit, and Spacer unit will be present.

The rate at which the thio-substituted succinimide of the Self-Stabilizing Linker when part of a Ligand-Drug Conjugate undergoes hydrolysis can be quantified using the t1/2 of hydrolysis. t1/2 of hydrolysis refers to the time taken for half of the compound of interest to hydrolyze, *i.e.,* undergo a ring opening, under stated conditions (e.g., pH 7.4 and 22°C). In some embodiments of the present invention, the t1/2 of hydrolysis of the thio-substituted succinimide of the Self-Stabilizing Linker unit is less than 4 hours, preferably less than 3 hours, even more preferably, less than 2 hours, less than 1 hour, less than 45 minutes, less than 30 minutes, less than 15 minutes using the following assay and stated conditions.

The hydrolysis reaction rates of maleimido drug linkers following conjugation to antibody cysteines can be determined by mass spectrometry, as the hydrolyzed product has a molecular weight 18 daltons greater than the unhydrolyzed conjugate. Reduction of the interchain disulfides of a human IgG1 creates a single reduced cysteine on the light chain and three reduced cysteines on the heavy chain. The self-stabilizing maleimide drug-linker can then be conjugated to the reduced antibody at pH 7.4 and 22°C and introduced to a high-resolution electrospray mass spectrometer via a reversed-phase HPLC column which separates the conjugated light and heavy chains. The masses of the conjugated light and heavy chains can thus be measured, and the peak intensities determined by standard mass spectrometry data processing software (e.g., MassLynx). By performing a series of injections over time, the disappearance of the peak corresponding to the mass of the original, unhydrolyzed conjugate and the appearance of the peak corresponding to the mass of the hydrolyzed conjugate can be monitored, the intensities of the peaks determined, and the percentage of hydrolyzed conjugate calculated at each timepoint. By plotting the hydrolysis percentage versus time, a curve is generated (e.g., using PRISM) which can be fit to a standard equation for exponential phenomena which includes a parameter for t1/2.

In some aspects, the Self-Stabilizing Linker will be designed such that the maleimide component of the Self-Stabilizing Linker does not substantially undergo hydrolysis prior to conjugation to the Ligand unit.

In some embodiments of the present invention, the t1/2 of hydrolysis of the thio-substituted succinimide of the Self-Stabilizing Linker is from about 5 or about 10 minutes to about 24 hours, preferably from about 5 or about 10 minutes to about 12 hours, more preferably from about 5 or about 10 minutes to about 5 hours, more preferably from about 5 or about 10 minutes to about 2.5 hours, even more preferably from about 5 or about 10 minutes to about 1 hour, even more preferably from about 5 or about 10 minutes to about 30 minutes, even more preferably from about 5 or about 10 minutes to about 20 minutes, and even more preferably from about 10 minutes to about 15 minutes at a pH of about 7 to about 7.5 (e.g., 7.4) and a temperature of about 22°C.

In some such embodiments wherein the t1/2 of hydrolysis is as stated above, the hydrolysis goes to completion. Complete hydrolysis is considered to be achieved if 90% of the thio-substituted succinimide hydrolyzes. Preferably, 95% or greater, 96%, 97%, 98%, 99% or 100% hydrolysis will be achieved. In some embodiments, the hydrolysis reaction will compete with a dilactam formation and will not achieve completion. In some such embodiments, at least 90% of the reaction product will be a combination of either a hydrolyzed thio-substituted succinimide Ligand-Drug Conjugate or a thio-substituted dilactam Ligand-Drug Conjugate. Preferably at least 95% or greater, 96%, 97%, 98%, 99% or 100% of the reaction product will be a combination of either a hydrolyzed thio-substituted Ligand-Drug Conjugate or a thio-substituted dilactam Ligand-Drug Conjugate. The percentage of hydrolysis can be calculated from the mass spectrometric data of the conjugate at the final timepoint by determining the intensity of the peak corresponding to the mass of the original, unhydrolyzed conjugate and the intensity of the peak corresponding to the mass of the hydrolyzed conjugate, and using the sum of the peak intensities to determine the percentage hydrolyzed and percentage unhydrolyzed.

In addition to characterizing the Ligand-Drug Conjugate by its t1/2 of hydrolysis and/or the efficiency of the hydrolysis reaction, the stability of the Ligand-Drug Conjugate can be characterized by the ability of the Ligand-Drug Conjugate to undergo an elimination reaction and for the Drug-Linker to be transferred from the Ligand unit to an alternative reactive thiol present in the milieu of the Ligand-Drug Conjugate. In some embodiments, the Drug-Linker will exhibit no or substantially no disassociation from the Ligand under the following assay and stated conditions. The phrase "substantially no disassociation from the Ligand" is considered to be achieved if less than 40%, preferably less than 20%, even more preferably less than 10%, or even more preferably less than 5% or less than 2% of the Drug-Linker in a sample disassociates from the Ligand.

The elimination of a drug-linker containing an enzyme-cleavable linker from an antibody can be measured in

*ex vivo* plasma by the following method. The conjugate is placed in sterile plasma and incubated at 37°C. At the beginning of the incubation and at varying timepoints from 1 hour to 1 week or longer, an aliquot is removed at frozen at -80°C. Upon completion of the timepoints, the samples are passed over a protein A affinity resin to capture the antibody, the resin is washed with buffer, and then drug is released from the captured antibody by treatment with an appropriate enzyme (e.g. papain or proteinase K for peptide-based cleavable linkers). The released drug can then be quantified by standard LC-MS methodology, and the quantity of drug measured at each timepoint divided by the quantity of drug measured for the pre-incubation aliquot to determine the percentage of drug remaining conjugated to the antibody at each timepoint. The precision of this assay can be improved by including an internal standard antibody-drug conjugate which is prepared using an isotopically labeled version of the same drug-linker, such that the drug which is released from it can be detected independently in the LC-MS assay from the drug released from the test drug-linker by virtue of its mass difference. This isotopically labeled internal standard antibody-drug conjugate is added to each sample in equal amounts immediately prior to the protein A capture step. The quantitation of the drug released from the test ADC is then performed ratiometrically to the signal from the internal standard by conventional LC-MS techniques.

An alternative method for evaluating the elimination of a maleimide drug-linker from an antibody (or other ligand) is to incubate the conjugate in buffer (e.g., phosphate-buffered saline) at slightly elevated pH (e.g., pH 8.0) in the presence of a large excess of a small-molecule thiol (e.g., N-acetyl cysteine, NAC) which will react with any maleimide that eliminates from the parent conjugate. LC-MS assays can be performed to detect and quantify the drug-linker conjugated to NAC, or the parent ligand-conjugate. In the latter case, the ratio of the ligand-conjugate to unconjugated ligand can be measured and will remain constant over time if the ligand-conjugate is stable. Additional methods are provided in the examples section.

**Treatment of Cancer**

The Ligand-Drug Conjugates are for use in inhibiting the multiplication of a tumor cell or cancer cell, causing apoptosis in a tumor or cancer cell, or for use in treating cancer in a patient. The Ligand-Drug Conjugates can be used accordingly in a variety of settings for the treatment of cancers. The Ligand-Drug Conjugates can be used to deliver a drug to a tumor cell or cancer cell. Without being bound by theory, in one embodiment, the Ligand unit of a Ligand-Drug Conjugate binds to or associates with a cancer-cell or a tumor-cell-associated antigen, and the Ligand-Drug Conjugate can be taken up (internalized) inside a tumor cell or cancer cell through receptor-mediated endocytosis or other internalization mechanism. The antigen can be attached to a tumor cell or cancer cell or can be an extracellular matrix protein associated with the tumor cell or cancer cell. Once inside the cell, via a cleavable or non-cleavable mechanism, depending upon the components of the linker system, the drug is released within the cell. In an alternative embodiment, the Drug or Drug unit is cleaved from the Ligand-Drug Conjugate outside the tumor cell or cancer cell, and the Drug or Drug unit subsequently penetrates the cell.

The Ligand-Drug Conjugates can provide conjugation-specific tumor or cancer drug targeting, thus reducing general toxicity of the drug. In some embodiments, the Linker units stabilize the Ligand-Drug Conjugates in blood, yet are capable of liberating drug once inside the cell.

In one embodiment, the Ligand unit binds to the tumor cell or cancer cell.

In another embodiment, the Ligand unit binds to a tumor cell or cancer cell antigen which is on the surface of the tumor cell or cancer cell.

In another embodiment, the Ligand unit binds to a tumor cell or cancer cell antigen which is an extracellular matrix protein associated with the tumor cell or cancer cell.

The specificity of the Ligand unit for a particular tumor cell or cancer cell can be important for determining those tumors or cancers that are most effectively treated. For example, a ligand drug conjugate having a BR96 Ligand unit can be useful for treating antigen positive carcinomas including those of the lung, breast, colon, ovaries, and pancreas. Ligand-Drug Conjugates having an anti-CD30 or an anti-CD70 binding Ligand unit can be useful for treating hematologic malignancies.

Other particular types of cancers that can be treated with a ligand drug conjugates include, but are not limited to, those disclosed in Table 1:

Table 1

Solid tumors, including but not limited to:

(continued)

fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophogeal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, retinoblastoma

blood-borne cancers, including but not limited to:

acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", hairy cell leukemia, multiple myeloma

acute and chronic leukemias:

lymphoblastic, myelogenous, lymphocytic, myelocytic leukemias

Lymphomas:

Hodgkin's disease, non-Hodgkin's Lymphoma, Multiple myeloma, Waldenström's macroglobulinemia, Heavy chain disease, Polycythemia vera

**Multi-Modality Therapy for Cancer**

Cancers, including, but not limited to, a tumor, metastasis, or other disease or disorder characterized by uncontrolled cell growth, can be treated or inhibited by administration of a Ligand-Drug Conjugate.

In other embodiments, an effective amount of a Ligand-Drug Conjugate and a chemotherapeutic agent for use in methods for treating cancer are provided, including administering to a patient in need thereof an effective amount of a Ligand-Drug Conjugate and a chemotherapeutic agent. In one embodiment the chemotherapeutic agent is that with which treatment of the cancer has not been found to be refractory. In another embodiment, the chemotherapeutic agent is that with which the treatment of cancer has been found to be refractory. The Ligand-Drug Conjugates can be administered to a patient that has also undergone surgery as treatment for the cancer.

In some embodiments, the patient also receives an additional treatment, such as radiation therapy. In a specific embodiment, the Ligand-Drug Conjugate is administered concurrently with the chemotherapeutic agent or with radiation therapy. In another specific embodiment, the chemotherapeutic agent or radiation therapy is administered prior or subsequent to administration of a ligand drug conjugate.

A chemotherapeutic agent can be administered over a series of sessions. Any one or a combination of the chemotherapeutic agents, such a standard of care chemotherapeutic agent(s), can be administered.

Additionally, the Ligand-Drug Conjugate for use in methods of treatment of cancer are provided as an alternative to chemotherapy or radiation therapy where the chemotherapy or the radiation therapy has proven or can prove too toxic, e.g., results in unacceptable or unbearable side effects, for the subject being treated. The patient being treated can, optionally, be treated with another cancer treatment such as surgery, radiation therapy or chemotherapy, depending on which treatment is found to be acceptable or bearable.

**COMPOSITIONS AND METHODS OF ADMINISTRATION**

Pharmaceutical compositions comprising the Ligand-Drug Conjugates are described herein and a pharmaceutically acceptable carrier. The Ligand-Drug Conjugates can be in any form that allows for the compound to be administered to a patient for treatment of a disorder associated with expression of the antigen to which the Ligand unit binds. For example, the conjugates can be in the form of a liquid or solid. The preferred route of administration is parenteral. Parenteral administration includes subcutaneous injections, intravenous, intramuscular, intrasternal injection or infusion techniques. The compositions can be administered parenterally. The compounds can be administered intravenously.

Pharmaceutical compositions comprising the Ligand-Functional Agent Conjugates are described herein and a

pharmaceutically acceptable carrier. The Ligand-Drug Conjugates can be in any form that allows for the compound to be administered to a patient for treatment of a disorder or for diagnostic purposes

Pharmaceutical compositions can be formulated so as to allow a compound to be bioavailable upon administration of the composition to a patient. Compositions can take the form of one or more dosage units, where for example, a tablet can be a single dosage unit.

Materials used in preparing the pharmaceutical compositions can be non-toxic in the amounts used. It will be evident to those of ordinary skill in the art that the optimal dosage of the active ingredient(s) in the pharmaceutical composition will depend on a variety of factors. Relevant factors include, without limitation, the type of animal (e.g., human), the particular form of the compound, the manner of administration, and the composition employed.

The composition can be, for example, in the form of a liquid. The liquid can be useful for delivery by injection. In a composition for administration by injection, one or more of a surfactant, preservative, wetting agent, dispersing agent, suspending agent, buffer, stabilizer and isotonic agent can also be included.

The liquid compositions, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as amino acids, acetates, citrates or phosphates; detergents, such as nonionic surfactants, polyols; and agents for the adjustment of tonicity such as sodium chloride or dextrose. A parenteral composition can be enclosed in ampoule, a disposable syringe or a multiple-dose vial made of glass, plastic or other material. Physiological saline is an exemplary adjuvant. An injectable composition is preferably sterile.

The amount of the conjugate that is effective in the treatment of a particular disorder or condition will depend on the nature of the disorder or condition, and can be determined by standard clinical techniques. In addition, *in vitro* or *in vivo* assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The compositions comprise an effective amount of a compound such that a suitable dosage will be obtained. Typically, this amount is at least about 0.01% of a compound by weight of the composition.

For intravenous administration, the composition can comprise from about 0.01 to about 100 mg of a Ligand-Drug Conjugate per kg of the animal's body weight. In one aspect, the composition can include from about 1 to about 100 mg of a Ligand-Drug Conjugate per kg of the animal's body weight. In another aspect, the amount administered will be in the range from about 0.1 to about 25 mg/kg of body weight of a compound.

Generally, the dosage of a compound administered to a patient is typically about 0.01 mg/kg to about 100 mg/kg of the subject's body weight. In some embodiments, the dosage administered to a patient is between about 0.01 mg/kg to about 15 mg/kg of the subject's body weight. In some embodiments, the dosage administered to a patient is between about 0.1 mg/kg and about 15 mg/kg of the subject's body weight. In some embodiments, the dosage administered to a patient is between about 0.1 mg/kg and about 20 mg/kg of the subject's body weight. In some embodiments, the dosage administered is between about 0.1 mg/kg to about 5 mg/kg or about 0.1 mg/kg to about 10 mg/kg of the subject's body weight. In some embodiments, the dosage administered is between about 1 mg/kg to about 15 mg/kg of the subject's body weight. In some embodiments, the dosage administered is between about 1 mg/kg to about 10 mg/kg of the subject's body weight. In some embodiments, the dosage administered is between about 0.1 to 4 mg/kg, even more preferably 0.1 to 3.2 mg/kg, or even more preferably 0.1 to 2.7 mg/kg of the subject's body weight over a treatment cycle.

The Ligand-Functional Agent Conjugates e.g., Ligand-Drug Conjugates) can be administered by any convenient route, for example by infusion or bolus injection, by absorption through epithelial or mucocutaneous linings (e.g., oral mucosa, rectal and intestinal mucosa). Administration can be systemic or local. Various delivery systems are known, e.g., encapsulation in liposomes, microparticles, microcapsules, capsules, and can be used to administer a compound. In certain embodiments, more than one compounds or composition is administered to a patient.

The term "carrier" refers to a diluent, adjuvant or excipient, with which a compound is administered. Such pharmaceutical carriers can be liquids, such as water and oils, including those of petroleum, animal, vegetable or synthetic origin, such as peanut oil, soybean oil, mineral oil, sesame oil. The carriers can be saline, gum acacia, gelatin, starch paste, talc, keratin, colloidal silica, urea,. In addition, auxiliary, stabilizing, thickening, lubricating and coloring agents can be used. In one embodiment, when administered to a patient, the compound or compositions and pharmaceutically acceptable carriers are sterile. Water is an exemplary carrier when the compounds are administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol. The present compositions, if desired, can also contain minor amounts of

wetting or emulsifying agents, or pH buffering agents.

In an embodiment, the conjugates are formulated in accordance with routine procedures as a pharmaceutical composition adapted for intravenous administration to animals, particularly human beings. Typically, the carriers or vehicles for intravenous administration are sterile isotonic aqueous buffer solutions. Where necessary, the compositions can also include a solubilizing agent. Compositions for intravenous administration can optionally comprise a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where a conjugate is to be administered by infusion, it can be dispensed, for example, with an infusion bottle containing sterile pharmaceutical grade water or saline. Where the conjugate is administered by injection, an ampoule of sterile water for injection or saline can be provided so that the ingredients can be mixed prior to administration.

The pharmaceutical compositions are generally formulated as sterile, substantially isotonic and in full compliance with all Good Manufacturing Practice (GMP) regulations of the U.S. Food and Drug Administration.

Pharmaceutical compositions can comprise the Ligand Drug Conjugates of the present invention and a pharmaceutically acceptable carrier. In some preferred embodiments, all, or substantially all, or more than 50% of the Ligand Drug Conjugates present in the pharmaceutical composition comprises a hydrolyzed thio-substituted succinimide. In some preferred embodiments, more than 55%, 60%, 65%, 70%, 75%, 80%, 85%, 90%, 91%, 92%, 93%, 94%, 95%, 96%, 97 %, 98%, or 99% of the Ligand Drug Conjugates present in the pharmaceutical composition comprises a hydrolyzed thio-substituted succinimide.

**Methods for preparing Ligand-Drug Conjugates**

In another aspect, methods of preparing Ligand-Drug Conjugates or Ligand-Functional Agent Conjugates comprising a Self-Stabilizing Linker are described.

In some embodiments, methods comprise the steps of providing a Drug-Linker or Linker unit as described herein, conjugating said Drug-Linker or Linker unit to a sulfhydryl group of a Ligand unit to form a conjugate, allowing the resultant conjugate to undergo a hydrolysis reaction to form a Ligand-Drug conjugate comprising a thio-substituted hydrolyzed succinimide.

The rate of the thio-substitued succinimide hydrolysis can be manipulated by adjusting the reaction conditions following conjugation of the Drug-Linker to the Ligand, *e.g.*, by adjusting the pH or temperature. In some embodiments of the present invention, all, substantially all, or at least 50%, 60%, 70%, 80%, 85%, 90% or even 95% of the thio-substituted succinimide is hydrolyzed without manipulation of the reaction conditions, *i.e.*, the hydrolysis reaction occurs under the same reaction conditions as the conjugation reaction. In some embodiments, all, substantially all, or at least 50%, 60%, 70%, 80%, 85%, 90% or even 95% of the thio-substituted succinimide is hydrolyzed from 20 minutes, to 4 hours following conjugation, preferably from 20 minutes to 2 hours following conjugation. In exemplary embodiments, the conjugation conditions are pH of about 7.4 and a temperature of about 22° C.

Methods for preparing a Ligand-Drug Conjugate comprises the steps of providing a Drug-Linker or Linker unit comprising a Self-Stabilizing Linker; conjugating said Drug-Linker or Linker unit to a sulfhydryl group of a Ligand to form a Ligand-Drug Conjugate conjugate comprising a non-hydrolyzed thio-substituted succinimide; allowing the non-hydrolyzed thio-substituted succinimide to undergo a hydrolysis reaction, wherein all, substantially all, or at least 50%, 60%, 70%, 80% or even 85% of the succinimide is hydrolyzed from 10 minutes to 4 hours following conjugation. In some embodiments, all, substantially all, or at least 50%, 60%, 70%, 80%, 85%, 90% or even 95 % of the succinimide is hydrolyzed by 10 minutes, by 20 minutes, 40 minutes 60 minutes, 90 minutes or 120 minutes following conjugation. In some embodiments, the hydrolysis reaction occurs under the same reaction conditions as the conjugation reaction. In exemplary embodiments, the conjugation conditions are pH of about 7.4 and a temperature of about 22° C.

**Methods for Synthesizing Self-Stabilizing Linkers**

The present invention provides, *inter alia*, Self-Stabilizing Linkers. Methods of preparing Self-Stabilizing Linker units are encompassed within the scope of the present invention.

Maleimide compounds are typically prepared from corresponding amines by reaction of the primary amine with maleic anhydride followed by cyclodehydration of the maleamic acid. The overall scheme for the preparation of maleimde compounds is shown in the scheme below.

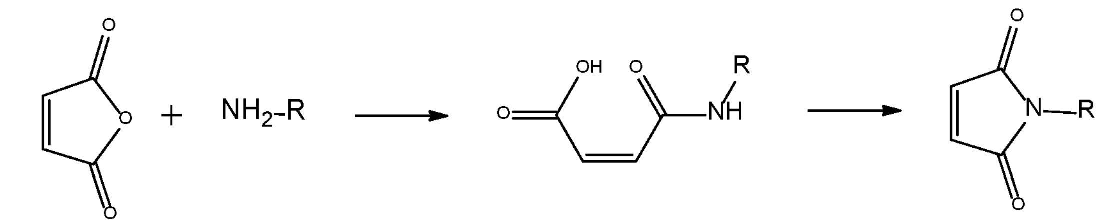

For preparation of maleimides containing basic groups in the side chain of the starting amine, such basic groups should be protected, if necessary. The appropriate protecting groups should be stable under conditions of maleimide preparation, yet should be removable later in the presence of maleimide. Suitable protecting groups consist, but are not limited to, acid labile protecting groups. "Boc" protecting group is one of the preferred protecting group.

The first step of maleimide preparation, the formation of the maleamic acid is very facile and can be usually accomplished in good yield by slow addition of the amine to a suspension containing a stoichiometric excess of the maleic anhydride.

The second step, cyclodehydration of the maleamic acid, can be accomplished in a number of ways known to skilled in the art. For example, the use of chemical dehydrating agents has been a well established method for accomplishing this step. Carbodiimides in combination with isomerizing alcohols, for example: DCC/HOBt, have been used to effect cyclodehydration of amic acids to maleimides.

Thermal cyclodehydration with use of azeotropic distillation in the presence of acid catalyst is another well known method to generate maleimides. The use of an azeotropic solvent permits the efficient removal of the water co-product as it forms, thereby driving reaction to maleimide. Suitable azeotropic solvents include cyclohexane, benzene, toluene, ethylbensene, mesitylene, and the like. Toluene is considered to be the most desirable since it boils at 110 °C at atmospheric pressure. Boiling temperatures below 200 °C are preferable to minimize possible thermal isomerization of maleamic acid to the more thermodynamically stable trans (fumaramic acid) structure.

The use of polar aprotic co-solvents can be beneficial for overall yield improvement as well as reducing time of cyclodehydration. Several polar aprotic solvents including dimethylformamide, dimethylacetamide, acetonitrile, N-methylpyrrolidone, dimethylsulfoxide, and sulfonate have been claimed to be useful. The most useful polar aprotic solvent is dimethylformamide.

Incorporation of certain amine salts instead of aprotic solvents can be further beneficial for maleimide formation according to Patent US 5,973,166.

One step microwave assisted maleimide synthesis has been also reported starting from maleic anhydride and an appropriate amine using no solvent (H. N. Borah, et al., J. Chem. Research (S), 1998, 272-272).

Example using water as a solvent for maleimide formation has been reported in ARKIVOC, 2001 (v) 60-67 by V. Ondrus, et al.

Alternatively, maleimide compounds can be generated from maleimide and appropriate alcohol using, for example, Mitsunobu reaction conditions as shown in the scheme below (M.A. Walker, Tetrahedron Letters, 1994, v. 35, n 5, pp. 665-668).

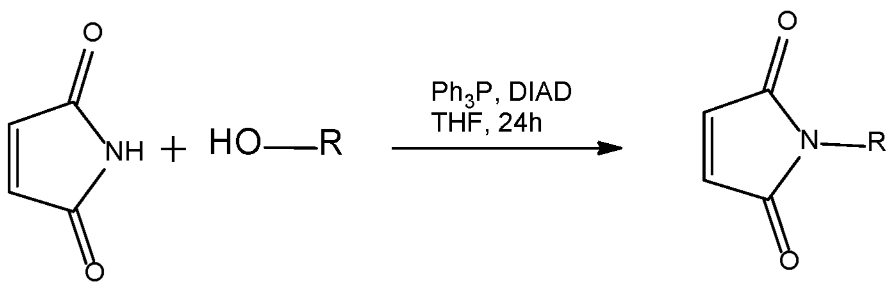


The self-stabilizing linker assembly of the present invention are linked to the Stretcher unit, Cleavable unit, Spacer unit, or Drug unit using the teachings described herein in combination with methods known in the art. The Linkers and Drug-Linkers are conjugated to Ligand units using teachings described herein in combination with methods known in the art. For example, for conjugation to interchain disulfides, an antibody can be treated with a reducing agent, such as dithiothreitol (DTT) to reduce some or all of the interchain disulfide cysteine residues to form highly nucleophilic cysteine thiol groups. The full reduced antibody or partially reduced antibody can be subsequently conjugated to the maleimide of the Linker Unit. In exemplary embodiments, conjugation conditions are gentle ones, pH of about 7 and a temperature of about 22 °C.

**Intermediates**

The present invention provides intermediates for use in making Self-Stabilizing Linkers. Intermediates include the following wherein T, c, $R^{11}$ and $R^{12}$ are as previously described.

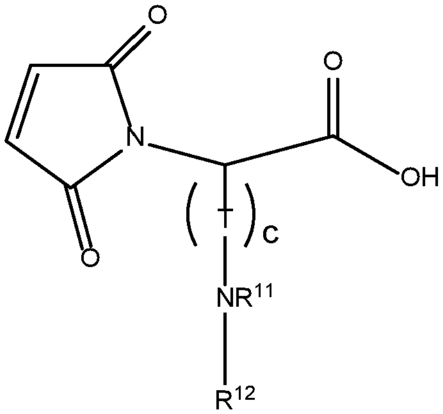

,

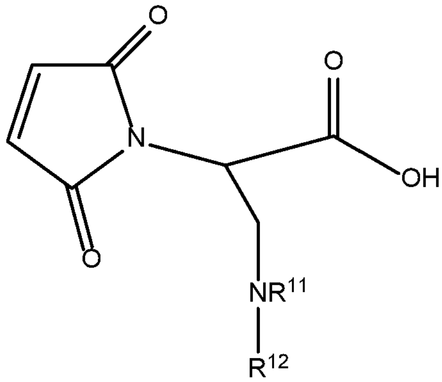

and

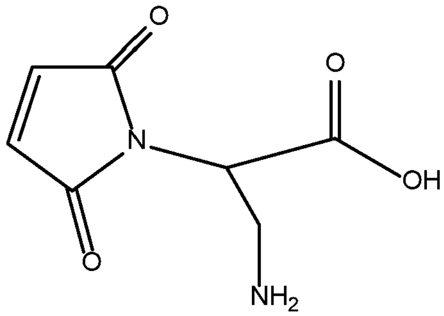

**Mono-thio-substituted or Di-thio-substituted Maleimide or Succinimide Self-Stabilizing Linkers**

In addition to designing self-stabilizing linkers for increasing the hydrolysis rates of mono thio-substituted succinimides, self-stabilizing linkers can also be used to increase the hydrolysis rate of mono-thio-substituted maleimides, di-thio-substituted maleimides, or di-thio-substituted succinimides.

In view of the above, a Ligand-Functional Agent Conjugate can comprise a Ligand unit and at least one Functional Agent selected from a Drug unit, a Detection unit, or a Stability unit, wherein the Ligand unit and each of the Functional Agent(s) are joined by a self-stabilizing linker assembly comprising a succinimide ring, a maleimide ring, a hydrolyzed succinimide ring or a hydrolyzed maleimide ring wherein the succinimide ring, maleimide ring, hydrolyzed succinimide ring or hydrolyzed maleimide ring is directly conjugated to the Ligand unit via one or two thioether linkages; and a base and an electron withdrawing group operably linked to stabilize the conjugate in plasma relative to a Ligand-Functional Agent Conjugate lacking the self-stabilizing linker assembly (i.e. by increasing the rate of succinimide or maleimide ring hydrolysis). In some aspects, the electron withdrawing group is positioned to increase the electrophilicity of the succinimide or maleimide rendering it more reactive with water and the base is positioned to assist the hydrolysis of the succinimide or maleimide ring (e.g., by an intramolecular base catalysis mechanism).

The maleimide ring can be conjugated to the Ligand unit via one or two thioether linkages as illustrated below both in non-hydrolyzed and hydrolyzed form and the succinimide ring can be conjugated to the Ligand unit via two thioether linkages as illustrated below in both non-hydrolyzed and hydrolyzed form wherein the wavy line indicates the point of attachment to the remainder of the linker conjugate or linker-functional agent conjugate:

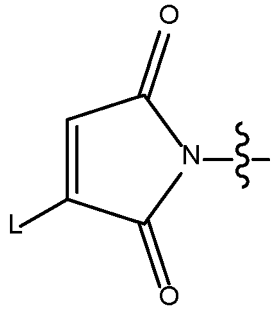

,

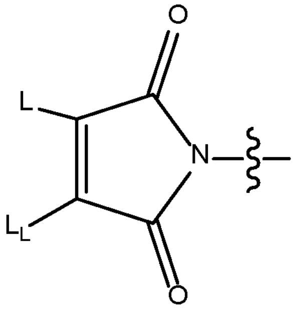

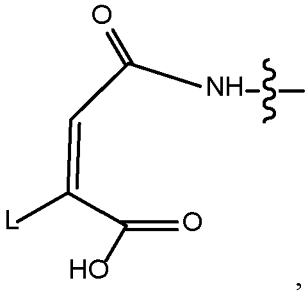

,

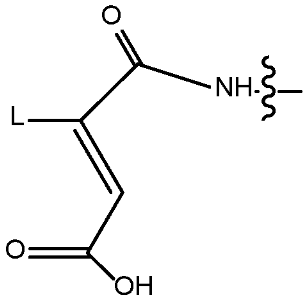

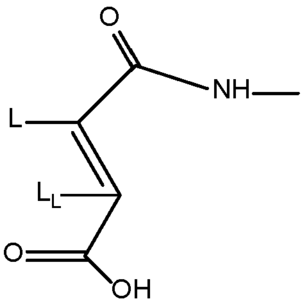

,

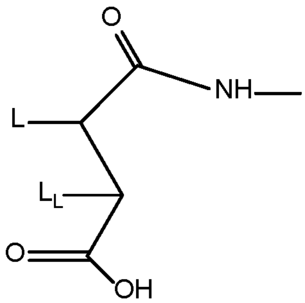

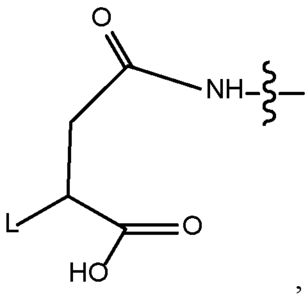

,

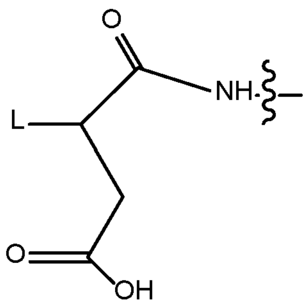

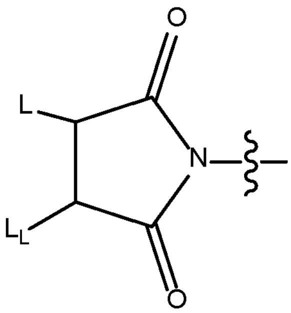

In embodiments wherein the maleimide ring, hydrolyzed maleimide, succinimide ring, or hydrolyzed succinimide is conjugated to the Ligand via two thioether linkages, p typically ranges from 1 to 10, or 1 to 8, or 1 to 4 and the maleimide or succinimide can be conjugated to the same or different polypeptide chains of the Ligand. In some aspects, the Ligand is an antibody. In other aspects, the Ligand is a non-antibody protein.

Methods of preparing mono- or di-thio-substituted halomaleimides as well as dithiosubstituted succinimides are known in the art as are methods of conjugating them to ligands, see for example, Ryan et al., Chem. Commun., 2011, 47, 5452-5454 and Smith et al., J. Am. Chem. Soc. 2010, 132(6), 1960-1965.

**EXAMPLES**

Example 1 - Synthesis of representative Self-Stabilizing Components

**Maleyl-Lysine(boc)OH**

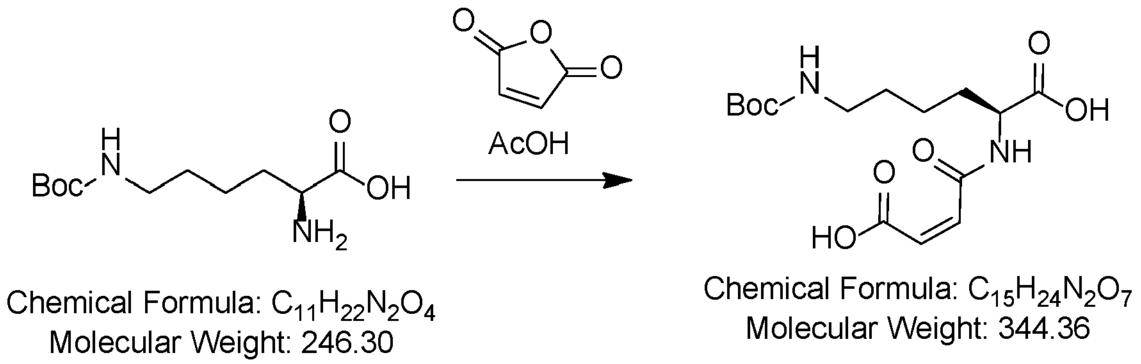


In a 50 ml round bottom flask H-Lys(boc)-OH (246mg, 1 mmol) and maleic anhydride (98 mg, 1 mmol) were dissolved in 1 ml (4 vol.) acetic acid and the solution was stirred at room temperature for 3 hours. The reaction mixture was concentrated to an oil on the rotovap, and the product was precipitated by adding ~ 10 ml dichloromethane . The precipitate was collected by vacuum filtration, washed with dichloromethane, and dried overnight in the vacuum oven. 270 mg of product was recovered as a white powder (85% yield)

**Maleoyl-Lysine(boc)-OH**

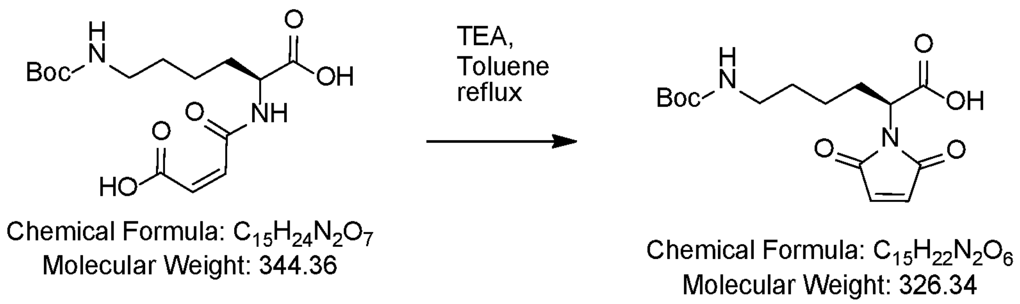


Maleyl-Lys(boc)-OH (100 mg, 0.29 mmol) was suspended in Toluene (3 ml) and triethylamine (224 uL) over molecular sieves in a 50 ml round bottom flask equipped with a condenser. DMA (-150 uL) was added to aid solubility. The solution was heated to 125 °C and refluxed for 4 hours after which the reaction was shown to be complete by LCMS. The reaction mixture was concentrated to dryness on the rotovap, redissolved in DMSO and purified by preparative HPLC. 56 mg of product was isolated as a white powder. (60% yield)

**Maleyl-DPR(boc)OH**

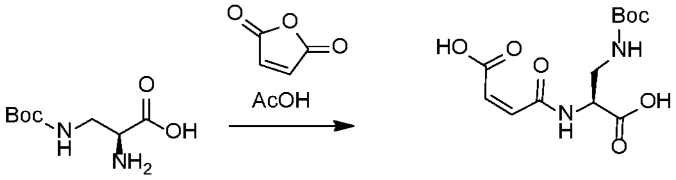


Chemical Formula: $C_8H_{16}N_2O_4$
Molecular Weight: 204.22

Chemical Formula: $C_{12}H_{18}N_2O_7$
Molecular Weight: 302.28

Maleyl-DPR(boc)-OH was prepared in the same manner as Maleyl-Lysine(boc)OH. (503 mg, 67%)

**Maleoyl-DPR(boc)-OH**

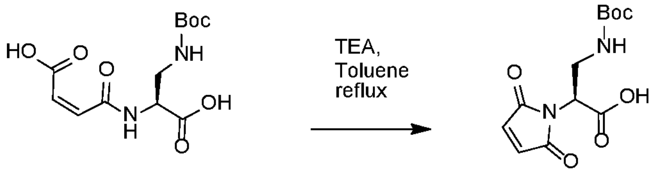


Chemical Formula: $C_{12}H_{18}N_2O_7$
Molecular Weight: 302.28

Chemical Formula: $C_{12}H_{16}N_2O_6$
Molecular Weight: 284.27

Maleoyl-DPR(boc)-OH was prepared in the same manner as Maleoyl-Lys(boc). (340 mg, 71%)

**Maleyl-Dimethyllysine**

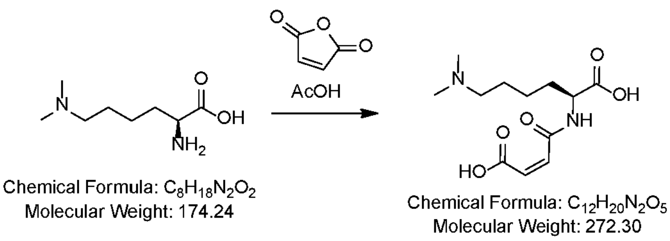


Chemical Formula: $C_8H_{18}N_2O_2$
Molecular Weight: 174.24

Chemical Formula: $C_{12}H_{20}N_2O_5$
Molecular Weight: 272.30

Maleyl-dimethyllysine was prepared in the same manner as Maleyl-lys(boc)-OH with the exception that the product did not precipitate after addition of dichloromethane. Instead the oil was co-evapaorated with 1:1 dichloromethane/hexane until a white foam was obtained and dried under high vacuum overnight. (109 mg, 99%)

**Maleoyl-dimethyllysine**

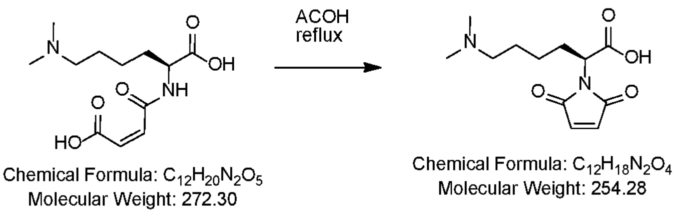


Chemical Formula: $C_{12}H_{20}N_2O_5$
Molecular Weight: 272.30

Chemical Formula: $C_{12}H_{18}N_2O_4$
Molecular Weight: 254.28

In a 10 ml round bottom flask ,Maleyl-dimethyllysine (100 mg) was dissolved in acetic acid (1 ml) and refluxed for 4 hours. After 4 hours the reaction mixture was concentrated to dryness on the rotovap and dried to a white foam under high vacuum. NMR of crude material shows -80% conversion based on ratio of the singlet at 6.9 ppm and the olefinic protons from the starting material.

Example 2 - Synthesis of mDPR-Val-Cit-PAB-MMAE

mDPR-Val-Cit-PAB-MMAE was prepared by coupling Boc-protected mDPR to Val-Cit-PAB-MMAE using standard methods for peptide coupling. The Boc group was removed in the final step.

Scheme:

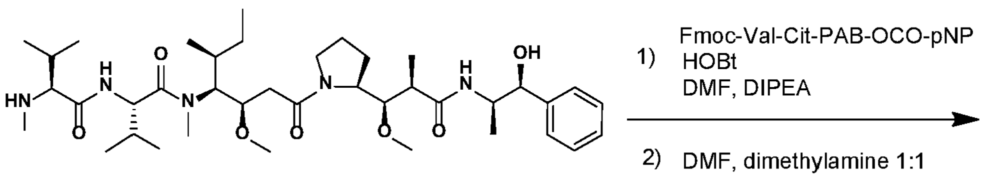


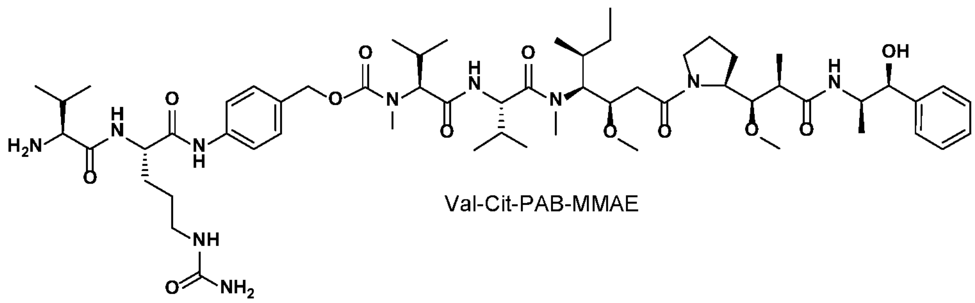

Val-Cit-PAB-MMAE

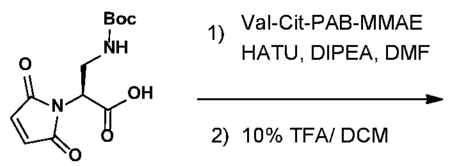


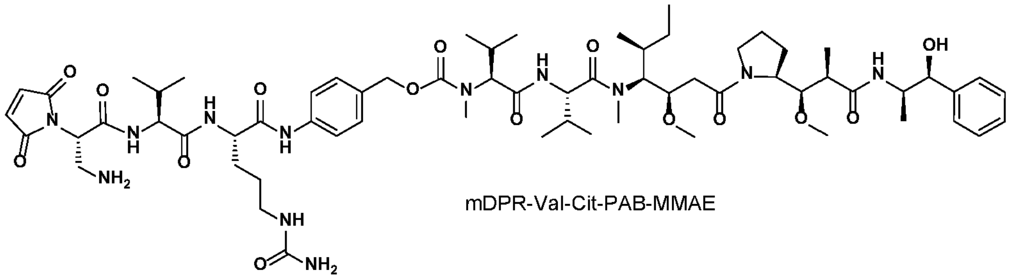

mDPR-Val-Cit-PAB-MMAE

*Preparation of Fmoc-Val-Cit-PAB-MMAE*

MMAE (5.34g, 6.94 mmol), Fmoc-Val-Cit-PAB-OCO-pNP (5.0g, 6.94 mmol) and HOBt (1.4 mmol) were charged to a 250 ml round bottom flask purged with N2 and dissolved in 15 ml of DMA. DIPEA (2.44 ml, 14 mmol) was then added, and the solution was stirred overnight at room temperature under inert atmosphere. The product was isolated by preparative HPLC, using a linear gradiant from 30% MeCN (0.05%TFA) to 100% MeCN (0.05% TFA) over 40 min. Fractions containing product were concentrated on the rotovap to a white powder, affording 3.2 g (34%)

*Preparation of Val-Cit-PAB-MMAE*

A solution of 3.2 g of Fmoc-Val-Cit-PAB-MMAE in 7 ml DMF and 7 ml diethylamine was stirred for 3 hours at room temperature. The reaction mixture was then concentrated to a thick oil on the rotovap. The product was precipitated in diethyl ether (100 ml) and filtered affording 2.0 g of product as an off white powder which was used without further purification.

*Preparation of mDPR (boc)Val-Cit-PAB-MMAE*

In a 50 ml round bottom flask mDPR(boc)-OH (25 mg, 0.089 mmol), Val-Cit-PAB-MMAE (100 mg, 0.089 mmol), and HATU (41 mg, 0.107 mmol) were dissolved in 2 ml DMF. DIPEA (34 uL) was added and the solution was stirred for 1 hr at rt. The reaction mixture was diluted with 1 ml DMSO and the product was isolated by preparative HPLC. (70 mg, 56%)

*Preparation of mDPR -Val-Cit-PAB-MMAE*

The above material was dissolved in 2 ml 10% TFA/dichloromethane and stirred for 1 h at rt. The reaction mixture was concentrated to dryness, reconstituted in 1 ml DMSO, and purified by preparative HPLC. (56 mg, 86%)

Example 3 - Monitoring thiosuccinimide hydrolysis

Thiosuccinimide hydrolysis of a self-stabilizing bioconjugate can be monitored by electrospray mass spectrometry, since the addition of water to the conjugate results in an increase of 18 Daltons to the observable molecular weight of the conjugate. When a conjugate is prepared by fully reducing the interchain disulfides of a human IgG1 antibody and conjugating the maleimide to the resulting cysteines, each light chain of the antibody will contain a single maleimide modification and each heavy chain will contain three maleimide modifications (see Figure 1, top). Upon complete hydrolysis of the resulting thiosuccinimides, the mass of the light chain will therefore increase by 18 Daltons, while the mass of the heavy chain will increase by 54 Daltons. This is illustrated in Figure 1 (bottom), with the conjugation and subsequent hydrolysis of a self-stabilizing maleimide drug-linker of the present invention (mDPR-Val-Cit-PAB-MMAE, molecular weight 1289 Da) to the fully reduced anti-CD30 antibody cAC10. The presence of the single N-linked glycosylation site on the heavy chain results in the heterogeneity of masses observed in the unconjugated antibody.

Example 4 - Monitoring 11/2 hydrolysis

By monitoring the intensities of the non-hydrolyzed and hydrolyzed peaks in the mass spectrum of a self-stabilizing bioconjugate over time (mDPR-Val-Cit-PAB-MMAE), the hydrolysis kinetics can be evaluated. This is done by plotting the percent of the total population which has hydrolyzed at each timepoint versus time (Figure 2, top). These data are then fit to the exponential equation

$$Y = Y_{max} \times \left(1 - e^{(-Kt)}\right)$$

where Y is the observed percent hydrolysis at time t, Ymax is the asymptotic maximal % hydrolysis, and K is the hydrolysis rate constant. The half-life for the hydrolysis reaction is defined as

$$t_{1/2} = {}^{\ln(2)}\!/_{K}$$

When this procedure is performed on the light chain of a reduced hIgG1 antibody, the analysis is quite straightforward as there is only one conjugation site per light chain and the reaction is a simple progression from the unhydrolyzed

species to the hydrolyzed species with a mass change of 18 Daltons. Performing this analysis on the heavy chain is complicated by the fact that there are a total of three conjugation sites, resulting in a series of peaks of +18, +36, and +54 Daltons as the conjugate undergoes hydrolysis. The analysis of the heavy chain is further complicated by the presence of multiple glycoforms. The analysis presented in Figure 2 was performed by only evaluating the peaks arising from the most abundant glycoform (the transition from 54195 Da to 54250 Da) and assuming that these peaks are a reasonable surrogate for the whole population of heavy chain glycoforms. As is evident in Figure 2, the observed kinetic profiles for light and heavy chains are very similar. For this reason, and because of the added complexities of quantifying hydrolysis rates on the heavy chain noted above, most of the data to characterize hydrolysis rates of self- stabilizing maleimides conjugated to antibodies was determined from evaluation of the light chain hydrolysis.

One limitation of this methodology is that the electrospray ionization process tends to produce a small proportion of sodium adducts in the observed peaks (approximately 10% under the conditions used to generate the data in Figure 2), which have an observed mass 22 Daltons greater than the parent mass. Many mass spectrometers do not have sufficiently high resolution to resolve this +22 mass from the +18 mass that results from hydrolysis on a protein with a total molecular mass in excess of 25,000 Daltons. Consequently, at the early timepoints when the degree of hydrolysis is low, the appearance of a peak at approximately +20 Daltons is a combination of these two effects which cannot be easily separated experimentally. As a result, the estimate of the percent of hydrolyzed product at the earliest timepoints is probably an overestimate, but the magnitude of this effect diminishes as the reaction proceeds toward completion

Example 5 - Evaluating spacing between the maleimide and basic group of the self-stabilizing linker assembly

It was hypothesized that the presence of a basic amino group adjacent to maleimide would accelerate the hydrolysis of thiosuccinimides prepared with those maleimides and thus result in stable bioconjugates. The distance between the maleimide and the basic amino group was recognized as an important parameter in the design of such self-stabilizing units. To evaluate the role of this spacing, a series of maleimides were prepared with the general structure

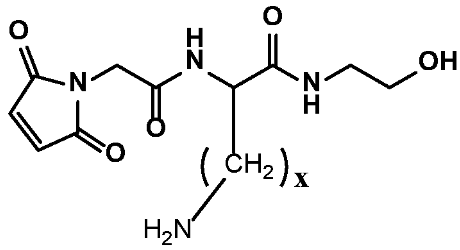

where x varied from 1 to 4. These maleimides were then conjugated to a fully reduced human IgG1 at pH8 and 37 °C and immediately monitored by electrospray mass spectrometry to determine the rate of hydrolysis. The distance between the basic group and the maleimide is inversely proportional to the hydrolysis rate--that is, the greater the distance, the slower the hydrolysis. This result illustrates that positioning a basic amino group close to a maleimide results in an increase in the rate of succinimide ring hydrolysis of bioconjugates prepared with the maleimide. However, even with the shortest spacing tested here (x=1), an antibody conjugate would have to be held at pH 8 and 37 °C for approximately 5 hours to achieve complete hydrolysis (about 5 half-lives). Exposure of an antibody or other protein to such conditions for extended periods can potentially result in covalent modifications and misfolding events, and so maleimides with even faster hydrolysis rates were sought.

To prepare bioconjugates with faster hydrolysis rates, a series of maleimides were prepared with the general structure

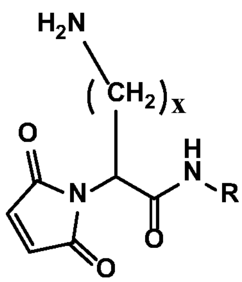

where x = 1 to 4 and R = val-cit-PAB-MMAE. These maleimides were then conjugated to a fully reduced human IgG1 at pH8 and 37 °C and immediately monitored by electrospray mass spectrometry to determine the rate of hydrolysis.

As shown in Figure 4 (top), the distance between the basic group and the maleimide within this series of structurally related compounds exerts a profound influence on the progress of the hydrolysis reaction. As in the prior example, the shorter the distance between the maleimide and the basic amine, the faster the hydrolysis. Since basic conditions (i.e. high pH) are known to increase the rate of maleimide and succinimide ring hydrolysis, this effect is presumably an example of intramolecular catalysis by a general base mechanism. Within this series the compounds with x=2 and x=3 did not attain complete hydrolysis during the 3 hour incubation, instead reaching an asymptote at approximately 80% and 50%, respectively (plots are normalized to the maximally achieved hydrolysis). This phenomenon may arise from a competing reaction such as direct nucleophilic attack of the primary amine on the succinimide ring, or may be due to an isomeric impurity in the maleimide which leads to biphasic hydrolysis kinetics.

Example 6 - Hydrolysis kinetics

The previous examples illustrate the influence that a basic group can have over the rate of succinimide ring hydrolysis in a bioconjugate, depending on the distance between the basic group and the maleimide of the parent molecule. However, it is expected that the presence of electron-withdrawing or -donating groups will also influence the rate of ring hydrolysis, since these groups will influence the electron density (and therefore electrophilicity) at the carbonyl carbons of the ring. In the conjugates of example 5, a carboxamide group is present in the alpha position relative to the nitrogen of the ring (i.e. a single carbon atom is present between the nitrogen of the ring and the carbonyl carbon of the carboxamide). Because the carboxamide is a weak electron withdrawing group, its presence is likely to influence the observed hydrolysis rates. To better understand the relative contributions of the basic amino group and the electron-withdrawing carboxamide group on the observed hydrolysis rates, a series of maleimides were conjugated to a reduced human IgG1 antibody at pH 7.4, 22 °C, and the hydrolysis rates determined by mass spectrometry (Figure 5). These maleimides contained just the carboxamide in the alpha position (triangles), just the primary amine in the beta position (inverted triangles), or both the carboxamide and the primary amine (circles). A control maleimide which contained neither group in proximity to the maleimide was also evaluated, although its hydrolysis is so slow that no reaction was observed under these conditions and no data is plotted. Under these conditions the self-stabilizing maleimide which contains both the base and the electron withdrawing group produced a bioconjugate with a hydrolysis $t_{1/2}$ of just 12 minutes, while the maleimide containing only the amine yielded a $t_{1/2}$ of 2.5 hours, and the maleimide containing only the carboxamide yielded a $t_{1/2}$ of 24 hours. This result indicates that the basic group and the electron withdrawing group act in concert to yield a conjugate with the very rapid hydrolysis kinetics which are most convenient for the manufacture of bioconjugates under the desirable mild conditions. Conjugates prepared with the diaminopropionyl maleimide (circles) exhibit ideal hydrolysis characteristics, with a $t_{1/2}$ of less than 15 minutes under very gentle conditions and the reaction approaching 100% completion in about 2 hours.

Example 7 - Evaluating spacing between the maleimide and carboxamide group of the self-stabilizing linker assembly

The rapid and complete succinimide hydrolysis observed in conjugates prepared with self-stabilizing diaminopropionyl maleimido drug-linkers acid (DPR) shown in examples 5 and 6 above indicates the importance of both the basic group and the electron withdrawing group to the design. A second, isomeric maleimido drug-linker was prepared with diaminopropionic acid to further evaluate the role of these two components on the hydrolysis behavior of the resulting conjugates. The structures are termed α-maleimido DPR and β-maleimido DPR and are shown below.

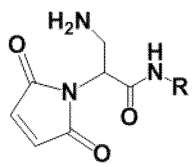

α-maleimido DPR

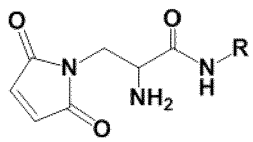

β-maleimido DPR

R = val-cit-PAB-MMAE

Both DPR maleimides possess a basic primary amine which is separated from the maleimido nitrogen by two carbon atoms. Both also possess an electron withdrawing carboxamide group, however the distance from the maleimido nitrogen the carboxamide varies from 1 to 2 carbon units (α and β, respectively). Finally, the separation between the basic amine and the carboxamide also varies from 1 to 2 carbon units (β and α, respectively). Collectively, this means that in β-DPR the carboxamide exerts less electron withdrawing influence on the maleimide ring but more electron

withdrawing influence on the primary amine, relative to α-DPR. This is expected to slow the rate of hydrolysis by reducing both the electrophilicity of the maleimide and the basicity of the primary amine. When these maleimido drug-linkers were conjugated to reduced antibody and monitored for succinimide hydrolysis, a 17-fold lower hydrolysis rate was observed for β-DPR relative to α-DPR (Figure 6). This example illustrates how the relative positioning of a basic group and an electron withdrawing group can be used to 'tune' the hydrolysis rate.

Examples 8-15

To evaluate the stability and pharmacological activity of ADCs prepared with self-stabilizing drug-linkers, a self-stabilizing maleimido-drug-linker was prepared. This drug-linker contains the maleimido-DPR group coupled to the cytotoxic agent MMAE via a protease-cleavable val-cit PAB self-immolative group (referred to herein as maleimido-DPR-val-cit-PAB-MMAE). For comparison, a non self-stabilizing drug-linker was used (referred to herein as maleimido-caproyl-val-cit-PAB-MMAE). The only difference between these agents is the unit between the maleimide and the valine group of the val-cit linker. The maleimide units of these drug-linkers can be prepared using maleic anhydride and mono-protected diaminopropionic acid and aminocaproic acid, respectively.

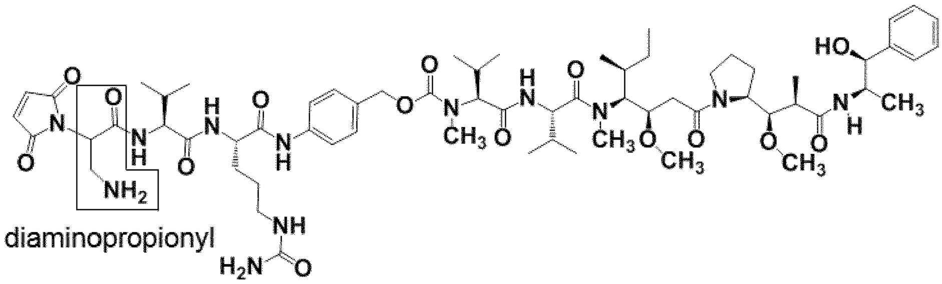

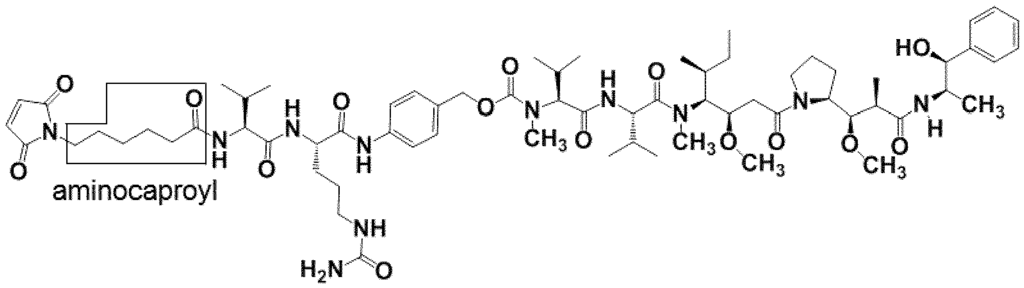

Example 8 - Evaluating stability of Ligand-Drug Conjugates in Buffer

In standard buffer systems, maleimide elimination from a bioconjugate prepared using thiol-maleimide chemistry is essentially undetectable because the eliminated maleimide quickly reacts again with the thiol, resulting in an equilibrium which lies far toward the side of the intact conjugate. However, the addition of a thiol scavenger to the buffer creates a system in which maleimide that eliminates from the bioconjugate can instead react with the scavenger, resulting in a persistent, observable loss of the maleimide from the protein. An experiment using such a system was performed with antibody-drug conjugates prepared with a self-stabilizing diaminoproprionyl (DPR) maleimido drug-linker alongside a non-stabilizing caproyl drug-linker. ADCs were prepared with 8 drugs per antibody of either maleimido-DPR-val-cit-PAB-MMAE or maleimido-caproyl-val-cit-PAB-MMAE using a fully reduced humanized IgG1. Drug loading was confirmed by reversed-phase HPLC on a polymeric PLRP-S column as described previously (Sun 2005). Complete succinimide hydrolysis of the self-stabilizing linker was also confirmed by electrospray mass spectrometry. These ADCs were placed in 150 mM Tris buffer, pH 8, at 2.5 mg/mL, containing 10 mM N-acetylcysteine as a scavenger, and incubated for 2 weeks at 37 °C. At seven timepoints during the incubation, an aliquot of each ADC was removed and frozen at -80°C. Upon completion of the time-course, all samples were analyzed by the above reversed-phase HPLC method to determine the drug : antibody ratio. The results of this study are shown in Figure 7. The ADC prepared with the self-stabilizing DPR maleimido drug-linker exhibited minimal loss of drug over this timecourse (from 8.0 to 7.9 drugs per antibody over 14 days), while the ADC prepared with the caproyl maleimido drug-linker lost approximately half of its drug load (from 8.0 to 3.9 drugs per antibody over 14 days) under these conditions.

Example 9 - Ex Vivo Plasma Stability (reversed phase method)

Assessing drug loading of humanized ADCs in non-human plasma samples by the reversed-phase HPLC method described in Example 8 can be achieved by first isolating the ADCs with IgSelect resin (GE Healthcare), which selectively binds to the human Fc domain. ADCs were prepared with 8 drugs per antibody of either maleimido-DPR-val-cit-PAB-MMAE or maleimido-caproyl-val-cit-PAB-MMAE using a fully reduced human IgG1. These ADCs (0.25 mg/mL) were incubated in sterile rat plasma for 7 days at 37 °C. At seven timepoints during the incubation, a 50 μL aliquot of each ADC was removed and frozen at -80 °C. Upon completion of the timecourse, ADCs were purified from each sample and analyzed by reversed-phase HPLC to determine the drug : antibody ratio. The results of this study are plotted in Figure 8. As was observed in buffer, incubation of an ADC prepared with a self-stabilizing maleimide in rat plasma also results in little or no observable loss of drug under conditions which result in the loss of approximately half of the drug from a maleimido-caproyl ADC.

Example 10 - Ex Vivo Plasma Stability (conjugated drug method)

A second assay format was utilized to assess ADC stability in rat and human plasma *ex vivo.* ADCs were prepared with 4 drugs per antibody of either maleimido-DPR-val-cit-PAB-MMAE or maleimido-caproyl-val-cit-PAB-MMAE using a human IgG1 partially reduced to a level of 4 thiols per antibody (resulting in an ADC with 4 drugs per antibody). These two ADCs were spiked into rat and human plasma and incubated at 37 °C for 7 days. At seven timepoints during this incubation, aliquots were removed and frozen at -80 °C until completion of the timecourse. ADCs were then isolated from each sample and MMAE released proteolytically from the isolated ADCs as described previously (Sanderson 2005). The released MMAE was then quantified by LC-MS/MS and normalized to the initial value for each ADC (Figure 9). In both rat and human plasma, the ADC prepared with a self-stabilizing maleimide lost little or no drug under these conditions, while approximately half of the drug was lost from a maleimido-caproyl ADC.

Example 11 - *In Vivo* Stability

As described in Example 10 above, the drug: antibody ratio can be measured for ADCs in rat plasma by reversed-phase HPLC analysis following purification with IgSelect resin. This method was applied to samples derived from an *in vivo* pharmacokinetic experiment in rats. ADCs were prepared with 4 drugs per antibody of either maleimido-DPR-val-cit-PAB-MMAE or maleimido-caproyl-val-cit-PAB-MMAE using a humanized IgG1 partially reduced to an average of 4 thiols per antibody (resulting in drug : antibody ratio of 4). These ADCs were further purified as described previously (Sanderson 2005) by hydrophobic interaction chromatography to isolate the species containing 4 drugs per antibody. These ADCs were dosed intravenously at 10 mg/kg in Sprague-Dawley rats. At five timepoints, three animals from each dosing group were sacrificed and the collected blood was processed to plasma and frozen at -80 °C. Upon completion of the study, all samples were processed by the IgSelect resin method described above, except that the sample volume varied. The drug : antibody ratio at each timepoint in this study are plotted in Figure 10. As was observed in rat plasma *ex vivo*, the ADC prepared with a self-stabilizing maleimide exhibits minimal loss of drug *in vivo,* dropping from an initial value of 4.1 drugs per antibody to a value of 3.6 drugs per antibody (12% reduction) after 7 days. During this same timeframe, the drug : antibody ratio of an ADC prepared with a maleimido-caproyl linker dropped from an initial value of 3.9 to a value of 1.5 (61% reduction). This illustrates that the increased stability of a self-stabilizing drug-linker that is observed *ex vivo* translates into an *in vivo* setting.

Example 12 - Pharmacokinetics

Because maleimido-caproyl ADCs are prone to loss of drug through maleimide elimination whereas self-stabilizing maleimide ADCs are not, it is reasonable to predict that exposure to antibody-conjugated drug will be greater following equivalent doses of the two ADCs. To confirm this prediction, ADCs were prepared with 4 drugs per antibody of either maleimido-DPR-val-cit-PAB-MMAE or maleimido-caproyl-val-cit-PAB-MMAE using a human IgG1 partially reduced to an average of 4 thiols per antibody (resulting in drug : antibody ratio of 4). These two ADCs were dosed at 2 mg/kg in Sprague-Dawley rats, and blood samples were taken at seven timepoints and processed to plasma. These plasma samples, along with standards of each ADC for the preparation of a calibration curve, were subjected to the mAb Select resin capture and papain release procedure described example 10 above to measure the concentration of antibody-conjugated MMAE. Antibody-conjugated drug concentrations were higher for the ADC prepared with the self-stabilizing drug-linker, with the magnitude of the difference increasing with time (data not shown). Initial antibody-conjugated drug concentrations are superimposable, reflecting the equivalence of the dose and drug : antibody ratio of the ADCs. However, divergence is observed within the first day, reaching a two-fold difference by day 3. These higher concentrations resulted in an approximately 40% greater antibody-conjugated drug AUC for the self-stabilized ADC

relative to the maleimido-caproyl ADC

Example 13 - Toxicology

To assess the impact of self-stabilizing maleimides on toxicology, ADCs were prepared with 4 drugs per antibody of either maleimido-DPR-val-cit-PAB-MMAE or maleimido-caproyl-val-cit-PAB-MMAE using a humanized IgG1 (which has no known binding to any rat antigen) partially reduced to an average of 4 thiols per antibody (resulting in an average drug : antibody ratio of 4). These ADCs were dosed intravenously in female CD®IGS rats (Charles River Laboratories) at 10 mg/kg (6 rats per test article plus 6 rats receiving vehicle only). Prior to dosing and at 3 post-dose timepoints, blood samples were taken for hematology and serum chemistry analysis for biomarkers of toxicity. Neutropenia induced by the MMAE ADC appeared less severe for the self-stabilized conjugate than for the maleimido-caproyl ADC (data not shown).

Example 14 - Plasma concentration of released drug

The toxicology experiment described in example 13 above also included blood draws at one hour and 24 hours post-dose, which along with the 4 day and 7 day post-dose samples were analyzed for unconjugated MMAE in plasma by LC-MS/MS. The results of this analysis indicated that peak concentrations of circulating MMAE are about 2-fold lower for the self-stabilizing maleimido-DPR-val-cit-PAB-MMAE ADC relative to the maleimido-caproyl-val-cit-PAB-MMAE (data not shown).

Example 15. Xenograft activity

To evaluate the impact of self-stabilizing drug-linkers on the antitumor activity of ADCs, conjugates were prepared with the anti-CD30 antibody cAC10 using drug-linkers containing the val-cit-PAB-MMAE cytotoxic payload linked to the antibody via either a maleimido-caproyl group or a self-stabilizing maleimido-DPR group. These ADCs were evaluated in two separate murine xenograft models of CD30+ human malignancies. In the first model (Figure 11), Karpas-299 (human ALCL) cells were implanted subcutaneously in female SCID mice and tumors were allowed to grow a volume of approximately 250 $mm^3$ before dosing at 1 mg/kg weekly for three doses (six mice per dose group). All six mice dosed with the maleimido-caproyl ADC experienced some tumor growth delay relative to the untreated group, and two animals experienced partial tumor shrinkage; however, all tumors grew out and the entire group was euthanized with large tumors. The self-stabilizing ADC dose group experienced complete responses (no detectable tumor) in all six animals, with five animals experiencing durable regressions for the course of the study and only one animal sacrificed after its tumor had returned on study day 55. The results of this study indicate a significantly greater in vivo antitumor activity for the ADC prepared with the self-stabilizing drug-linker. In the second model, L428 (human Hodgkin Lymphoma) cells were implanted subcutaneously in female NSG mice and tumors were allowed to grow a volume of approximately 100 $mm^3$ before dosing at 1 mg/kg every four days for four doses (six mice per dose group). All animals in both ADC dose groups experienced significant growth delay during treatment, however all tumors began growing out after study day 28 with no significant difference between maleimido-caproyl and self-stabilizing ADCs. Thus, the improvement in antitumor activity observed with the self-stabilizing ADC in xenograft studies appears to be model-dependent.

## Claims

1. A Ligand-Drug Conjugate having the formula:

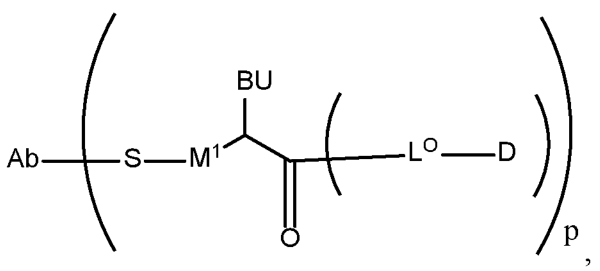

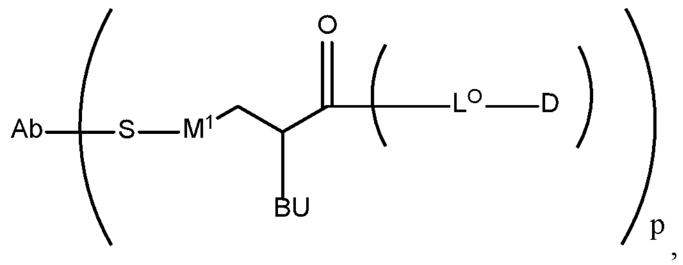

or

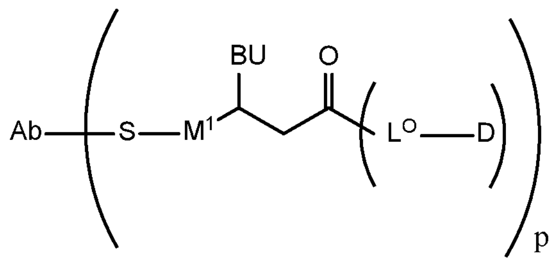

or a salt thereof, wherein D is a Drug unit, and Ab is an antibody; and wherein:

S is a sulfur atom of the antibody;
$M^1$ is a succinimide ring or hydrolyzed succinimide or together with BU forms a dilactam resulting from the reaction of the base of BU with the succinimide ring;
BU is a Basic Unit selected from the group consisting of $-(CH_2)_xNH_2$, $-(CH_2)_xNHR^a$, and $-(CH_2)_xNR^a{}_2$, wherein x is an integer of from 0-4 and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the succinimide (hydrolyzed or non-hydrolyzed) or dilactam;
the Drug unit is a cytotoxic agent;
the subscript p ranges from 1 to 20; and
$L^O$ is an optional secondary linker assembly having the formula:

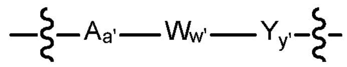

wherein -A- is an optional Stretcher unit, the subscript a' is 0 or 1; -W- is an optional Cleavable unit, the subscript w' is 0 or 1; and -Y- is an optional Spacer unit, and the subscript y' is 0 or 1; and the wavy lines indicate the points of attachment to the self-stabilizing linker assembly and the Drug unit, optionally wherein:

(i) w' is 1 and W is conjugated directly to the Drug unit,
(ii) w' is 1 and W is conjugated directly to the Spacer unit,
(iii) w' is 1 and W is conjugated directly to the Drug unit or Spacer unit via a cleavable peptide, disulfide or hydrazine bond,
(iv) W is absent, or
(v) W is present and the Spacer unit and Stretcher unit are absent.

**2.** A Ligand-Drug Conjugate according to claim 1, which is of formula:

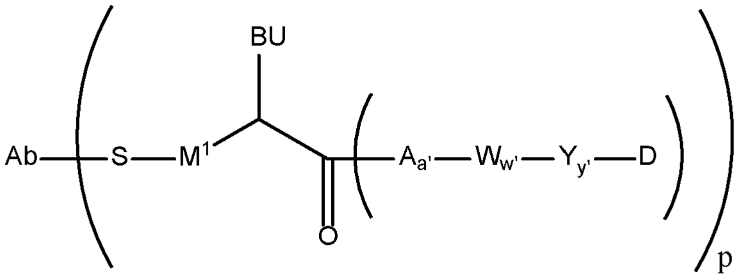

or a pharmaceutically acceptable salt thereof, wherein
subscript p ranges from 1 to 12;
BU is $-CH_2NH_2$, $-CH_2CH_2NH_2$, $-CH_2CH_2CH_2 NH_2$, or $-CH_2CH_2CH_2CH_2 NH_2$; and $M^1$ is a succinimide ring or a hydrolyzed succinimide ring and D is selected from the group consisting of auristatins and pyrrolo[1,4]benzodiazepines (PBDs).

**3.** A Ligand-Drug Conjugate according to claim 1, having the structure:

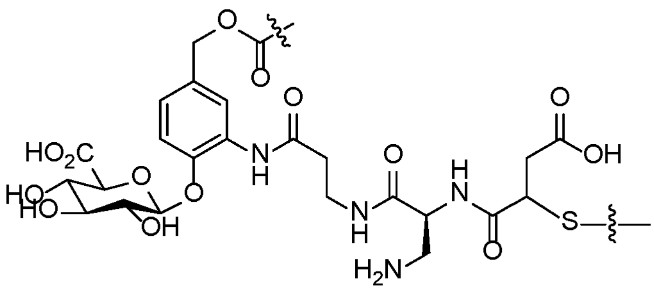

wherein the wavy line adjacent to the carbonyl indicates covalent attachment to the Drug unit (D), optionally via a Spacer unit (Y), and S is a sulfur atom of the antibody (Ab) for covalent attachment of the Linker Unit.

**4.** A Drug-Linker Conjugate having the formula:

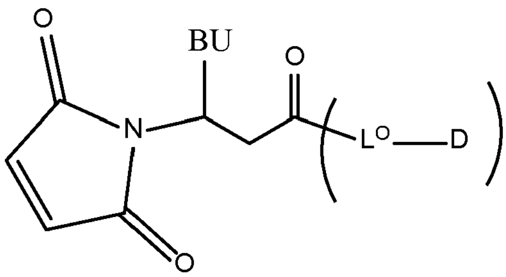

,

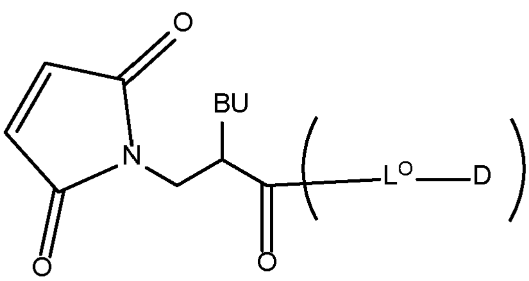

,

or

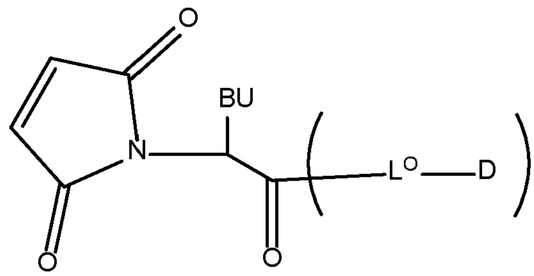

or a salt thereof, wherein

D is a Drug unit;
BU is a Basic Unit selected from the group consisting of $-(CH_2)_xNH_2$, $-(CH_2)_xNHR^a$, and $-(CH_2)_xNR^a{}_2$, wherein x is an integer of from 0-4 and each $R^a$ is independently selected from the group consisting of $C_{1-6}$ alkyl and $C_{1-6}$ haloalkyl, or two $R^a$ groups are combined with the nitrogen to which they are attached to form an azetidinyl, pyrrolidinyl or piperidinyl group provided that there are no less than 2 intervening atoms between the base of the Basic unit and the nitrogen atom of the maleimide;
the Drug unit is a cytotoxic agent; and
$L^O$ is an optional secondary linker assembly having the formula:

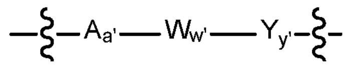

wherein -A- is an optional Stretcher unit, the subscript a' is 0 or 1; -W- is an optional Cleavable unit, the subscript w' is 0 or 1; and -Y- is an optional Spacer unit, and the subscript y' is 0 or 1; and the wavy lines indicate the points of attachment to the self-stabilizing linker assembly and the Drug unit, optionally wherein:

(i) w' is 1 and W is conjugated directly to the Drug unit,
(ii) w' is 1 and W is conjugated directly to the Spacer unit,
(iii) w' is 1 and W is conjugated directly to the Drug unit or Spacer unit via a cleavable peptide, disulfide or hydrazine bond,
(iv) W is absent, or
(v) W is present and the Spacer unit and Stretcher unit are absent.

**5.** A Drug-Linker Conjugate according to claim 4 having the formula:

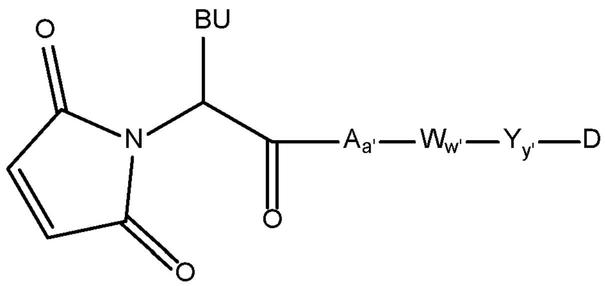

or salt thereof, wherein
BU is $-CH_2NH_2$, $-CH_2CH_2NH_2$, $-CH_2CH_2CH_2$ $NH_2$, or $-CH_2CH_2CH_2CH_2$ $NH_2$; and D is selected from the group consisting of auristatins and pyrrolo[1,4]benzodiazepines (PBDs).

**6.** A Ligand-Drug Conjugate according to any one of claims 1 to 3 or a Drug-Linker Conjugate according to claim 4, wherein D is selected from the group consisting of:

(a) antitubulin agents, auristatins, DNA minor groove binders, DNA replication inhibitors, alkylating agents, anthracyclines, antibiotics, antifolates, antimetabolites, chemotherapy sensitizers, duocarmycins, etoposides, fluorinated pyrimidines, ionophores, lexitropsins, nitrosoureas, platinols, pore-forming compounds, purine antimetabolites, puromycins, radiation sensitizers, steroids, taxanes, topoisomerase inhibitors, and vinca alkaloids;
(b) auristatins, camptothecins, duocarmycins, etoposides, maytansines and maytansinoids, taxanes, benzodiazepines, and vinca alkaloids; or
(c) auristatins and pyrrolo[1,4]benzodiazepines (PBDs).

**7.** A Ligand-Drug Conjugate according to any one of claims 1 to 3 or a Drug-Linker Conjugate according to any one of claims 4 to 6, wherein D is selected from the group consisting of auristatins having the structures of:

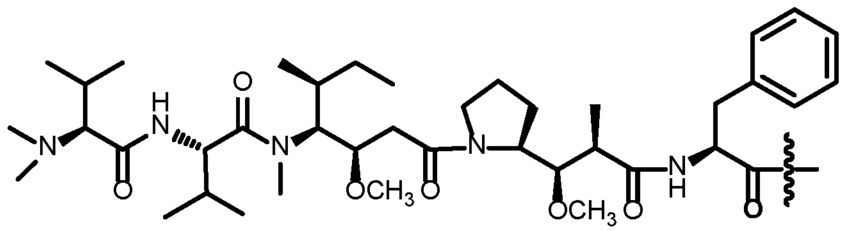

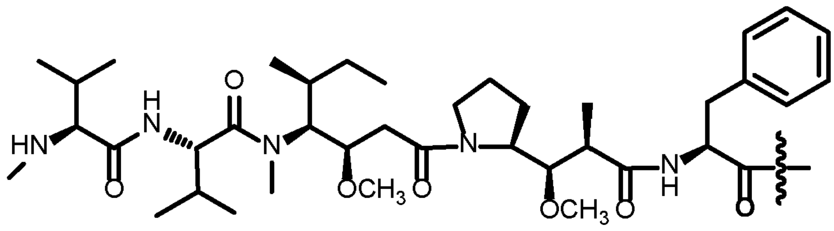

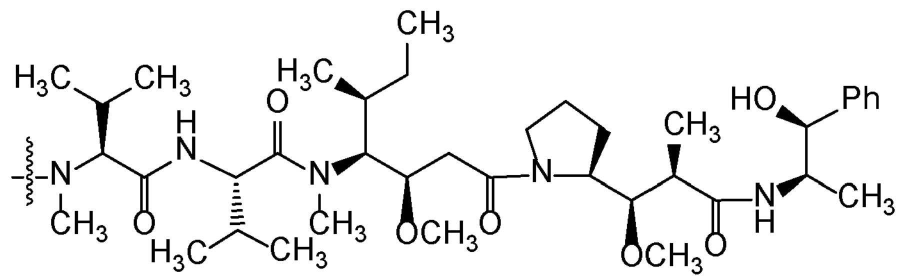

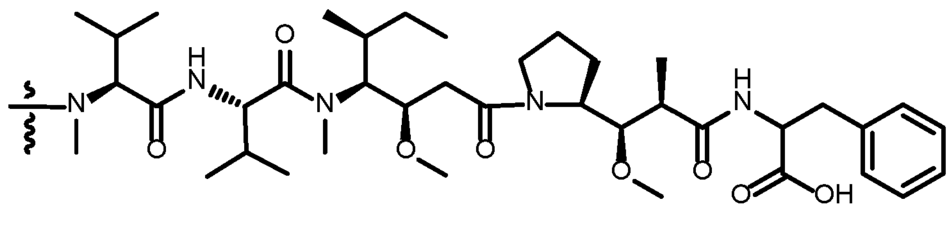

and pharmaceutically acceptable salt thereof wherein the wavy line indicates the site of attachment to the Linker unit.

**8.** A Ligand-Drug Conjugate according to any one of claims 1 to 2 or claim 7, or a Drug-Linker Conjugate according to any one of claims 4 to 7, wherein the Stretcher Unit (A) is selected from the group consisting of -NH-$C_1$-$C_{10}$ alkylene-, -NH-$C_1$-$C_{10}$ alkylene-NH-C(O)-$C_1$-$C_{10}$ alkylene-, -NH-$C_1$-$C_{10}$ alkylene-C(O)-NH-$C_1$-$C_{10}$ alkylene-, -NH-$(CH_2CH_2O)_s$-, -NH-$(CH_2CH_2O)_s$-$CH_2$-, -NH-$(CH_2CH_2NH)_s$-$(CH_2)_s$, -NH-$(CH_2CH_2NH)_s$-$(CH_2)_s$-NH-C(O)-$(CH_2)_s$, - NH-($C_3$-$C_8$ carbocyclo)-, -NH-(arylene)-, and -NH-($C_3$-$C_8$ heterocyclo)-, wherein each s is independently 1-10, or wherein the Stretcher Unit (A) has the formula of:

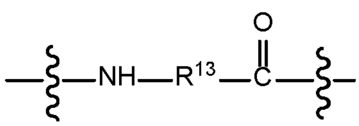

wherein $R^{13}$ is -$C_1$-$C_{10}$ alkylene-, -$C_3$-$C_8$carbocyclo-, -arylene-, -$C_1$-$C_{30}$heteroalkylene-, - $C_3$-$C_8$heterocyclo-, -$C_1$-$C_{10}$alkylene-arylene-, -arylene-$C_1$-$C_{10}$alkylene-, -$C_1$-$C_{10}$alkylene-($C_3$-Cscarbocyclo)-, -($C_3$-$C_8$carbocyclo)-$C_1$-$C_{10}$alkylene-, -$C_1$-$C_{10}$alkylene-($C_3$-$C_8$ heterocyclo)-, -($C_3$-$C_8$ heterocyclo)-$C_1$-$C_{10}$ alkylene-, -$(CH_2CH_2O)_{1-10}(-CH_2)_{1-3}$-, or -$(CH_2CH_2NH)_{1-10}(-CH_2)_{1-3}$-, preferably -$C_1$-$C_{10}$ alkylene- or -$C_1$-$C_{30}$heteroalkylene-, more preferably $R^{13}$ is -$C_1$-$C_{10}$ alkylene-, - $(CH_2CH_2O)_{1-10}(-CH_2)_{1-3}$-, or -$(CH_2CH_2NH)_{1-10}(-CH_2)_{1-3}$-.

**9.** A Ligand-Drug Conjugate according to any one of claims 1 to 2 or claims 7 to 8, or a Drug-Linker Conjugate according to any one of claims 4 to 8, wherein -$W_{w'}$- is represented by - $(AA)_{1-12}$-, or -$(AA\text{-}AA)_{1-6}$- wherein AA is at each occurrence independently selected from natural or non-natural amino acids, preferably independently selected from natural amino acids, and the bond between the Cleavable unit (W) and the Drug Unit (D) or Spacer unit (Y) is enzymatically cleavable by a tumor-associated protease, preferably by cathepsin B, C and D, or a plasmin protease.

**10.** A Ligand-Drug Conjugate according to any one of claims 1 to 2 or claims 7 to 9, or a Drug-Linker Conjugate according to any one of claims 4 to 9, wherein -$W_{w'}$- is -Val-Cit-, -Phe-Lys- or -Val-Ala-.

**11.** A Ligand-Drug Conjugate according to any one of claims 1 to 2 or claims 7 to 10, or a Drug-Linker Conjugate according to any one of claims 4 to 10, wherein -$Y_{y'}$- is represented by:

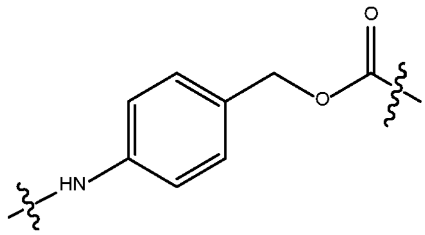

.

**12.** A Ligand-Drug Conjugate according to any one of claims 1 to 2 or claim 11, or a Drug-Linker Conjugate according to any one of claims 4 to 8 or 11, wherein -$W_{w'}$- is a Glucuronide Unit having the structure of:

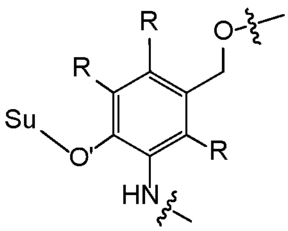

or

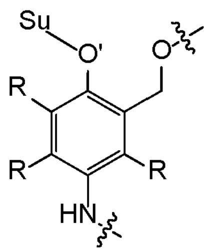

;

wherein Su is a Sugar moiety;

-O'- represents a glycosidic bond providing a β-glucuronidase-cleavage site that is cleavable by human lysosomal β-glucuronidase;
each R is independently hydrogen, a halogen, -CN, or -$NO_2$;
the wavy bond adjacent to the nitrogen atom indicates covalent attachment to the Stretcher unit or to the antibody (Ab); and
the wavy bond adjacent to the oxygen indicates covalent attachment to the Spacer unit (Y) or to the Drug unit (D).

**13.** A Drug-Linker Conjugate according to claim 4, having the structure:

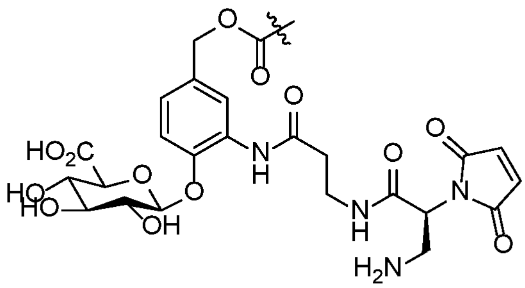

wherein the wavy line adjacent to the carbonyl indicates covalent attachment to the Drug (D).

**14.** A Drug-Linker Conjugate according to claim 4, having the structure:

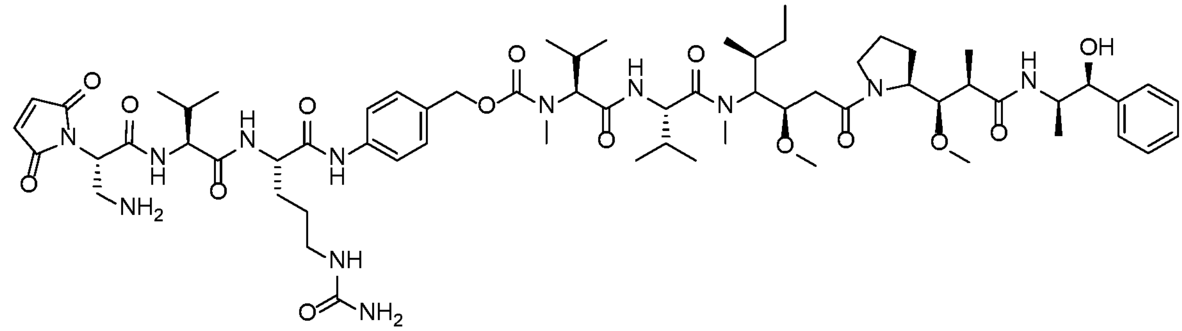

.

**15.** A Ligand-Drug Conjugate according to claim 1, having the structure:

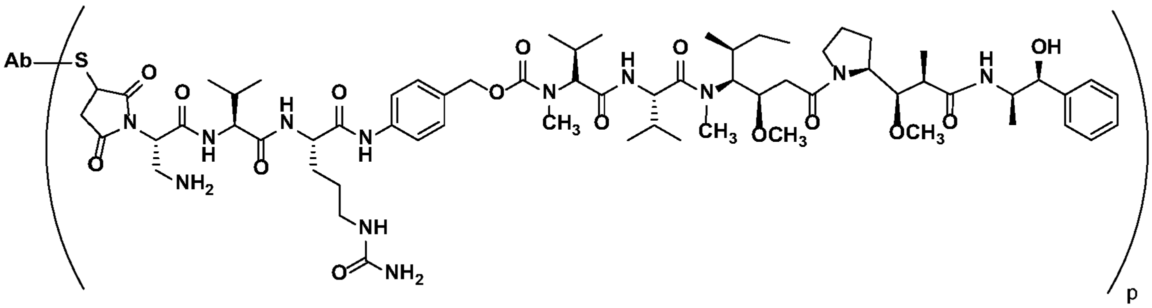

,

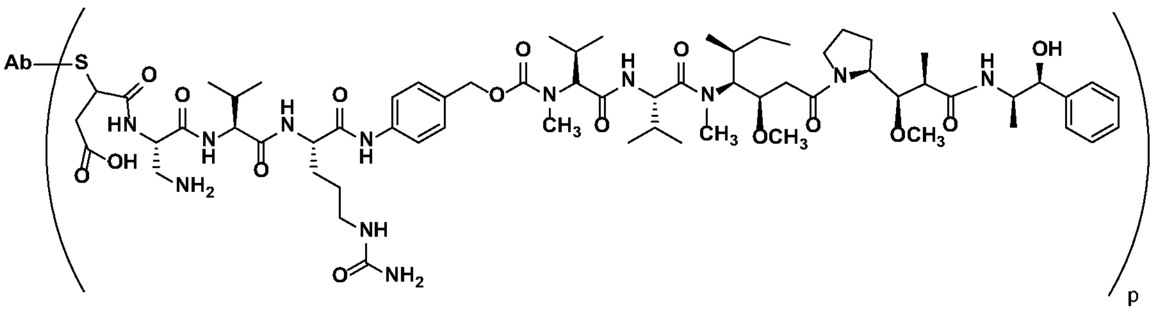

,

or

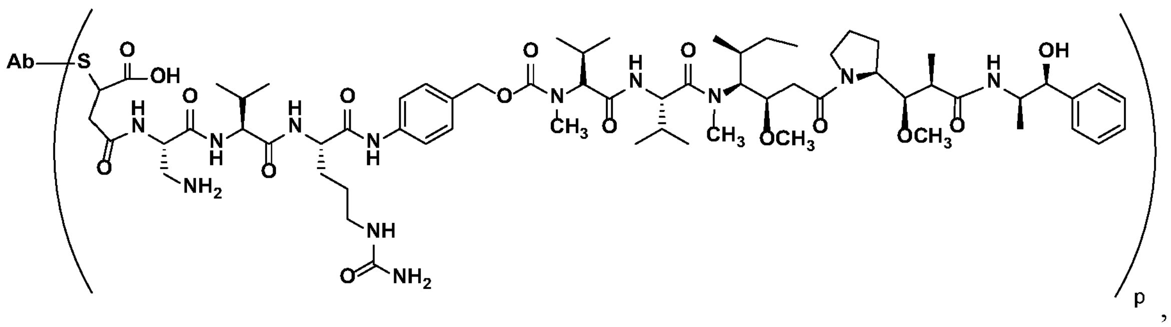

or a salt thereof, wherein
Ab is a monoclonal antibody and S is a sulfur atom from the monoclonal antibody.

**16.** A Ligand-Drug Conjugate according to any one of claims 1 to 3, 6 to 12, or 15 for use in method of treating cancer, the cancer cells of which express a target antigen, wherein the antibody (Ab) is a monoclonal antibody that specifically binds to the target antigen.

## Patentansprüche

**1.** Ligand-Arzneimittel-Konjugat der folgenden Formel:

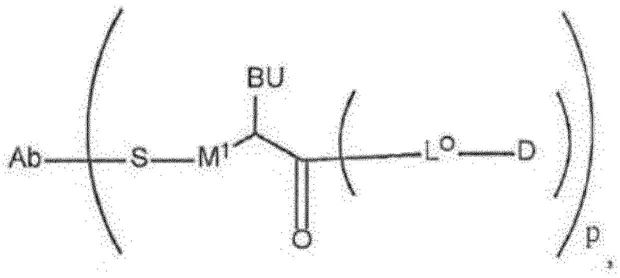

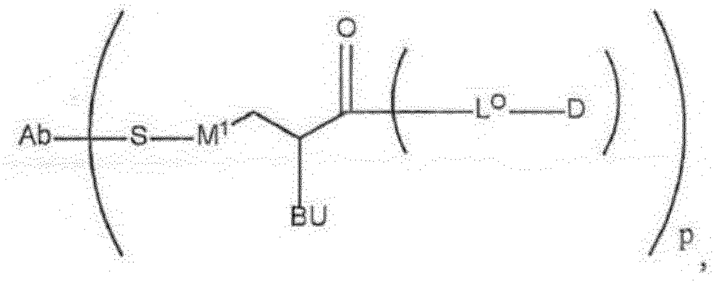

or

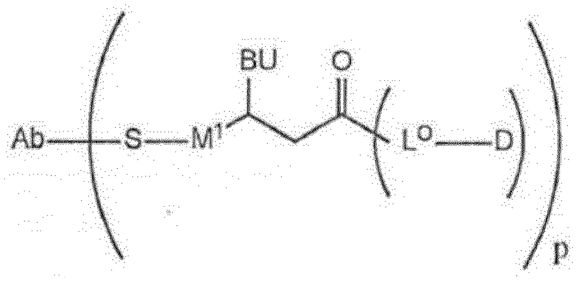

oder ein Salz davon, worin D eine Arzneimittel-Einheit ist und Ab ein Antikörper ist;

und worin:

S ein Schwefelatom des Antikörpers ist;
$M^1$ ein Succinimid-Ring oder hydrolysiertes Succinimid ist oder zusammen mit BU ein Dilactam bildet, das von der Reaktion der Base von BU mit dem Succinimid-Ring herrührt;
BU eine Grundeinheit ist, die aus der aus $-(CH_2)_xNH_2$, $-(CH_2)_xNHR^a$ und $-(CH_2)_xNR^a_2$ bestehenden Gruppe ausgewählt ist, worin x eine ganze Zahl von 0 bis 4 ist und die $R^a$ jeweils unabhängig aus der aus $C_{1-6}$-Alkyl und $C_{1-6}$-Halogenalkyl bestehenden Gruppe ausgewählt sind oder zwei Gruppen $R^a$ zusammen mit dem Stickstoffatom, an das sie gebunden sind, eine Azetidinyl-, Pyrrolidinyl- oder Piperidinyl-Gruppe bilden, mit der Maßgabe, dass nicht weniger als 2 Atome zwischen der Base der Grundeinheit und dem Stickstoffatom des Succinimids (hydrolysiert oder nicht hydrolysiert) oder Dilactams liegen;
der Index p im Bereich von 1 bis 20 liegt; und
$L^O$ eine optionale sekundäre Linker-Anordnung der folgenden Formel ist:

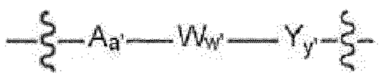

worin -A- eine optionale Strecker-Einheit ist, der Index a' = 0 oder 1 ist; -W- eine optionale spaltbare Einheit ist, der Index w' = 0 oder 1 ist; und -Y- eine optionale Spacer-Einheit ist und der Index y' = 0 oder 1 ist; und die Wellenlinien die Anbindungspunkte an die selbststabilisierende Linker-Anordnung und die Arzneimittel-Einheit anzeigen, wobei gegebenenfalls:

(i) w' = 1 ist und W direkt an die Arzneimittel-Einheit konjugiert ist,
(ii) w' = 1 ist und W direkt an die Spacer-Einheit konjugiert ist,
(iii) w' = 1 ist und W über eine spaltbare Peptid-, Disulfid- oder Hydrazin-Bindung direkt an die Arzneimittel-Einheit oder die Spacer-Einheit konjugiert ist,
(iv) W fehlt oder
(v) W vorhanden ist und die Spacer-Einheit und die Strecker-Einheit fehlen.

**2.** Ligand-Arzneimittel-Konjugat nach Anspruch 1 der folgenden Formel:

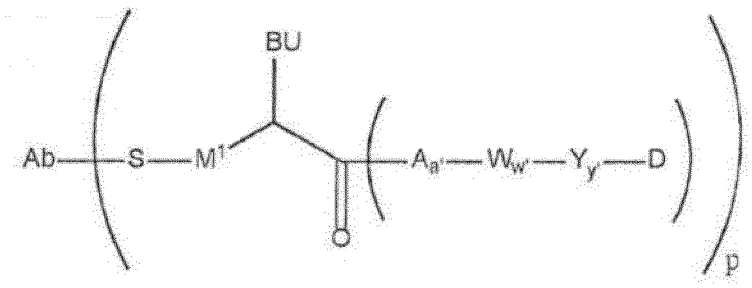

oder ein pharmazeutisch annehmbares Salz davon, worin

der Index p im Bereich von 1 bis 12 liegt;
BU $-CH_2NH_2$, $-CH_2CH_2NH_2$, $-CH_2CH_2CH_2NH_3$ oder $-CH_2CH_2CH_2CH_2NH_2$ ist; und $M^1$ ein Succinimid-Ring oder ein hydrolysierter Succinimid-Ring ist und D aus der aus Auristatinen und Pyrrolo[1,4]benzodiazepinen (PBDs) bestehenden Gruppe ausgewählt ist.

**3.** Ligand-Arzneimittel-Konjugat nach Anspruch 1 mit der folgenden Struktur:

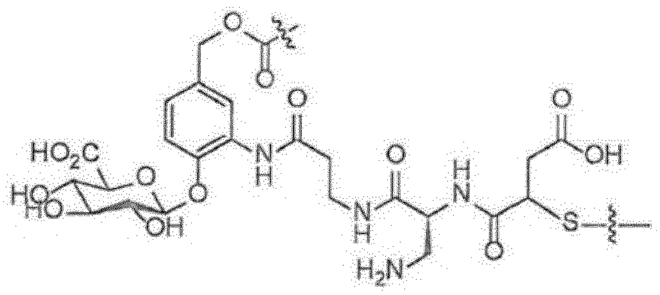

worin die Wellenlinie neben dem Carbonyl eine kovalente Anbindung an die Arzneimittel-Einheit (D), gegebenenfalls über eine Spacer-Einheit (Y), anzeigt und S ein Schwefelatom des Antikörpers (Ab) zur kovalenten Anbindung an die Linkereinheit ist.

**4.** Arzneimittel-Linker-Konjugat der folgenden Formel:

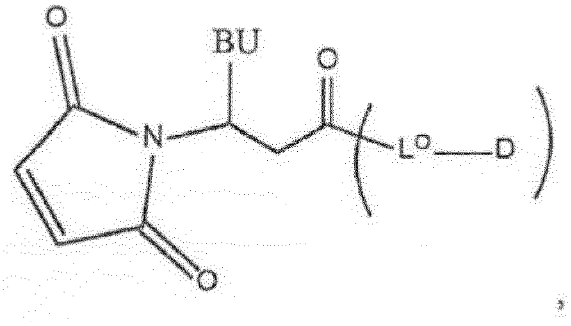

,

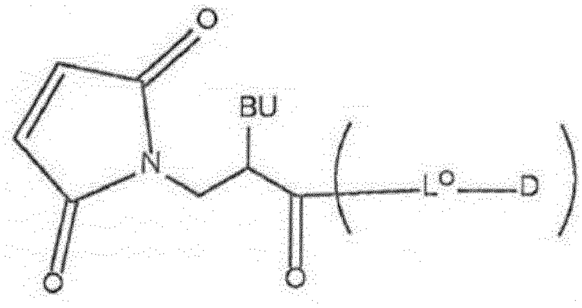

oder

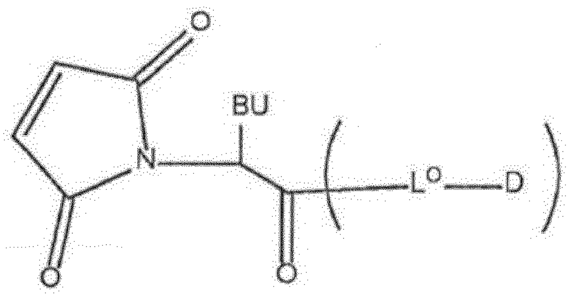

oder ein Salz davon, worin

D eine Arzneimittel-Einheit ist;
BU eine Grundeinheit ist, die aus der aus $-(CH_2)_xNH_2$, $-(CH_2)_xNHR^a$ und $-(CH_2)_xNR^a_2$ bestehenden Gruppe ausgewählt ist, worin x eine ganze Zahl von 0 bis 4 ist und die $R^a$ jeweils unabhängig aus der aus der aus

$C_{1-6}$-Alkyl und $C_{1-6}$-Halogenalkyl bestehenden Gruppe ausgewählt sind oder zwei $R^a$-Gruppen zusammen mit dem Stickstoffatom, an das sie gebunden sind, eine Azetidinyl-, Pyrrolidinyl- oder Piperidinyl-Gruppe bilden, mit der Maßgabe, dass nicht weniger als 2 Atome zwischen der Base der Grundeinheit und dem Stickstoffatom des Succinimids (hydrolysiert oder nicht hydrolysiert) oder Maleinimids liegen; das Arzneimittel ein zytotoxisches Mittel ist; und

$L^O$ eine optionale sekundäre Linker-Anordnung der folgenden Formel ist:

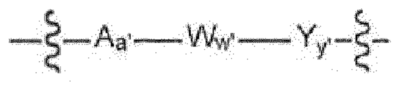

worin -A- eine optionale Strecker-Einheit ist, der Index a' = 0 oder 1 ist; -W- eine optionale spaltbare Einheit ist, der Index w' = 0 oder 1 ist; und -Y- eine optionale Spacer-Einheit ist, und der Index y' = 0 oder 1 ist; und die Wellenlinien die Anbindungspunkte an die selbststabilisierende Linker-Anordnung und die Arzneimittel-Einheit anzeigen, wobei gegebenenfalls:

(i) w' = 1 ist und W direkt an die Arzneimittel-Einheit konjugiert ist,
(ii) w' = 1 ist und W direkt an die Spacer-Einheit konjugiert ist,
(iii) w' = 1 ist und W über eine spaltbare Peptid-, Disulfid- oder Hydrazin-Bindung direkt an die Arzneimittel-Einheit oder die Spacer-Einheit konjugiert ist,
(iv) W fehlt oder
(v) W vorhanden ist und die Spacer-Einheit und die Strecker-Einheit fehlen.

**5.** Arzneimittel-Linker-Konjugat nach Anspruch 4 der folgenden Formel:

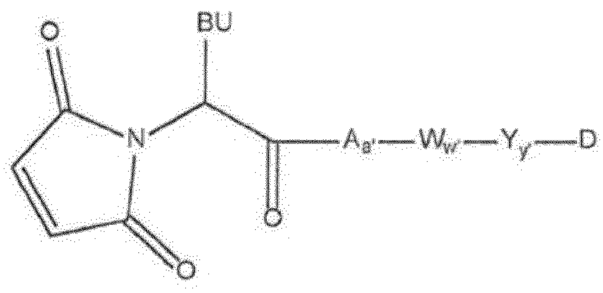

oder ein Salz davon, worin
BU $-CH_2NH_2$, $-CH_2CH_2NH_2$, $-CH_2CH_2CH_2NH_2$ oder $-CH_2CH_2CH_2CH_2NH_2$ ist und D aus der aus Auristatinen und Pyrrolo[1,4]benzodiazepinen (PBDs) bestehenden Gruppe ausgewählt ist.

**6.** Ligand-Arzneimittel-Konjugat nach einem der Ansprüche 1 bis 3 oder Arzneimittel-Linker-Konjugat nach Anspruch 4, worin D aus der aus Folgendem bestehenden Gruppe ausgewählt ist:

(a) Antitubulinmittel, Auristatinen, an die kleine Furche von DNA bindenden Mitteln, DNA-Replikationsinhibitoren, Alkylierungsmitteln, Anthracyclinen, Antibiotika, Antifolaten, Antimetaboliten, Chemotherapie-Sensibilisierungsmitteln, Duocarmycinen, Etoposiden, fluorierten Pyrimidinen, lonophoren, Lexitropsinen, Nitrosoharnstoffen, Platinolen, porenbildenden Verbindungen, Purinantimetaboliten, Puromycinen, Strahlungssensibilisierungsmitteln, Steroiden, Taxanen, Topoisomeraseinhibitoren und Vincaalkaloiden;
(b) Auristatinen, Camptothecinen, Duocarmycinen, Etoposiden, Maytansinen und Mayiansinoiden, Taxanen, Benzodiazepinen und Vincaalkaloiden; oder
(c) Auristatinen und Pyrrolo[1,4]benzodiazepinen (PBDs).

**7.** Ligand-Arzneimittel-Konjugat nach einem der Ansprüche 1 bis 3 oder Arzneimittel-Linker-Konjugat nach einem der Ansprüche 4 bis 6, worin D aus der aus Auristatinen mit der folgenden Struktur bestehenden Gruppe:

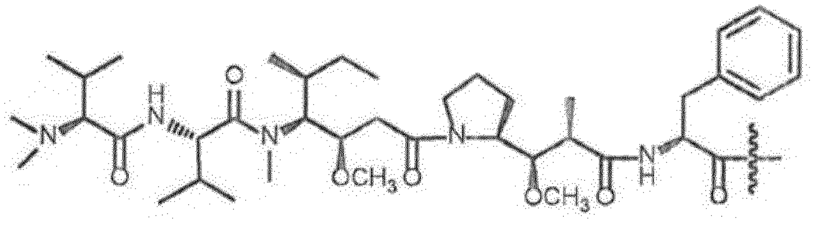

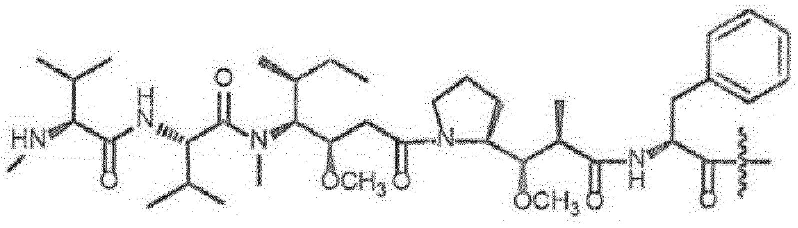

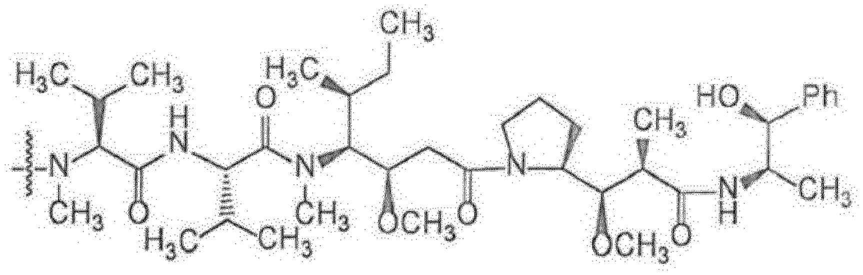

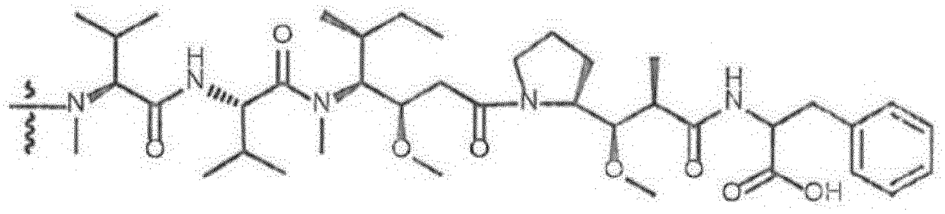

und pharmazeutisch annehmbaren Salzen davon ausgewählt ist, worin die Wellenlinie die Anbindungsstelle an die Linkereinheit anzeigt.

**8.** Ligand-Arzneimittel-Konjugat nach einem der Ansprüche 1 bis 2 oder Anspruch 7 oder Arzneimittel-Linker-Konjugat nach einem der Ansprüche 4 bis 7, wobei die Strecker-Einheit (A) aus der aus $-NH-C_1-C_{10}$-Alkylen-, $-NH-C_1-C_{10}$-Alkylen-NH-C(O)-$C_1-C_{10}$-alkylen-, -N H-$C_1-C_{10}$-Alkylen-C(O)-NH-$C_1-C_{10}$-alkylen-, $-NH-(CH_2CH_2O)_s$, $-NH-(CH_2CH_2O)_sCH_2-$, $-NH-(CH_2CH_2NH)_s(CH_2)_s$ $-NH-(CH_2CH_2NH)_s(CH_2)_sNH-C(O)-(CH_2)_s$ -NH-($C_3-C_8$-Carbocyclo)-, -NH-(Arylen)- und -NH-($C_3-C_8$-Heterocyclo)- bestehenden Gruppe ausgewählt ist, worin die s jeweils unabhängig 1 bis 10 sind, oder wobei die Strecker-Einheit (A) die folgende Formel aufweist:

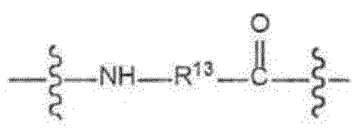

worin $R^{13}$ -$C_1-C_{10}$-Alkylen-, -$C_3-C_8$-Carbocyclo-, -Arylen-, -$C_1-C_{30}$-Heteroalkylen-, -$C_3-C_8$-Heterocyclo-, -$C_1-C_{10}$-Alkylenarylen-, -Arylen-$C_1-C_{10}$-alkylen-, -$C_1-C_{10}$-Alkylen-($C_3-C_8$-Carbocyclo)-, -($C_3-C_8$-Carbocyclo)-$C_1-C_{10}$-alkylen-, -$C_1-C_{10}$-Alkylen-($C_3-C_8$-heterocyclo)-, -($C_3-C_8$-Heterocyclo)-$C_1-C_{10}$-alkylen-, $-(CH_2CH_2O_{1-10}(-CH_2)_{1-3}-$ oder $-(CH_2CH_2NH)_{1-10}(-CH_2)_{1-3}-$, vorzugsweise -$C_1-C_{10}$-Alkylen oder -$C_1-C_{30}$-Heteroalkylen-, noch bevorzugter ist $R^{13}$ -$C_1-C_{10}$-Alkylen-, $-(CH_2CH_2O_{1-10}(-CH_2)_{1-3}-$ oder $-(CH_2CH\ NH)_{1-10}(-CH_2)_{1-3}-$, ist.

**9.** Ligand-Arzneimittel-Konjugat nach einem der Ansprüche 1 bis 2 oder der Ansprüche 7 bis 8 oder Arzneimittel-Linker-Konjugat nach einem der Ansprüche 4 bis 8, worin $-W_{w'}$-durch $-(AA)_{1-12}$- oder $-(AA-AA)_{1-6}$- dargestellt ist, worin AA bei jedem Vorkommen unabhängig aus natürlichen oder nicht natürlichen Aminosäuren ausgewählt ist, vorzugsweise unabhängig aus natürlichen Aminosäuren ausgewählt ist, und die Bindung zwischen der spaltbaren Einheit (W) und der Arzneimittel-Einheit (D) oder der Spacer-Einheit (Y) durch eine tumorassoziierte Protease, vorzugsweise Cathepsin B, C und D, oder eine Plasmidprotease enzymatisch spaltbar ist.

**10.** Ligand-Arzneimittel-Konjugat nach einem der Ansprüche 1 bis 2 oder Ansprüche 7 bis 9 oder Arzneimittel-Linker-Konjugat nach einem der Ansprüche 4 bis 9, worin $-W_{w'}$--Val-Cit-, -Phe-Lys- oder -Val-Ala- ist.

**11.** Ligand-Arzneimittel-Konjugat nach einem der Ansprüche 1 bis 2 oder Ansprüche 7 bis 10 oder Arzneimittel-Linker-Konjugat nach einem der Ansprüche 4 bis 10, worin $-Y_y$-dargestellt ist durch:

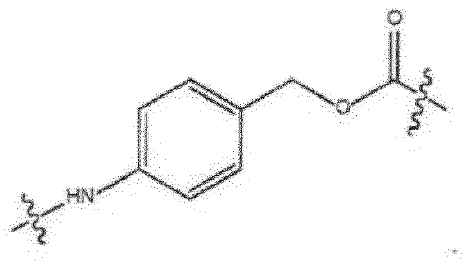

**12.** Ligand-Arzneimittel-Konjugat nach einem der Ansprüche 1 bis 2 oder Anspruch 11 oder Arzneimittel-Linker-Konjugat nach einem der Ansprüche 4 bis 8 oder 11, worin $-W_{w'}$-eine Glucuronid-Einheit mit der folgenden Struktur ist:

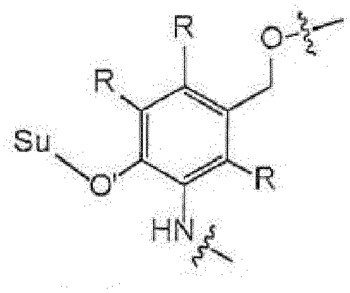

oder

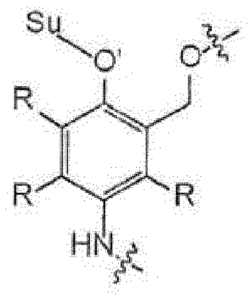

;

worin Su eine Zuckergruppierung ist;

-O'- für eine glykosidische Bindung steht, die eine β-Glucuronidase-Spaltstelle bereitstellt, die durch menschliche lysosomale β-Glucuronidase spaltbar ist;
die R jeweils unabhängig Wasserstoff, ein Halogen, -CN oder $-NO_2$ sind;
die gewellte Bindung neben dem Stickstoffatom eine kovalente Anbindung an die Strecker-Einheit oder an den Antikörper (Ab) anzeigt; und
die gewellte Bindung neben dem Sauerstoff eine kovalente Anbindung an die Spacer-Einheit (Y) oder die Arzneimittel-Einheit (D) anzeigt.

**13.** Arzneimittel-Linker-Konjugat nach Anspruch 4 mit der folgenden Struktur:

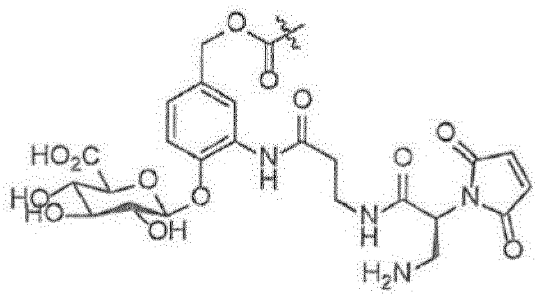

worin die Wellenlinie neben dem Carbonyl eine kovalente Anbindung an das Arzneimittel (D) anzeigt.

**14.** Arzneimittel-Linker-Konjugat nach Anspruch 4 mit der folgenden Struktur:

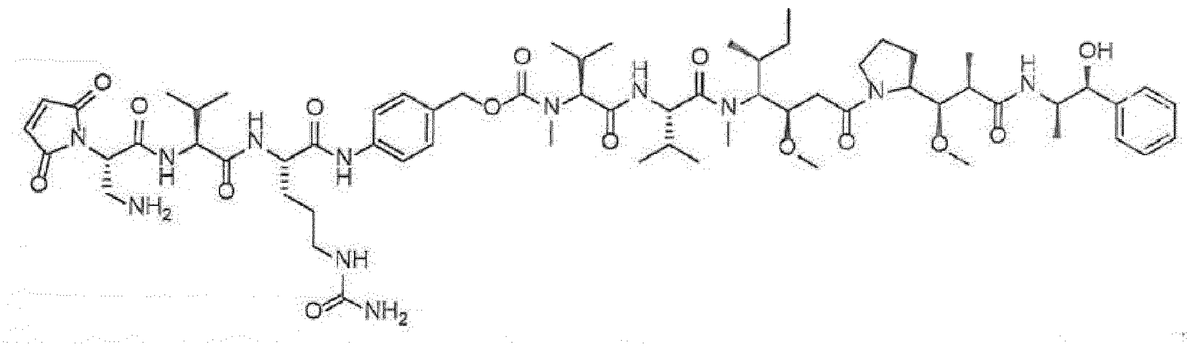

**15.** Arzneimittel-Linker-Konjugat nach Anspruch 1 mit der folgenden Struktur:

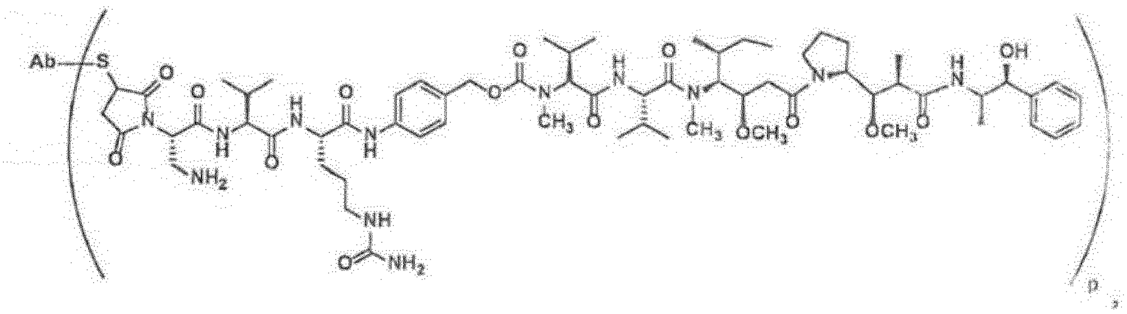

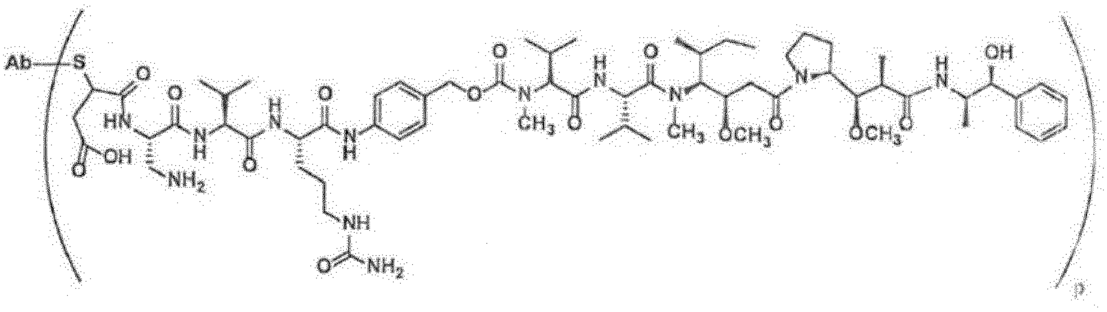

oder

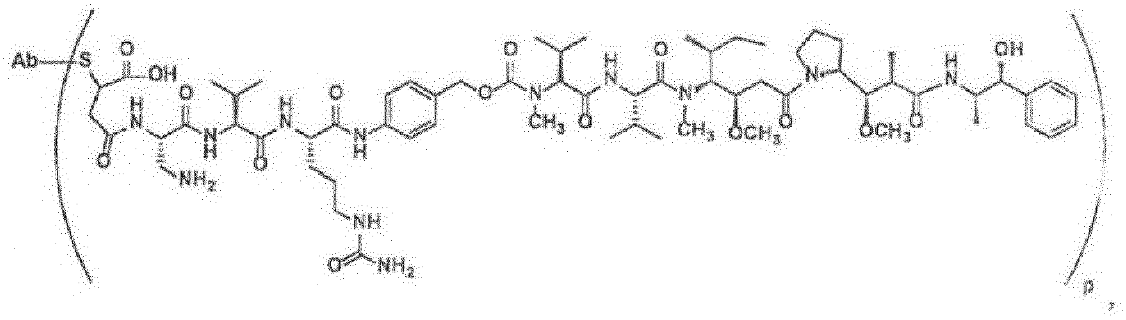

oder ein Salz davon, worin Ab ein monoklonaler Antikörper ist und S ein Schwefelatom des monoklonalen Antikörpers ist.

16. Ligand-Arzneimittel-Konjugat nach einem der Ansprüche 1 bis 3, 6 bis 12 oder 15 zur Verwendung in einem Verfahren zur Behandlung von Krebs, dessen Krebszellen ein Zielantigen exprimieren, wobei der Antikörper (Ab) ein monoklonaler Antikörper ist, der spezifisch an das Zielantigen bindet.

## Revendications

1. Conjugué ligand-médicament ayant la formule :

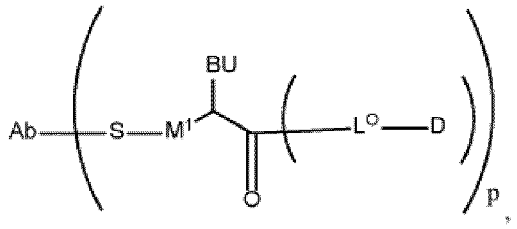

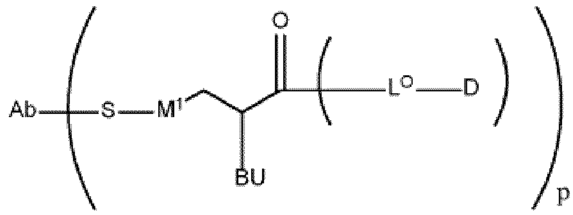

ou

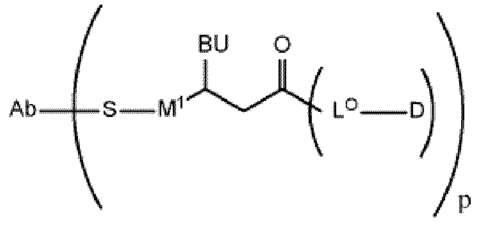

ou un sel de celui-ci, dans lequel D est une unité de médicament, et Ab est un anticorps ;
et dans lequel :

S est un atome de soufre de l'anticorps ;
$M^1$ est un noyau succinimide ou un succinimide hydrolysé ou conjointement avec BU forme un dilactame résultant de la réaction de la base de BU avec le noyau succinimide ;

BU est une unité basique choisie dans le groupe constitué de $-(CH_2)_xNH_2$, $-(CH_2)_xNHR^a$ et $-(CH_2)_xNR^a{}_2$, dans lequel x est un nombre entier de 0 à 4 et chaque $R^a$ est indépendamment choisi dans le groupe constitué d'un alkyle en $C_{1-6}$ et d'un halogénoalkyle en $C_{1-6}$, ou deux groupes $R^a$ sont combinés avec l'azote auquel ils sont fixés pour former un groupe azétidinyle, pyrrolidinyle ou pipéridinyle à condition qu'il n'existe pas moins de 2 atomes intermédiaires entre la base de l'unité basique et l'atome d'azote du succinimide (hydrolysé ou non hydrolysé) ou du dilactame ;
l'unité de médicament est un agent cytotoxique ;
l'indice inférieur p se situe dans une plage de 1 à 20 ; et
$L^O$ est un ensemble lieur secondaire facultatif ayant la formule :

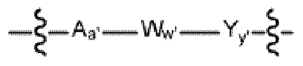

dans lequel -A- est une unité d'étirement facultative, l'indice inférieur a' vaut 0 ou 1 ; -W- est une unité clivable facultative, l'indice inférieur w' vaut 0 ou 1 ; et -Y- est une unité d'espacement facultative, et l'indice inférieur y' vaut 0 ou 1 ; et les lignes ondulées indiquent les points de fixation à l'ensemble lieur à auto-stabilisation et à l'unité de médicament, facultativement dans lequel :

(i) w' vaut 1 et W est conjugué directement à l'unité de médicament,
(ii) w' vaut 1 et W est conjugué directement à l'unité d'espacement,
(iii) w' vaut 1 et W est conjugué directement à l'unité de médicament ou à l'unité d'espacement par l'intermédiaire d'une liaison peptide, disulfure ou hydrazine clivable,
(iv) W est absent, ou
(v) W est présent et l'unité d'espacement et l'unité d'étirement sont absentes.

**2.** Conjugué ligand-médicament selon la revendication 1, qui est de formule :

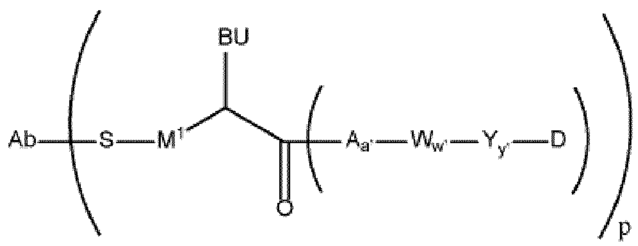

ou un sel pharmaceutiquement acceptable de celui-ci, dans lequel

l'indice inférieur p se situe dans une plage de 1 à 12 ;
BU est $-CH_2NH_2$, $-CH_2CH_2NH_2$, $-CH_2CH_2CH_2\ NH_2$ ou $-CH_2CH_2CH_2CH_2\ NH_2$ ; et $M^1$ est un noyau succinimide ou un noyau succinimide hydrolysé et D est choisi dans le groupe constitué d'auristatines et de pyrrolo[1,4]benzodiazépines (PBD).

**3.** Conjugué ligand-médicament selon la revendication 1, ayant la structure :

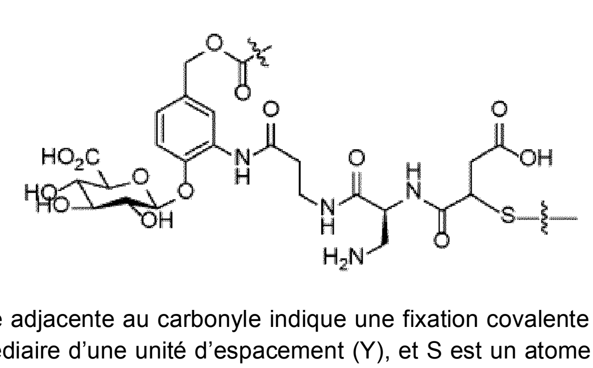

dans lequel la ligne ondulée adjacente au carbonyle indique une fixation covalente à l'unité de médicament (D), facultativement par l'intermédiaire d'une unité d'espacement (Y), et S est un atome de soufre de l'anticorps (Ab) pour une fixation covalente de l'unité de lieur.

**4.** Conjugué médicament-lieur ayant la formule :

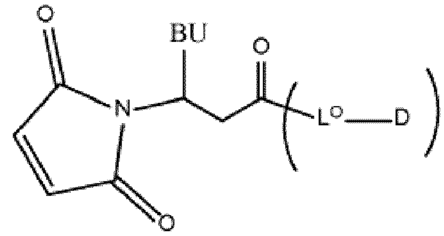

,

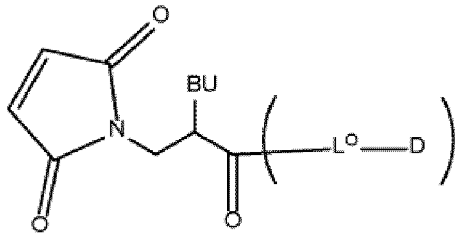

ou

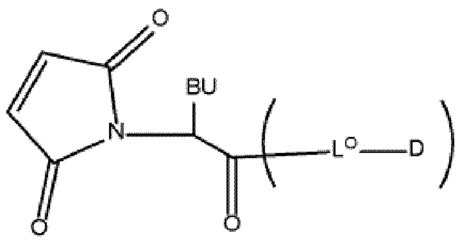

ou un sel de celui-ci, dans lequel

D est une unité de médicament ;
BU est une unité basique choisie dans le groupe constitué de $-(CH_2)_xNH_2$, $-(CH_2)_xNHR^a$ et $-(CH_2)_xNR^a{}_2$, dans lequel x est un nombre entier de 0 à 4 et chaque $R^a$ est indépendamment choisi dans le groupe constitué d'un alkyle en $C_{1-6}$ et d'un halogénoalkyle en $C_{1-6}$, ou deux groupes $R^a$ sont combinés avec l'azote auquel ils sont fixés pour former un groupe azétidinyle, pyrrolidinyle ou pipéridinyle à condition qu'il n'existe pas moins de 2 atomes intermédiaires entre la base de l'unité basique et l'atome d'azote du maléimide ;
l'unité de médicament est un agent cytotoxique ; et
$L^O$ est un ensemble lieur secondaire facultatif ayant la formule :

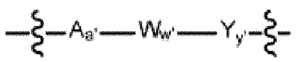

dans lequel -A- est une unité d'étirement facultative, l'indice inférieur a' vaut 0 ou 1 ; -W- est est une unité clivable facultative, l'indice inférieur w' vaut 0 ou 1 ; et -Y- est une unité d'espacement facultative, et l'indice inférieur y' vaut 0 ou 1 ; et les lignes ondulées indiquent les points de fixation à l'ensemble lieur à auto-stabilisation et à l'unité de médicament, facultativement dans lequel :

(i) w' vaut 1 et W est conjugué directement à l'unité de médicament,
(ii) w' vaut 1 et W est conjugué directement à l'unité d'espacement,
(iii) w' vaut 1 et W est conjugué directement à l'unité de médicament ou à l'unité d'espacement par l'intermédiaire d'une liaison peptide, disulfure ou hydrazine clivable,
(iv) W est absent, ou
(v) W est présent et l'unité d'espacement et l'unité d'étirement sont absentes.

**5.** Conjugué médicament-lieur selon la revendication 4 ayant la formule :

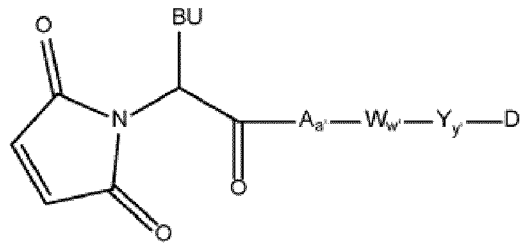

ou sel de celui-ci, dans lequel
BU est $-CH_2NH_2$, $-CH_2CH_2NH_2$, $-CH_2CH_2CH_2$ $NH_2$ ou $-CH_2CH_2CH_2CH_2$ $NH_2$ ; et D est choisi dans le groupe constitué d'auristatines et de pyrrolo[1,4]benzodiazépines (PBD).

**6.** Conjugué ligand-médicament selon l'une quelconque des revendications 1 à 3 ou conjugué médicament-lieur selon la revendication 4, dans lequel D est choisi dans le groupe constitué de :

(a) agents antitubuline, auristatines, agents de liaison au petit sillon d'ADN, inhibiteurs de réplication d'ADN, agents d'alkylation, anthracyclines, antibiotiques, antifolates, antimétabolites, sensibilisateurs de chimiothérapie, duocarmycines, étoposides, pyrimidines fluorées, ionophores, lexitropsines, nitrosourées, platinols, composés porogènes, antimétabolites de purine, puromycines, sensibilisateurs de rayonnement, stéroïdes, taxanes, inhibiteurs de topoisomérase et vinca-alcaloïdes ;
(b) auristatines, camptothécines, duocarmycines, étoposides, maytansines et maytansinoïdes, taxanes, benzodiazépines et vinca-alcaloïdes ; ou
(c) auristatines et pyrrolo[1,4]benzodiazépines (PBD).

**7.** Conjugué ligand-médicament selon l'une quelconque des revendications 1 à 3 ou conjugué médicament-lieur selon l'une quelconque des revendications 4 à 6, dans lequel D est choisi dans le groupe constitué d'auristatines ayant les structures de :

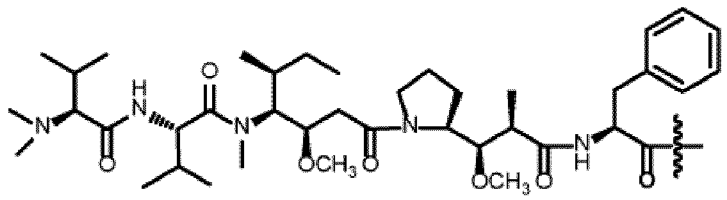

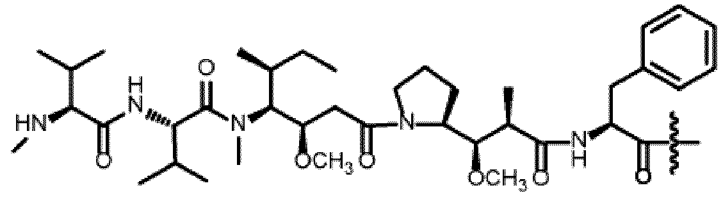

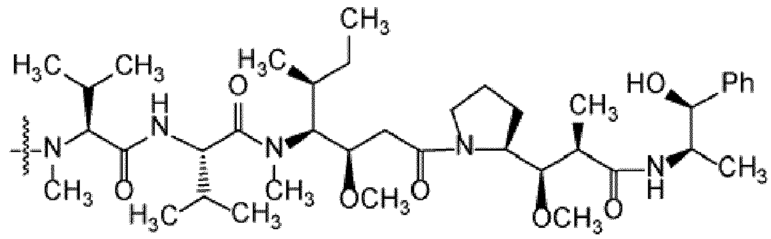

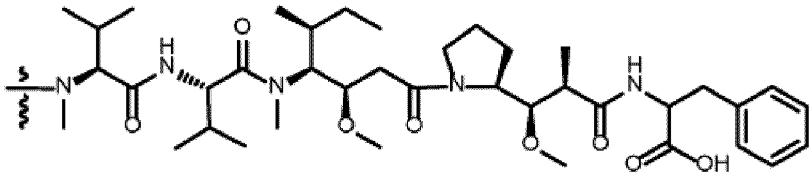

et sel pharmaceutiquement acceptable de celui-ci dans lequel la ligne ondulée indique le site de fixation à l'unité de lieur.

**8.** Conjugué ligand-médicament selon l'une quelconque des revendications 1 à 2 ou la revendication 7, ou conjugué médicament-lieur selon l'une quelconque des revendications 4 à 7, dans lequel l'unité d'étirement (A) est choisie dans le groupe constitué de -NH-alkylène en $C_1$-$C_{10}$-, -NH-alkylène en $C_1$-$C_{10}$-NH-C(O)-alkylène en $C_1$-$C_{10}$-, -NH-alkylène en $C_1$-$C_{10}$-C(O)-NH-alkylène en $C_1$-$C_{10}$-, -NH-$(CH_2CH_2O)_s$-, -NH-$(CH_2CH_2O)_s$-$CH_2$-, -NH-$(CH_2CH_2NH)_s$-$(CH_2)_s$, -NH-$(CH_2CH_2NH)_s$-$(CH_2)_s$-NH-C(O)-$(CH_2)_s$, -NH- (carbocyclo en $C_3$-$C_8$)-, -NH- (arylène) - et -NH- (hétérocyclo en $C_3$-$C_8$)-, dans lequel chaque s vaut indépendamment 1 à 10, ou dans lequel l'unité d'étirement (A) a la formule de :

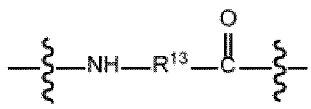

dans lequel $R^{13}$ est un -alkylène en $C_1$-$C_{10}$-, -carbocyclo en $C_3$-$C_8$-, -arylène-, -hétéroalkylène en $C_1$-$C_{30}$-, -hétérocyclo en $C_3$-$C_8$-, -alkylène en $C_1$-$C_{10}$-arylène-, -arylène-alkylène en $C_1$-$C_{10}$-, -alkylène en $C_1$-$C_{10}$- (carbocyclo en $C_3$-$C_8$)-, - (carbocyclo en $C_3$-$C_8$)-alkylène en $C_1$-$C_{10}$-, -alkylène en $C_1$-$C_{10}$-(hétérocyclo en $C_3$-$C_8$)-, -(hétérocyclo en $C_3$-$C_8$)-alkylène en $C_1$-$C_{10}$-, -$(CH_2CH_2O)_{1-10}$(-$CH_2)_{1-3}$- ou -$(CH_2CH_2NH)_{1-10}$(-$CH_2)_{1-3}$-, de préférence un -alkylène en $C_1$-$C_{10}$- ou un -hétéroalkylène en $C_1$-$C_{30}$-, de manière davantage préférée $R^{13}$ est un - alkylène en $C_1$-$C_{10}$-, -$(CH_2CH_2O)_{1-10}$(-$CH_2)_{1-3}$- ou -$(CH_2CH_2NH)_{1-10}$(-$CH_2)_{1-3}$-.

**9.** Conjugué ligand-médicament selon l'une quelconque des revendications 1 à 2 ou des revendications 7 à 8, ou conjugué médicament-lieur selon l'une quelconque des revendications 4 à 8, dans lequel -$W_{w'}$- est représenté par - $(AA)_{1-12}$- ou -$(AA\text{-}AA)_{1-6}$- dans lequel AA est à chaque occurrence indépendamment choisi parmi des acides aminés naturels ou non naturels, de préférence indépendamment choisi parmi des acides aminés naturels, et la liaison entre l'unité clivable (W) et l'unité de médicament (D) ou l'unité d'espacement (Y) est clivable par voie enzymatique par une protéase associée à une tumeur, de préférence par la cathépsine B, C et D, ou une plasmine protéase.

**10.** Conjugué ligand-médicament selon l'une quelconque des revendications 1 à 2 ou des revendications 7 à 9, ou conjugué médicament-lieur selon l'une quelconque des revendications 4 à 9, dans lequel -$W_{w'}$- est -Val-Cit-, -Phe-Lys- ou -Val-Ala-.

**11.** Conjugué ligand-médicament selon l'une quelconque des revendications 1 à 2 ou des revendications 7 à 10, ou conjugué médicament-lieur selon l'une quelconque des revendications 4 à 10, dans lequel -$Y_{y'}$- est représenté par :

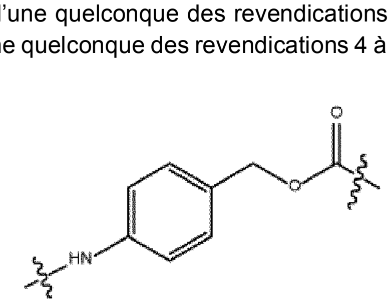

.

**12.** Conjugué ligand-médicament selon l'une quelconque des revendications 1 à 2 ou de la revendication 11, ou conjugué médicament-lieur selon l'une quelconque des revendications 4 à 8 ou 11, dans lequel -$W_{w'}$- est une unité glucuronide ayant la structure de :

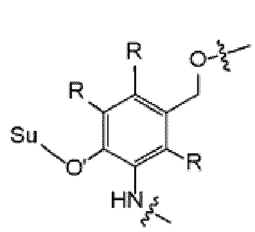 ou 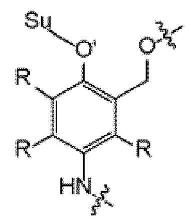 ;

dans lequel Su est un groupement sucre ;

-O'- représente une liaison glycosidique fournissant un site de clivage de β-glucuronidase qui est clivable par une β-glucuronidase lysosomale humaine ;
chaque R est indépendamment un hydrogène, un halogène, -CN ou $-NO_2$ ;
la liaison ondulée adjacente à l'atome d'azote indique une fixation covalente à l'unité d'étirement ou à l'anticorps (Ab) ; et
la liaison ondulée adjacente à l'oxygène indique une fixation covalente à l'unité d'espacement (Y) ou à l'unité de médicament (D).

**13.** Conjugué médicament-lieur selon la revendication 4, ayant la structure :

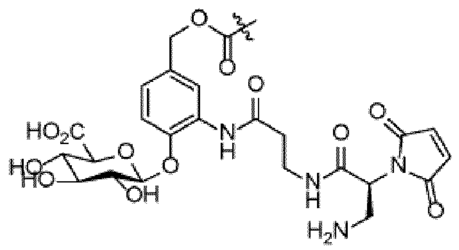

dans lequel la ligne ondulée adjacente au carbonyle indique une fixation covalente au médicament (D).

**14.** Conjugué médicament-lieur selon la revendication 4, ayant la structure :

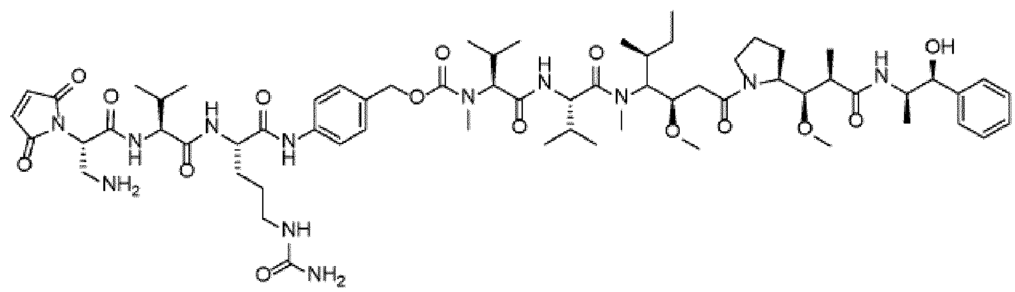

.

**15.** Conjugué ligand-médicament selon la revendication 1, ayant la structure :

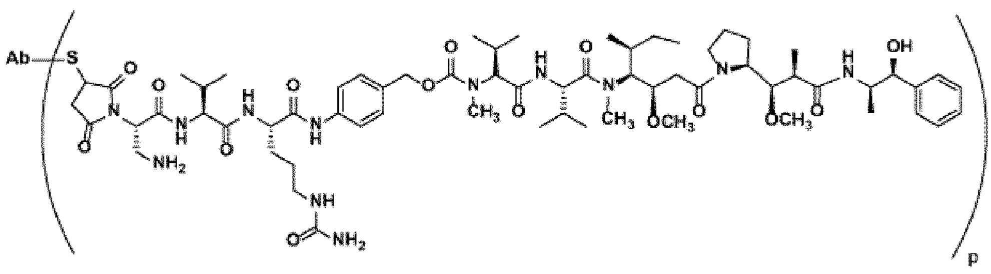

,

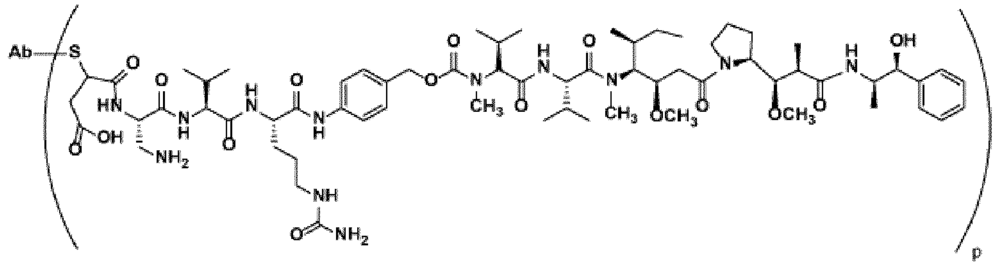

ou

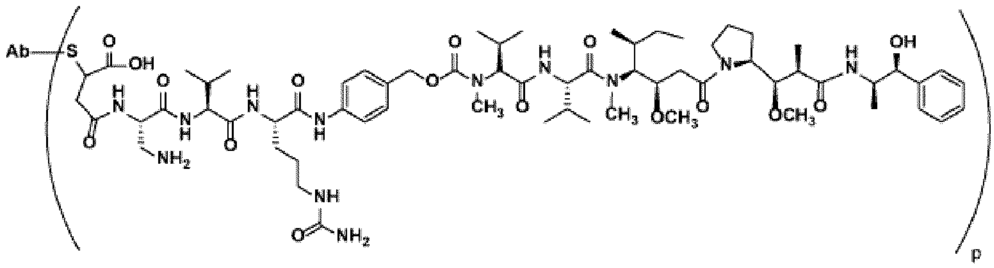

,

ou un sel de celui-ci, dans lequel
Ab est un anticorps monoclonal et S est un atome de soufre de l'anticorps monoclonal.

**16.** Conjugué ligand-médicament selon l'une quelconque des revendications 1 à 3, 6 à 12, ou 15 pour une utilisation dans un procédé de traitement d'un cancer, dont les cellules cancéreuses expriment un antigène cible, dans lequel l'anticorps (Ab) est un anticorps monoclonal qui se lie spécifiquement à l'antigène cible.

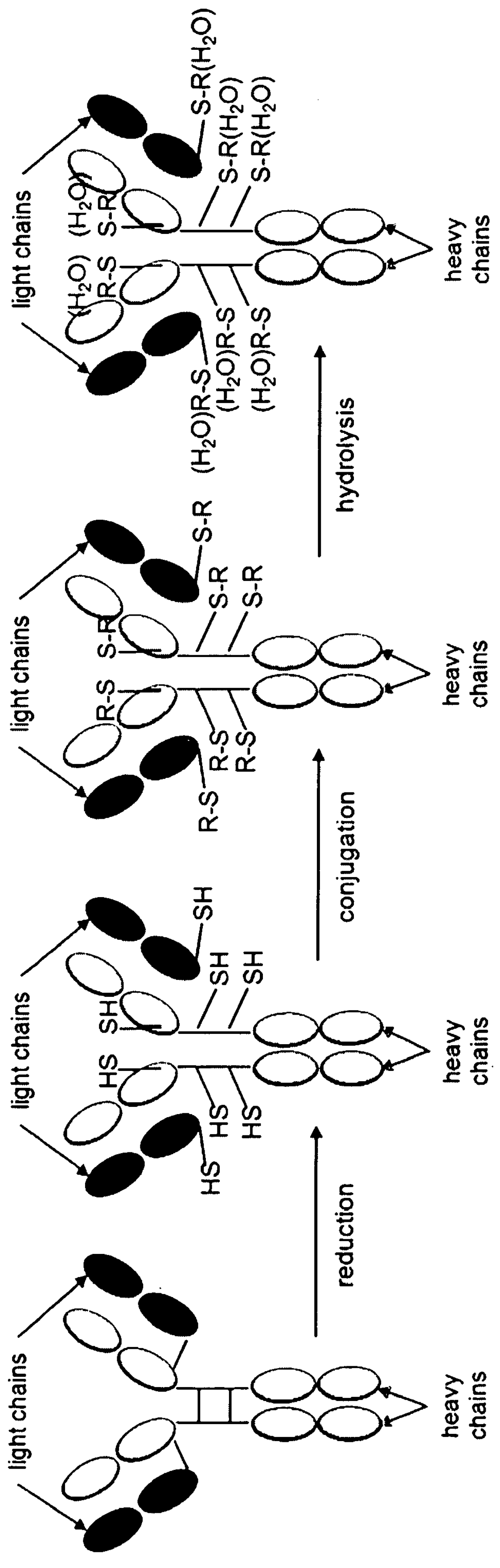
light chains
heavy chains
reduction
HS
SH
conjugation
R-S
S-R
hydrolysis
($H_2O$)R-S
S-R($H_2O$)

FIG. 1

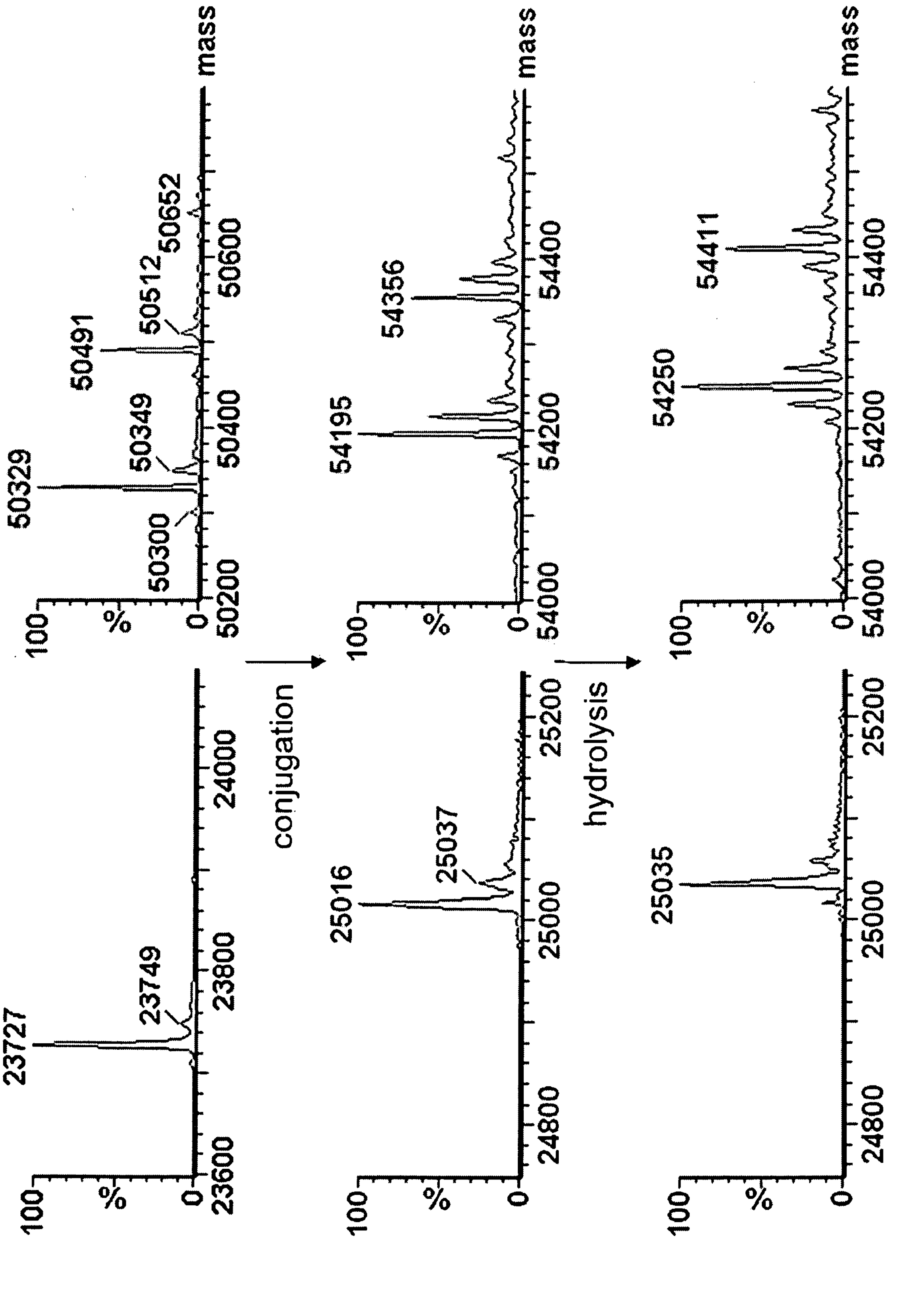

100
%
0
23600
23727
23749
23800
24000
conjugation
hydrolysis
24800
25000
25016
25037
25200
25035
50200
50300
50329
50349
50400
50491
50512
50600
50652
mass
54000
54195
54200
54356
54400
54250
54411


*FIG. 1 (cont.)*

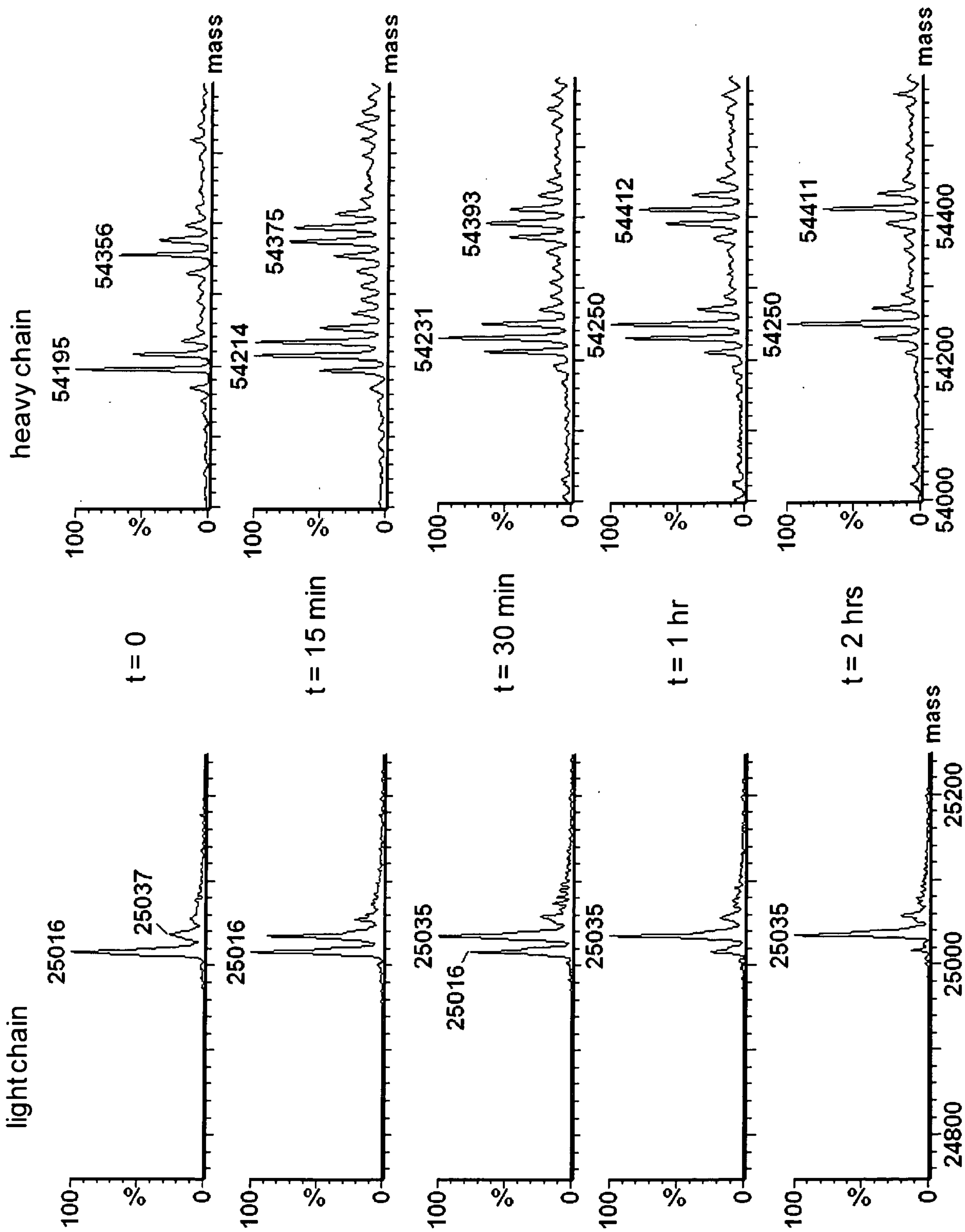
light chain
heavy chain
t = 0
t = 15 min
t = 30 min
t = 1 hr
t = 2 hrs
100
%
0
mass
25016
25037
25016
25035
25016
25035
25035
24800
25000
25200
54195
54356
54214
54375
54231
54393
54250
54412
54250
54411
54000
54200
54400

*FIG. 2*

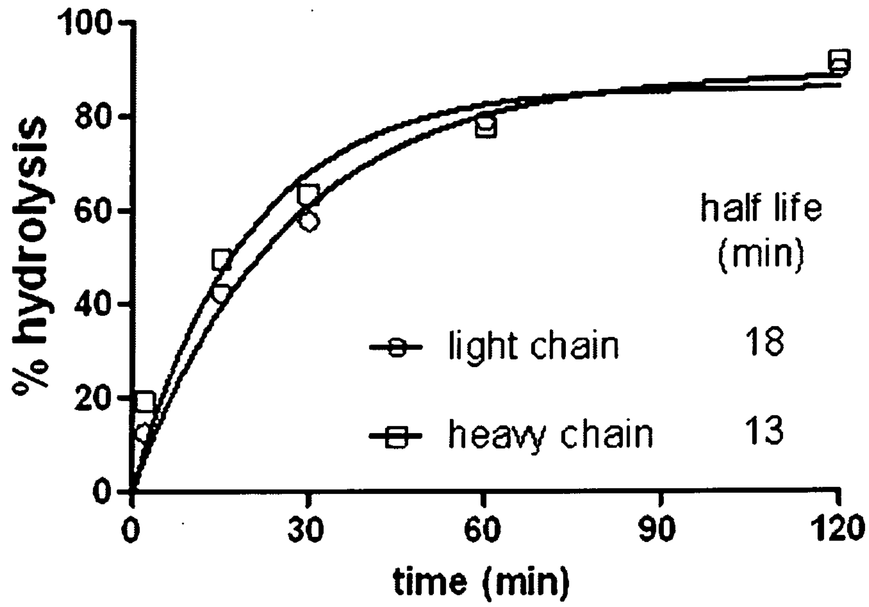
100
80
60
40
20
0
% hydrolysis
0
30
60
90
120
time (min)
half life (min)
light chain 18
heavy chain 13

*FIG. 2 (cont.)*

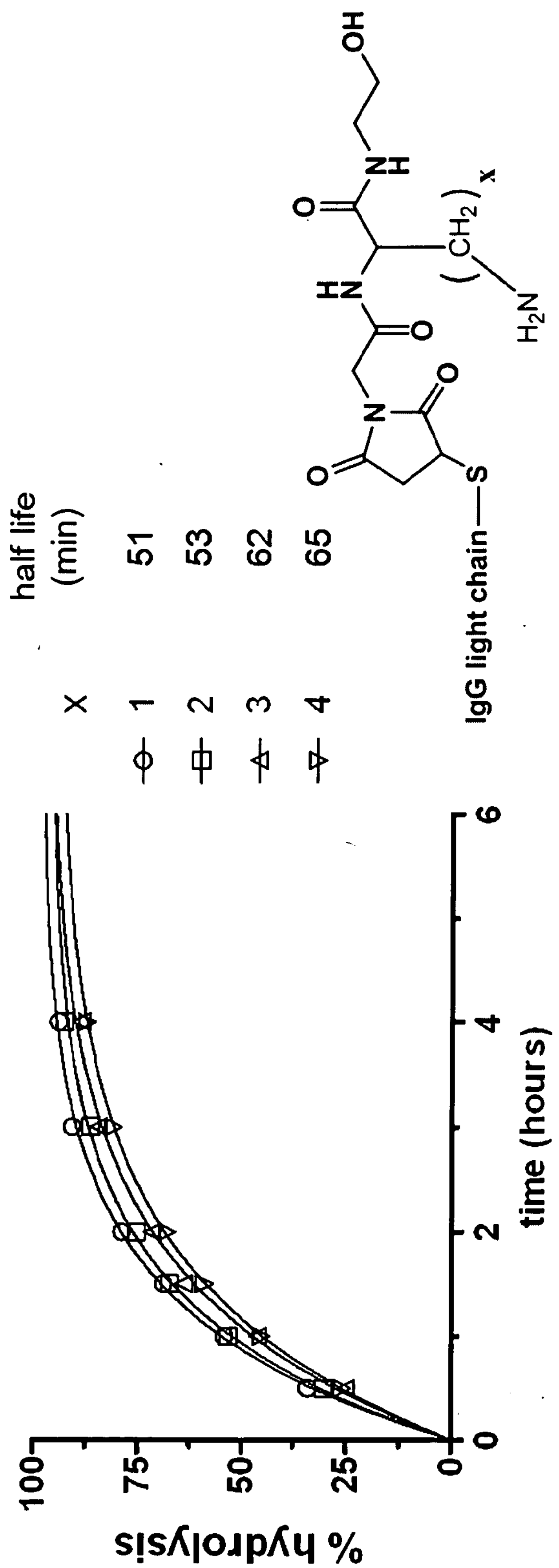
% hydrolysis
100
75
50
25
0
0
2
4
6
time (hours)
x
1
2
3
4
half life (min)
51
53
62
65

*FIG. 3*

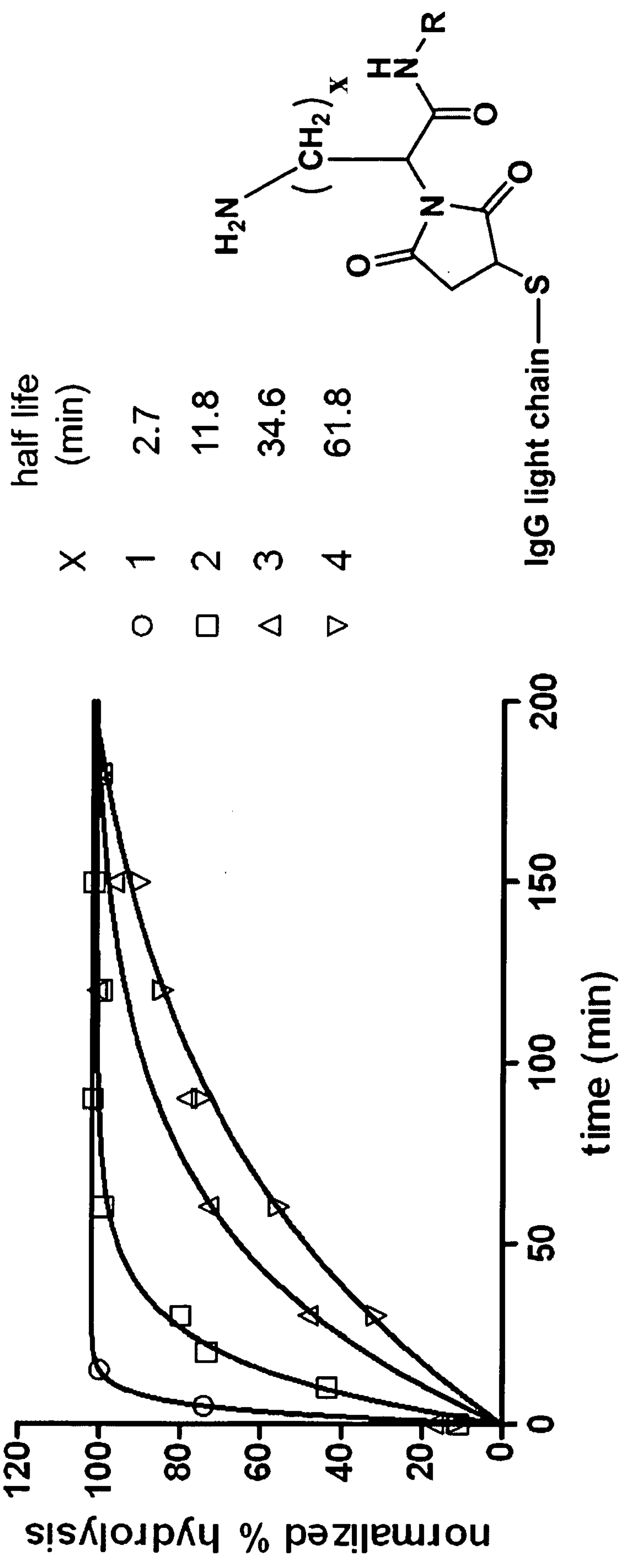
normalized % hydrolysis
0
20
40
60
80
100
120
0
50
100
150
200
time (min)
x
half life (min)
1
2.7
2
11.8
3
34.6
4
61.8

*FIG. 4*

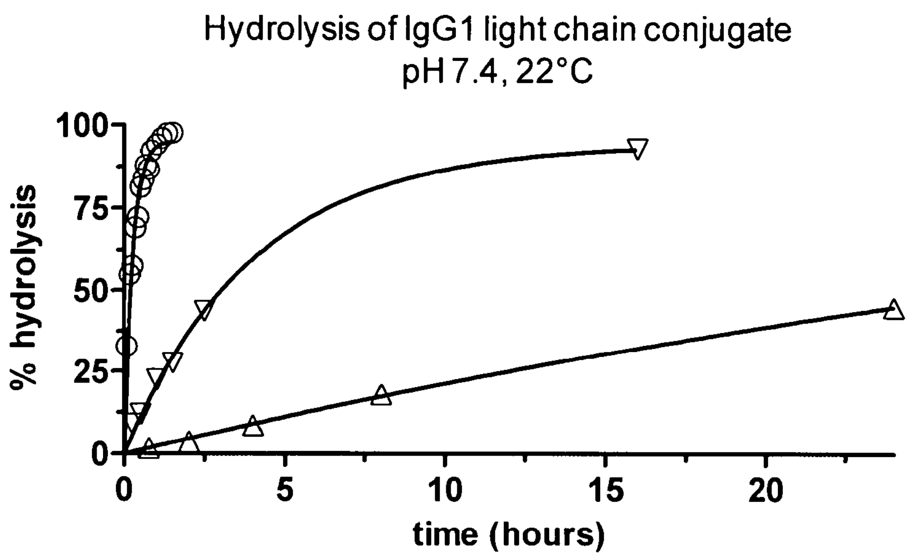


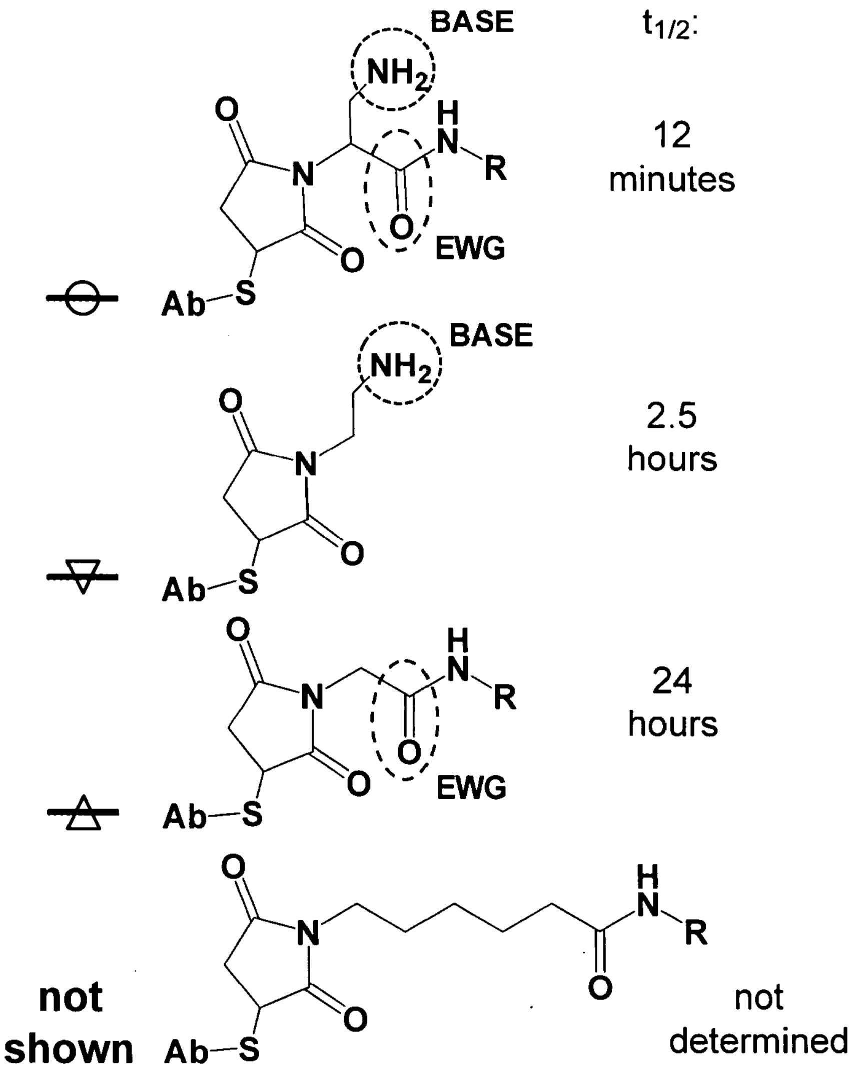

not shown

$t_{1/2}$:

12 minutes

2.5 hours

24 hours

not determined

*FIG. 5*

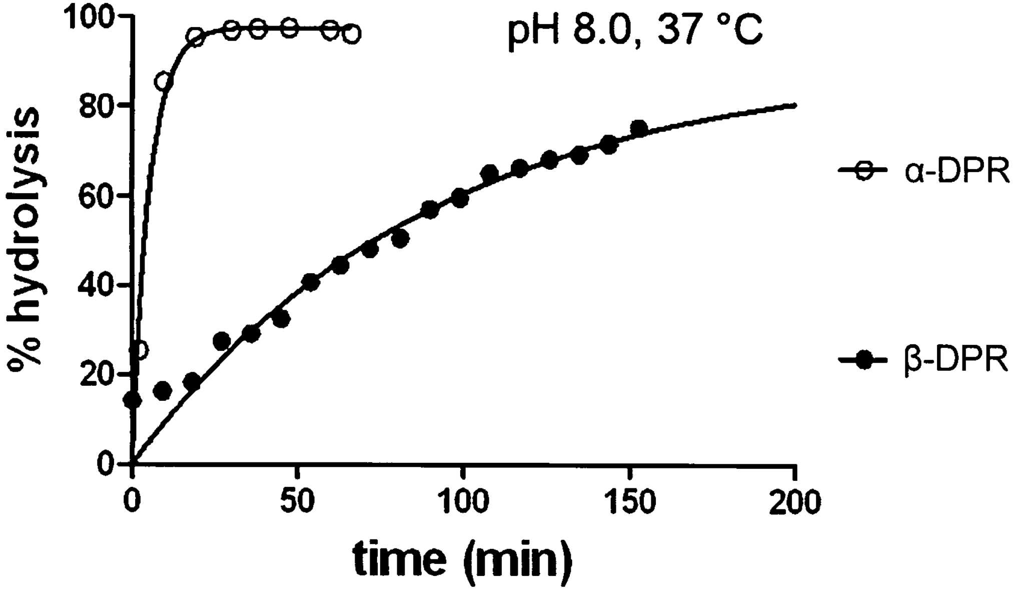


hydrolysis
$t_{1/2}$
(minutes):

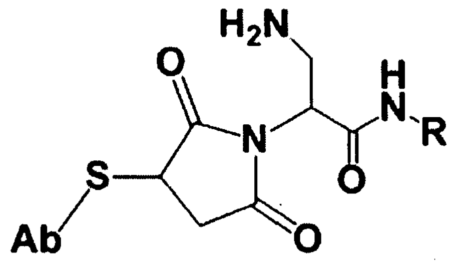

3.7

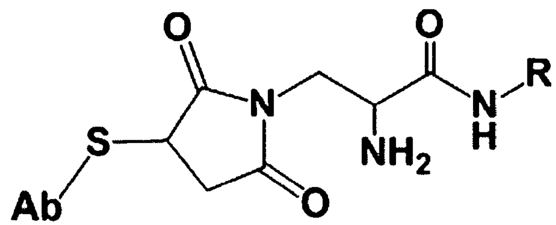

63

*FIG. 6*

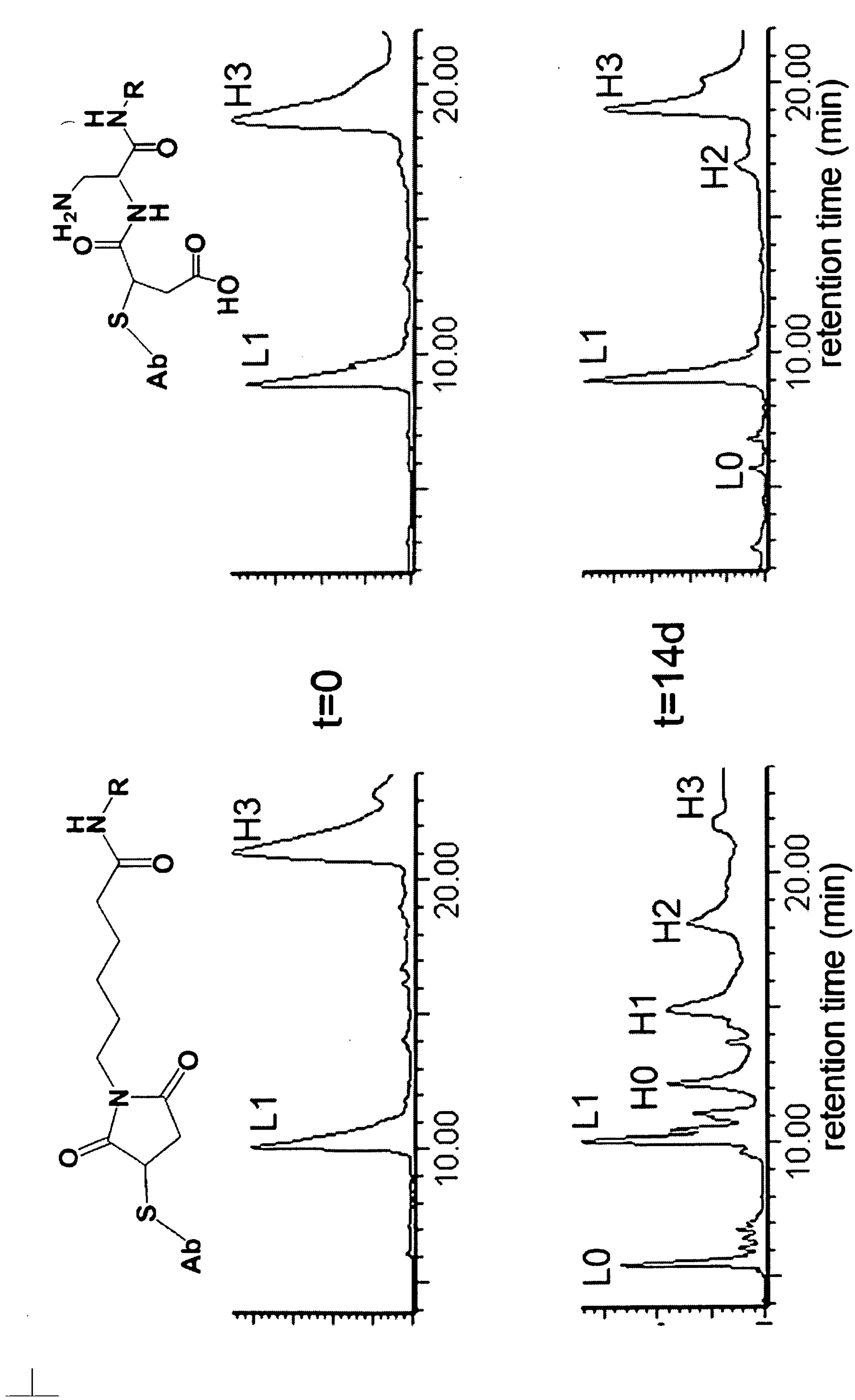


*FIG. 7*

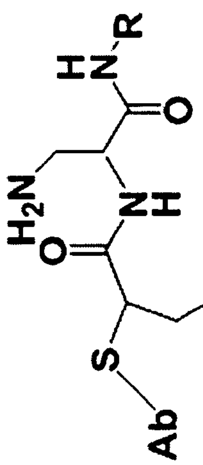

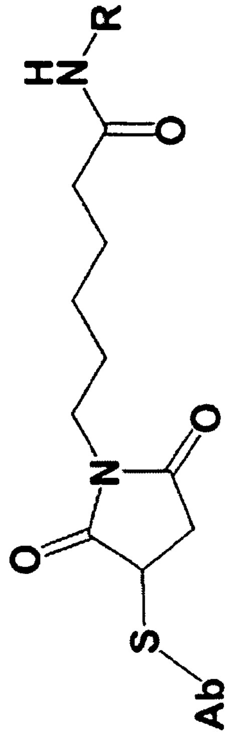

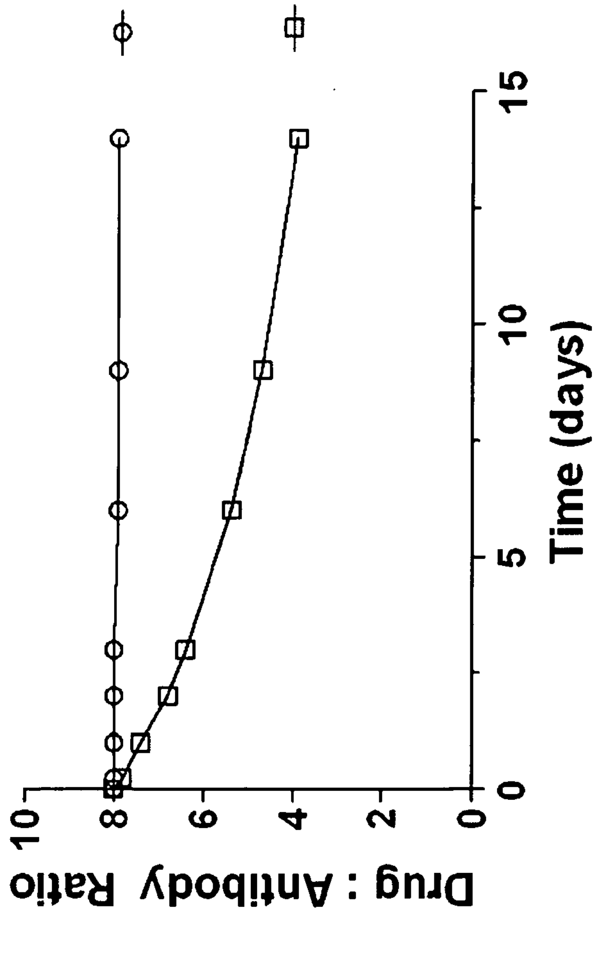


*FIG. 7 (cont.)*

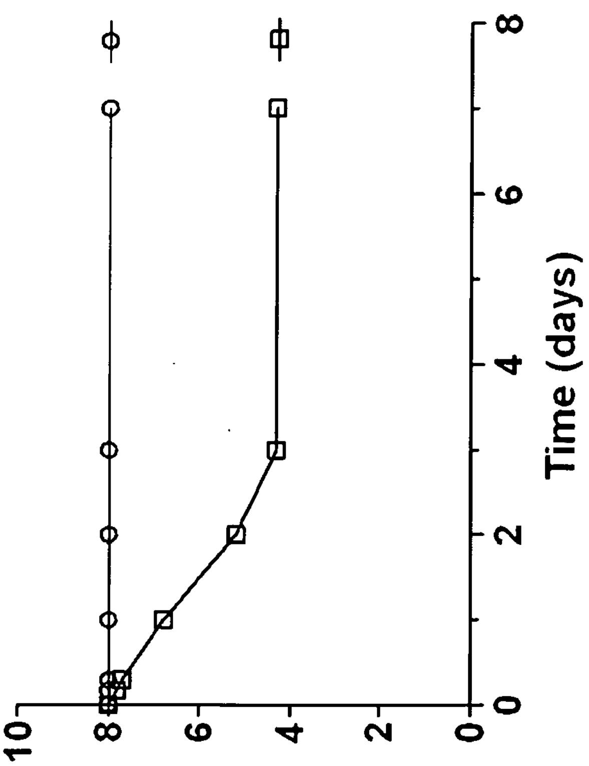


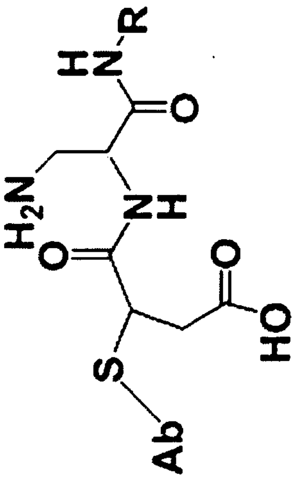

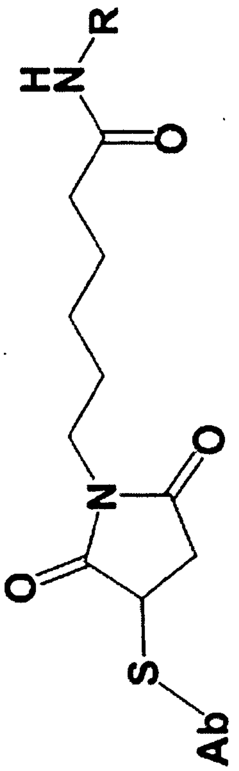

FIG. 8

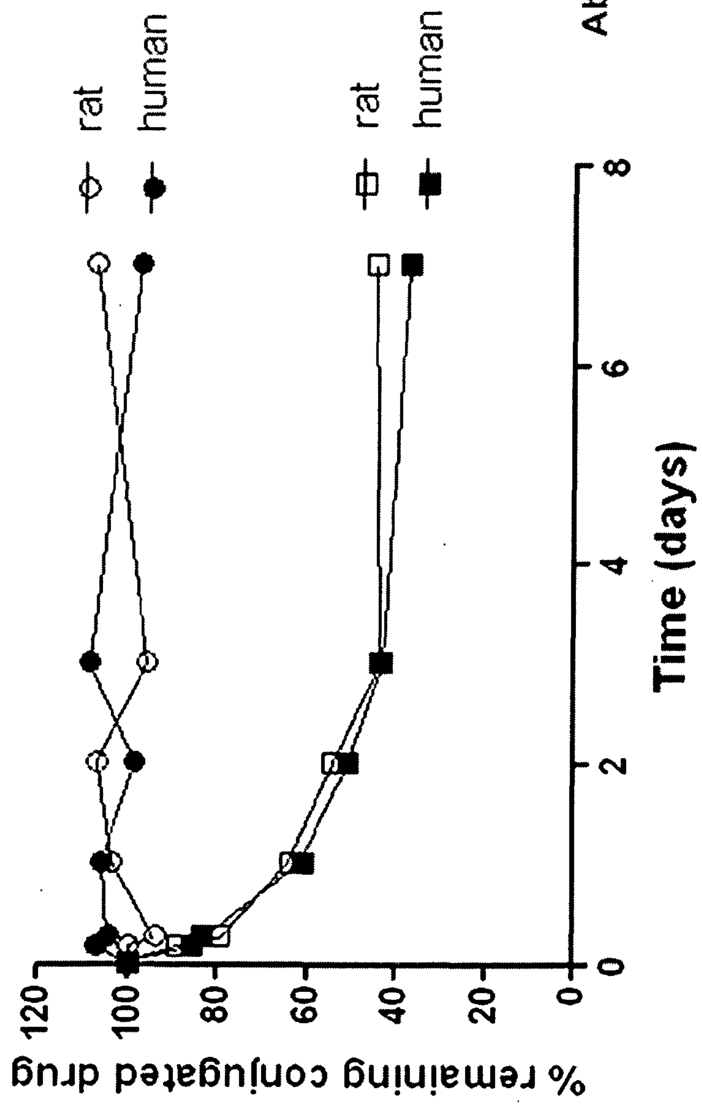

% remaining conjugated drug
120
100
80
60
40
20
0
0
2
4
6
8
Time (days)
rat
human
rat
human


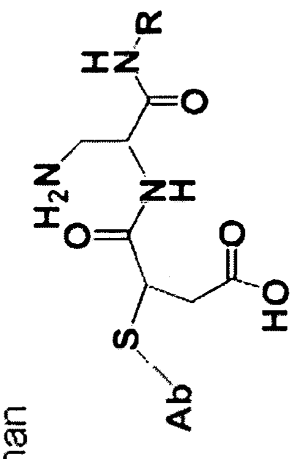

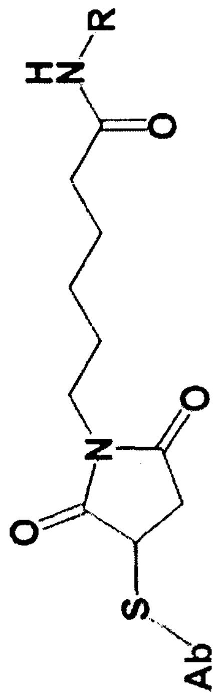

*FIG. 9*

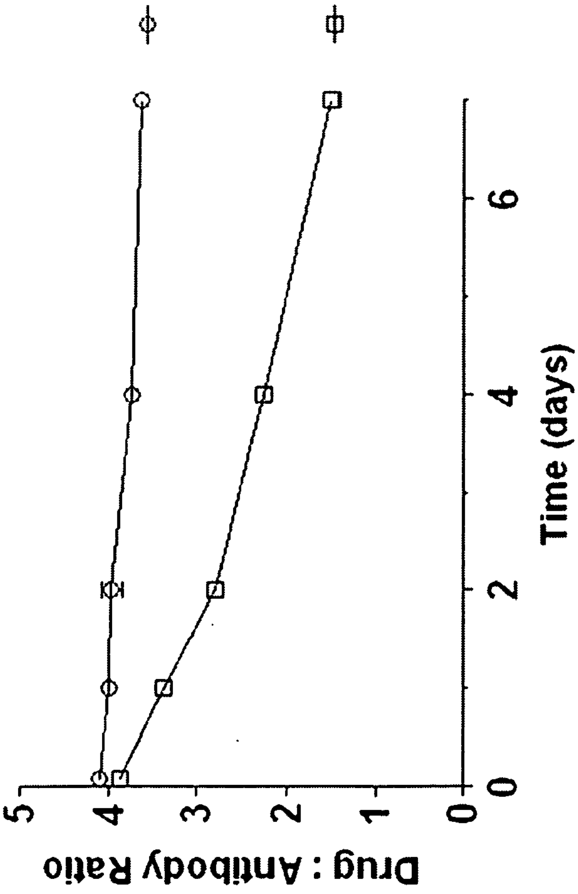


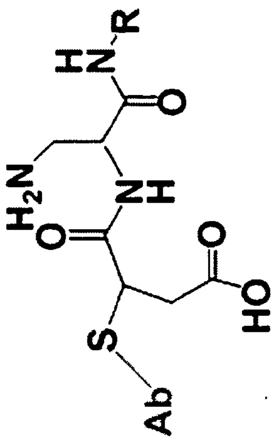

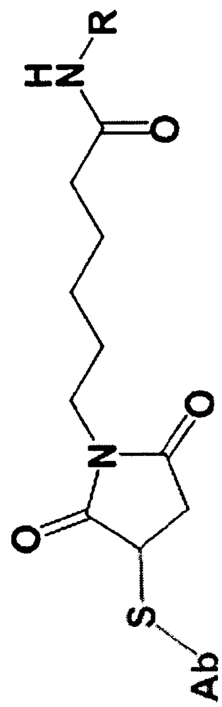

*FIG. 10*

untreated

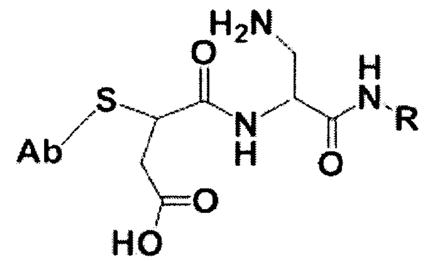

Ab = cAC10 (anti-CD30)
R = val-cit-PAB-MMAE

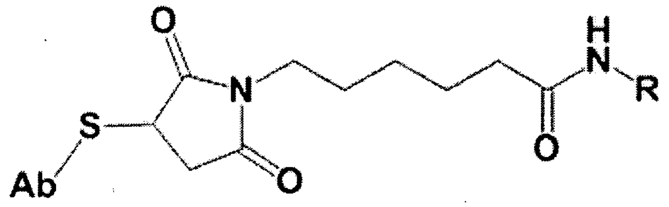

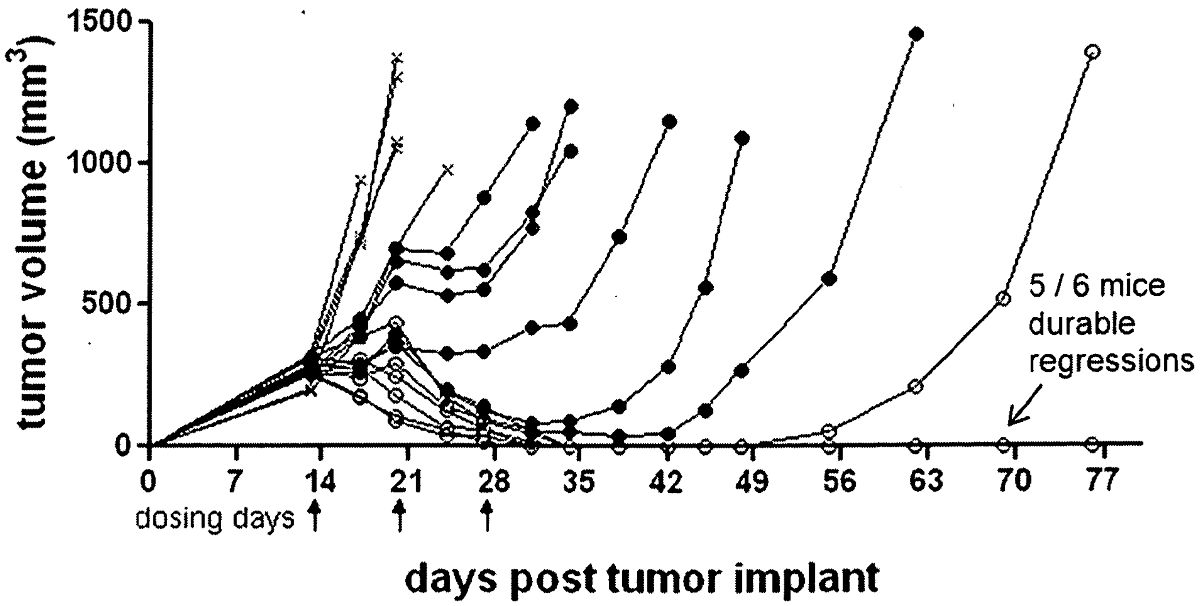


*FIG. 11*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61647373
- US 61773067
- US 79924413
- EP 0446071 A1
- US 4816567 A
- US 4816397 A
- US 5585089 A
- WO 8702671 A
- EP 0184187 A
- EP 0171496 A
- EP 0173494 A
- WO 8601533 A
- EP 012023 A
- US 5225539 A
- WO 9734631 A
- WO 2010091150 A
- WO 2012112708 A
- WO 2007085930 A
- WO 2011023883 A
- US 6130237 A
- US 20060024317 A
- US 8163888 B
- US 20030083263
- US 20050238649
- US 20050009751
- US 20090111756
- US 20110020343
- WO 04010957 A
- WO 02088172 A
- US 7659241 B
- US 8343928 B
- US 7498298 B
- US 20110256157 A
- WO 2005112919 A
- US 8142784 B
- US 4880935 A
- US 5122368 A
- US 5824805 A
- US 5622929 A
- US 20050238649 A
- WO 2004010957 A
- WO 2007038658 A
- US 6214345 B
- US 7968687 B
- US 20090111756 A
- US 20090018086 A
- US 20090274713 A
- US 5973166 A


### Non-patent literature cited in the description


- **ALLEY et al.** *Bioconjugate Chem.,* 2008, vol. 19, 759-765
- **SHEN et al.** *Nature Biotech,* 2012, vol. 30 (2), 184-9
- **STEPHEN C. ALLEY et al.** *Bioconjugate chemistry,* 01 March 2008, vol. 19 (3), 759-765
- **JANEWAY et al.** Immuno. Biology. Garland Publishing, 2001
- **TENG et al.** *Proc. Natl. Acad. Sci. USA.,* 1983, vol. 80, 7308-7312
- **KOZBOR et al.** *Immunology Today,* 1983, vol. 4, 72-79
- **OLSSON et al.** *Meth. Enzymol.,* 1982, vol. 92, 3-16
- **KABAT et al.** Sequences of Proteins of Immunological Interest. National Institute of Health, 1991
- **KABAT E et al.** *J. Immunology,* 1980, vol. 125 (3), 961-969
- **BERTER et al.** *Science,* 1988, vol. 240, 1041-1043
- **LIU et al.** *Proc. Natl. Acad. Sci. USA,* 1987, vol. 84, 3439-3443
- **LIU et al.** *J. Immunol.,* 1987, vol. 139, 3521-3526
- **SUN et al.** *Proc. Natl. Acad. Sci. USA,* 1987, vol. 84, 214-218
- **NISHIMURA et al.** *Cancer. Res.,* 1987, vol. 47, 999-1005
- **WOOD et al.** *Nature,* 1985, vol. 314, 446-449
- **SHAW et al.** *J. Natl. Cancer Inst.,* 1988, vol. 80, 1553-1559
- **MORRISON.** *Science,* 1985, vol. 229, 1202-1207
- **OI et al.** *BioTechniques,* 1986, vol. 4, 214
- **JONES et al.** *Nature,* 1986, vol. 321, 552-525
- **VERHOEYAN et al.** *Science,* 1988, vol. 239, 1534
- **BEIDLER et al.** *J. Immunol.,* 1988, vol. 141, 4053-4060
- **CHARI et al.** *Cancer Res.,* 1992, vol. 52, 127-131

- **PAGE et al.** *Intl. J. of Oncology,* 1993, vol. 3, 473-476
- **SKEHAN et al.** *J. Nat'l Cancer Inst.,* 1990, vol. 82, 1107-12
- **THORPE et al.** *Cancer Res.,* 1987, vol. 47, 5924-5931
- **WAWRZYNCZAK et al.** Immunoconjugates: Antibody Conjugates in Radioimagery and Therapy of Cancer. Oxford U. Press, 1987
- **DUBOWCHIK ; WALKER.** *Pharm. Therapeutics,* 1999, vol. 83, 67-123
- **NEVILLE et al.** *Biol. Chem.,* 1989, vol. 264, 14653-14661
- **HAY et al.** *Bioorg. Med. Chem. Lett.,* 1999, vol. 9, 2237
- **RODRIGUES et al.** *Chemistry Biology,* 1995, vol. 2, 223
- **STORM et al.** *J. Amer. Chem. Soc.,* 1972, vol. 94, 5815
- **AMSBERRY et al.** *J. Org. Chem,* 1990, vol. 55, 5867
- **KINGSBURY et al.** *J. Med. Chem.,* 1984, vol. 27, 1447
- **H. N. BORAH et al.** *J. Chem. Research (S),* 1998, 272-272
- **V. ONDRUS.** *ARKIVOC,* 2001, 60-67
- **M.A. WALKER.** *Tetrahedron Letters,* 1994, vol. 35 (5), 665-668
- **RYAN et al.** *Chem. Commun.,* 2011, vol. 47, 5452-5454
- **SMITH et al.** *J. Am. Chem. Soc.,* 2010, vol. 132 (6), 1960-1965